# EUROPEAN PATENT SPECIFICATION

(11) **EP 0 662 958 B1**
(45) Date of publication and mention of the grant of the patent: **11.12.2002**
(21) Application number: 93922748.4
(22) Date of filing: 27.09.1993
(51) Int. Cl.: C07D 211/60, C07D 401/12, C07D 487/04, A61K 31/445

(54) **1-(2-OXO-ACETYL)-PIPERIDINE-2-CARBOXYLIC ACID DERIVATIVES AS MULTI-DRUG-RESISTENT CANCER CELL SENSITIZERS**
1-(2-OXO-ACETYL)-PIPERIDIN-2-CARBONSÄURE DERIVATE ALS SENSIBILISATOREN FÜR MULTI-DRUG-RESISTENTE KREBSZELLEN
DERIVES DE L'ACIDE 1-(2-OXO-ACETYL)-PIPERIDINE-2-CARBOXYLIQUE UTILES COMME SENSIBILISATEURS DE CELLULES CANCEREUSES RESISTANT A DE MULTIPLES MEDICAMENTS

(30) Priority: 28.09.1992 US 952299
(43) Date of publication of application: 19.07.1995
(73) Proprietor: VERTEX PHARMACEUTICALS INCORPORATED, Cambridge, MA 02139-4211 (US)
(72) Inventor: ARMISTEAD, David, M., Maynard, MA 01574 (US); SAUNDERS, Jeffrey, O., Acton, MA 01720 (US); BOGER, Joshua, S., Concord, MA 01742 (US)
(74) Representative: VOSSIUS & PARTNER
(86) International application number: US9309145
(87) International publication number: WO94007858

(56) References cited:
- EP-A- 0 515 071
- WO-A-92/00278
- WO-A-92/19593
- GB-A- 2 247 456
- SCIENTIFIC AMERICAN, SPECIAL ISSUE MEDICINE 1993, REPRINTED FROM THE MARCH 1989 ISSUE. March 1989 pages 110 - 117 N. KARTNER, V. LING 'Multidrug Resistance in Cancer'
- TETRAHEDRON LETTERS. vol. 31, no. 34 , 1990 , OXFORD GB pages 4845 - 4848 GOULET M. T.,BOGER J. 'Degradative Studies on the Tricarbonyl Containing Macrolide Rapamycin.'

## Description

### TECHNICAL FIELD OF THE INVENTION

The present invention relates to novel compounds which maintain, increase, or restore sensitivity of cells to therapeutic or prophylactic agents. This invention also relates to pharmaceutical compositions comprising these compounds. The compounds and pharmaceutical compositions of this invention are particularly well-suited for treatment of multi-drug resistant cells, for prevention of the development of multi-drug resistance and for use in multi-drug resistant cancer therapy.

### BACKGROUND OF THE INVENTION

A major problem affecting the efficacy of chemotherapy is the evolution of cells which, upon exposure to a chemotherapeutic drug, become resistant to a multitude of structurally unrelated drugs and therapeutic agents. The appearance of such multi-drug resistance often occurs in the presence of overexpression of the 170-kDA membrane P-glycoprotein (gp-170). The gp-170 protein is present in the plasma membranes of some healthy tissues, in addition to cancer cell lines, and is homologous to bacterial transport proteins (Hait et al., Cancer Communications, Vol. 1(1), 35 (1989); West, TIBS, Vol. 15, 42 (1990)). The protein acts as an export pump, conferring drug resistance through active extrusion of toxic chemicals. Although the mechanism for the pump is unknown, it is speculated that the gp-170 protein functions by expelling substances that share certain chemical or physical characteristics, such as hydrophobicity, the presence of carbonyl groups, or the existence of a glutathione conjugate (see West).

Various chemical agents have been administered to repress multi-drug resistance and restore drug sensitivity. While some drugs have improved the responsiveness of MDR cells to chemotherapeutic agents, they often have been accompanied by undesirable clinical side effects (see Hait et al.). For example, although cyclosporin A ("CsA"), a widely accepted immunosuppressant, can sensitize certain carcinoma cells to chemotherapeutic agents (Slater et al., Br. J. Cancer, Vol. 54, 235 (1986)), the concentrations needed to achieve that effect produce significant immunosuppression in patients whose immune systems are already compromised by chemotherapy (see Hait et al.). Similarly, calcium transport blockers and calmodulin inhibitors both sensitize multi-drug resistant ("MDR") cells, but each produces undesirable physiological effects (see Hait et al.; Twentyman et al., Br. J. Cancer, Vol. 56, 55 (1987)).

Recent developments have led to agents said to be of potentially greater clinical value in the sensitization of MDR cells. These agents include analogs of CsA which do not exert an immunosuppressive effect, such as 11-methyl-leucine cyclosporin (11-met-leu CsA) (see Hait et al.; Twentyman et al.), or agents that may be effective at low doses, such as the immunosuppressant FK-506 (Epand and Epand, Anti-Cancer Drug Design 6, 189 (1991)). Despite these developments, the need remains for effective agents which may be used to resensitize MDR cells to therapeutic or prophylactic agents or to prevent the development of multi-drug resistance.

### SUMMARY OF THE INVENTION

The present invention provides novel compounds that are useful to maintain, increase or restore drug sensitivity in multi-drug resistant ("MDR") cells, compositions containing those compounds and methods for using them. The compounds of this invention may be used alone or in combination with other therapeutic or prophylactic agents to maintain, increase or restore the therapeutic or prophylactic effects of drugs in cells, especially MDR cells, or to prevent the development of MDR cells. According to one embodiment of this invention, these novel compounds, compositions and methods are advantageously used to aid or enhance chemotherapy regimens for the treatment or prophylaxis of cancer and other diseases.

The present invention also provides methods for preparing the compounds of this invention and intermediates useful in those methods.

### DETAILED DESCRIPTION OF THE INVENTION

This invention relates to a novel class of compounds characterized by the ability to prevent multi-drug resistance or to maintain, increase or restore drug sensitivity in multi-drug resistant ("MDR") cells. More particularly, these compounds are represented by the formula (I): 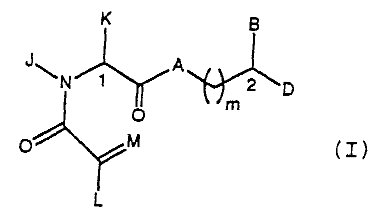 wherein A is CH₂, oxygen, NH or N-(C1-C4 alkyl);
wherein B and D are independently:
(i) hydrogen, Ar, (C1-C10)-straight or branched alkyl, (C2-C10)-straight or branched alkenyl or alkynyl, (C5-C7)-cycloalkyl substituted (C1-C6)-straight or branched alkyl, (C2-C6)-straight or branched alkenyl or alkynyl, (C5-C7)-cycloalkenyl substituted (C1-C6)-straight or branched alkyl, (C2-C6)-straight or branched alkenyl or alkynyl, or Ar substituted (C1-C6)-straight or branched alkyl, (C2-C6)-straight or branched alkenyl or alkynyl
wherein, in each case, any one of the CH₂ groups of said alkyl, alkenyl or alkynyl chains may be optionally replaced by a heteroatom selected from the group consisting of O, S, SO, SO₂, N, and NR, wherein R is selected from the group consisting of hydrogen, (C1-C4)-straight or branched alkyl, (C2-C4)-straight or branched alkenyl or alkynyl, and (C1-C4) bridging alkyl
wherein a bridge is formed between the nitrogen and a carbon atom of said heteroatom-containing chain to form a ring, and wherein said ring is optionally fused to an Ar group; or 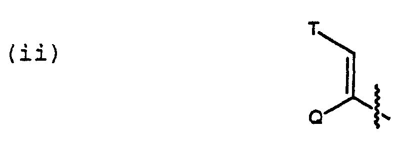 wherein Q is hydrogen, (C1-C6)-straight or branched alkyl or (C2-C6)-straight or branched alkenyl or alkynyl;
wherein T is Ar or substituted 5-7 membered cycloalkyl with substituents at positions 3 and 4 which are independently selected from the group consisting of oxo, hydrogen, hydroxyl, O-(C1-C4)-alkyl, and O-(C2-C4)-alkenyl;
wherein Ar is a carbocyclic aromatic group selected from the group consisting of phenyl, 1-naphthyl, 2-naphthyl, indenyl, azulenyl, fluorenyl, anthracenyl, and mono and bicyclic heterocyclic ring systems with individual ring sizes being 5 or 6 which may contain in either or both rings a total of 1-4 heteroatoms independently selected from oxygen, nitrogen, and sulfur -- such ring systems include heterocyclic aromatic groups selected from the group consisting of 2-furyl, 3-furyl, 2-thienyl, 3-thienyl, 2-pyridyl, 3-pyridyl, 4-pyridyl, pyrrolyl, oxazolyl, thiazolyl, imidazolyl, pyraxolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, benzo[b]thiophenyl, 1H-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, carbazolyl, acridinyl, phenazinyl, phenothiazinyl, and phenoxazinyl;
wherein Ar may contain one to three substituents which are independently selected from the group consisting of hydrogen, halogen, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl, N-(C1-C5-straight or branched alkyl or alkenyl) carboxamides, N,N-di-(C1-C5-straight or branched alkyl or C2-C5-straight or branched alkenyl)carboxamides, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, and q is 0-2;
wherein L is either hydrogen or U; M is either oxygen or CH-U, provided that if L is hydrogen, then M is CH-U or if M is oxygen then L is U;
wherein U is hydrogen, O-(C1-C4)-straight or branched alkyl or O-(C2-C4)straight or branched alkenyl, (C1-C6)-straight or branched alkyl or (C2-C6)-straight or branched alkenyl, (C5-C7)-cycloalkyl or (C5-C7)-cycloalkenyl substituted with (C1-C4)-straight or branched alkyl or (C2-C4)-straight or branched alkenyl, [(C1-C4)-alkyl or (C2-C4)-alkenyl]-Y or Y;
wherein Y is selected from the group consisting of phenyl, 1-naphthyl, 2-naphthyl, indenyl, azulenyl, fluorenyl, anthracenyl, 2-pyrrolinyl, 3-pyrrolinyl, pyrolidinyl, 1,3-dioxolyl, 2-imidazolinyl, imidazolidinyl, 2H-pyranyl, 4H-pyranyl, piperidyl, 1,4-dioxanyl, morpholinyl, 1,4-dithianyl, thiomorpholinyl, piperazinyl, quinuclidinyl, and heterocyclic aromatic groups as defined above;
where Y may contain one to three substituents which are independently selected from the group consisting of hydrogen, halogen, hydroxyl, nitro, trifluoromethyl, trifluoromethoxy, (C1-C6)-straight or branched alkyl, (C1-C6)-straight or branched alkenyl, O-(C1-C4)-straight or branched alkyl, O-(C2-C4)-straight or branched alkenyl, O-benzyl, O-phenyl, 1,2-methylenedioxy, amino, and carboxyl;
wherein J is hydrogen, (C1-C2) alkyl or benzyl; K is (C1-C4)-straight or branched alkyl, benzyl or cyclohexylmethyl, or wherein J and K may be taken together to form a 5-7 membered heterocyclic ring which may contain a heteroatom selected from the group consisting of O, S, SO and SO₂; and
wherein m is 0-3.

The stereochemistry at positions 1 and 2 (formula I) may be independently R or S.

Preferably, at least one of B or D is independently a straight chain terminated by an aryl group, i.e., a group represented by the formula -(CH₂)ᵣ-(X)-(CH₂)ₛ-Ar, wherein
r is 0-4;
s is 0-1;
Ar is as defined above; and
each X is independently selected from the group consisting of CH₂, O, S, SO, SO₂, N, and NR, wherein R is selected from the group consisting of hydrogen, (C1-C4)-straight or branched alkyl, (C2-C4)-straight or branched alkenyl or alkynyl, and (C1-C4) bridging alkyl wherein a bridge is formed between the nitrogen atom and the Ar group.

According to one embodiment of this invention, the heterocyclic aromatic groups are selected from the group consisting of furan, thiophene, pyrrole, pyridine, indolizine, indole, isoindole, benzo[b]furan, benzo[b]thiophene, 4H-quinolizine, quinoline, isoquinoline, 1,2,3,4-tetrahydroquinoline, isoxazole, and 1,2,3,4-tetrahydroisoquinoline.

According to another embodiment of this invention, at least one of B or D is selected from the group consisting of (C2-C10)-straight or branched alkynyl, (C5-C7)-cycloalkyl substituted (C2-C6)-straight or branched alkynyl, (C5-C7)-cycloalkenyl substituted (C2-C6)-straight or branched alkynyl, and Ar substituted (C2-C6)-straight or branched alkynyl.

Also within the scope of this invention are compounds of formula (I), wherein at least one of B or D is selected from the group consisting of Ar', Ar'-substituted (C1-C6)-straight or branched alkyl, and Ar'-substituted (C2-C6)-straight or branched alkenyl or alkynyl; wherein Ar' is an Ar group substituted with one to three substituents which are independently selected from the group consisting of N-(straight or branched C1-C5 alkyl or C2-C5 alkenyl) carboxamides, N,N-di-(straight or branched C1-C5 alkyl or C2-C5 alkenyl)carboxamides, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q} -O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, wherein q is 0-2.

Examples of some preferred compounds of formula (I), wherein J and K are taken together to form a 5-7 membered heterocyclic ring, are shown in Table 1 and are further illustrated in the examples herein.*

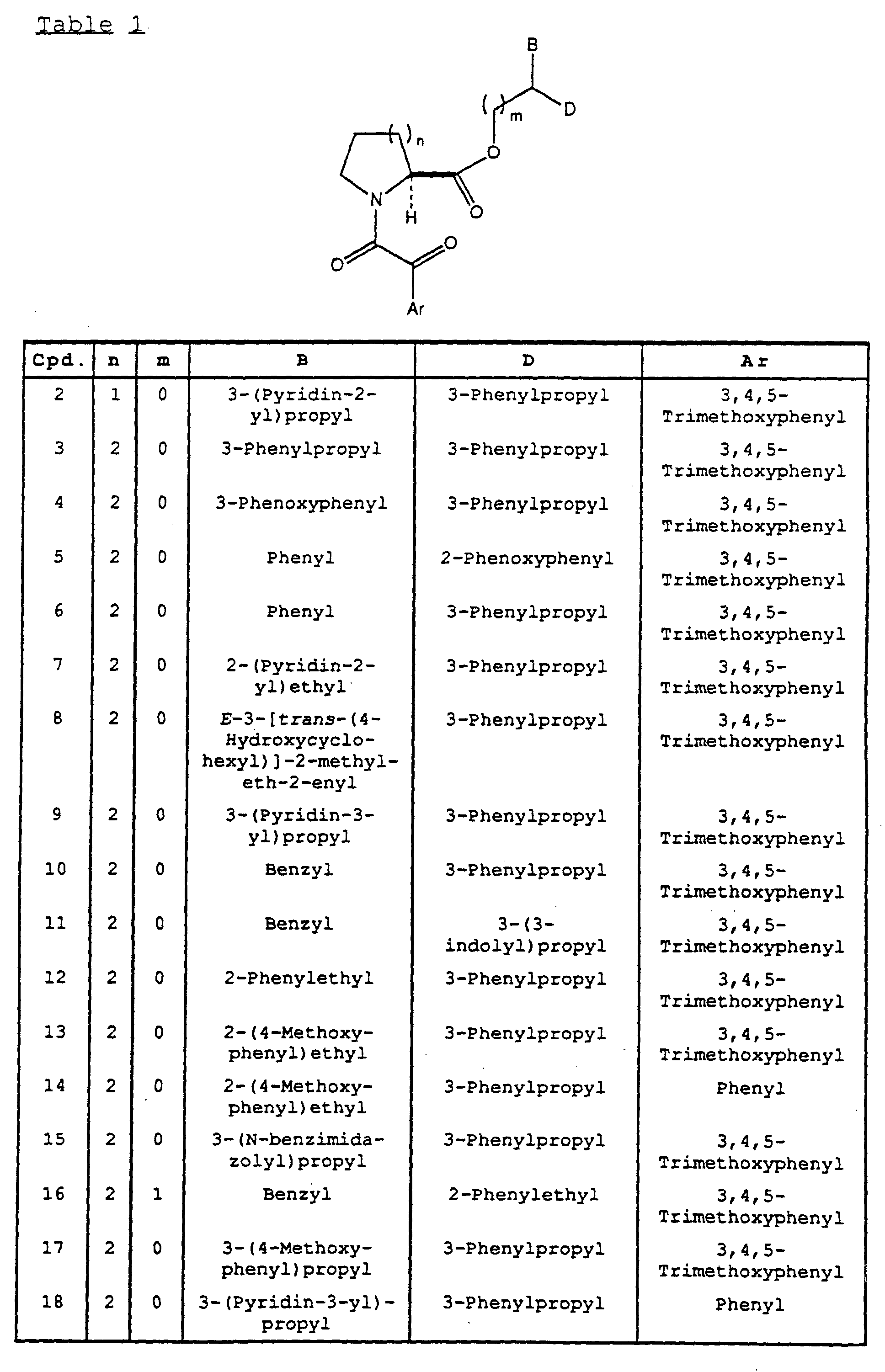

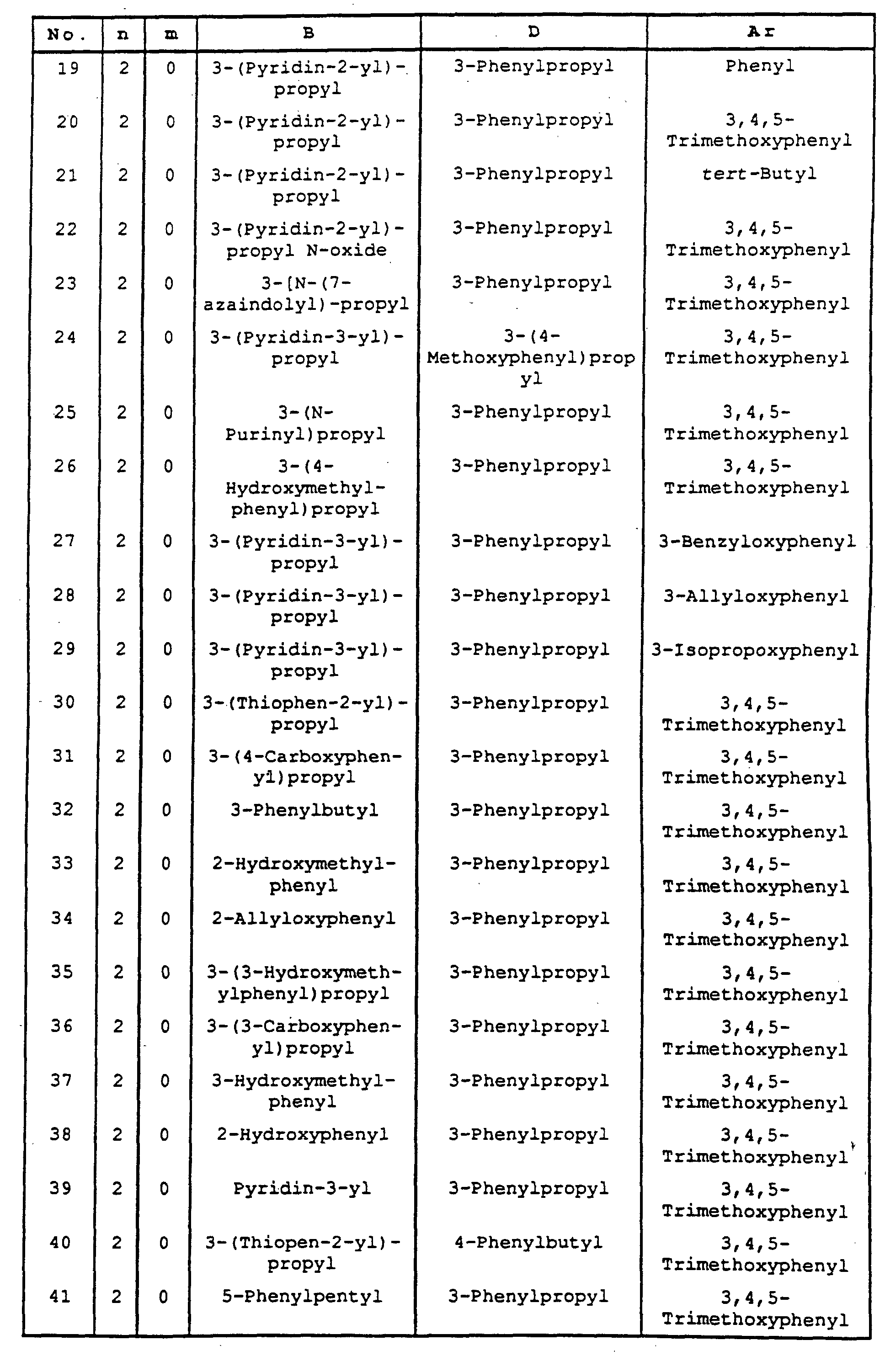

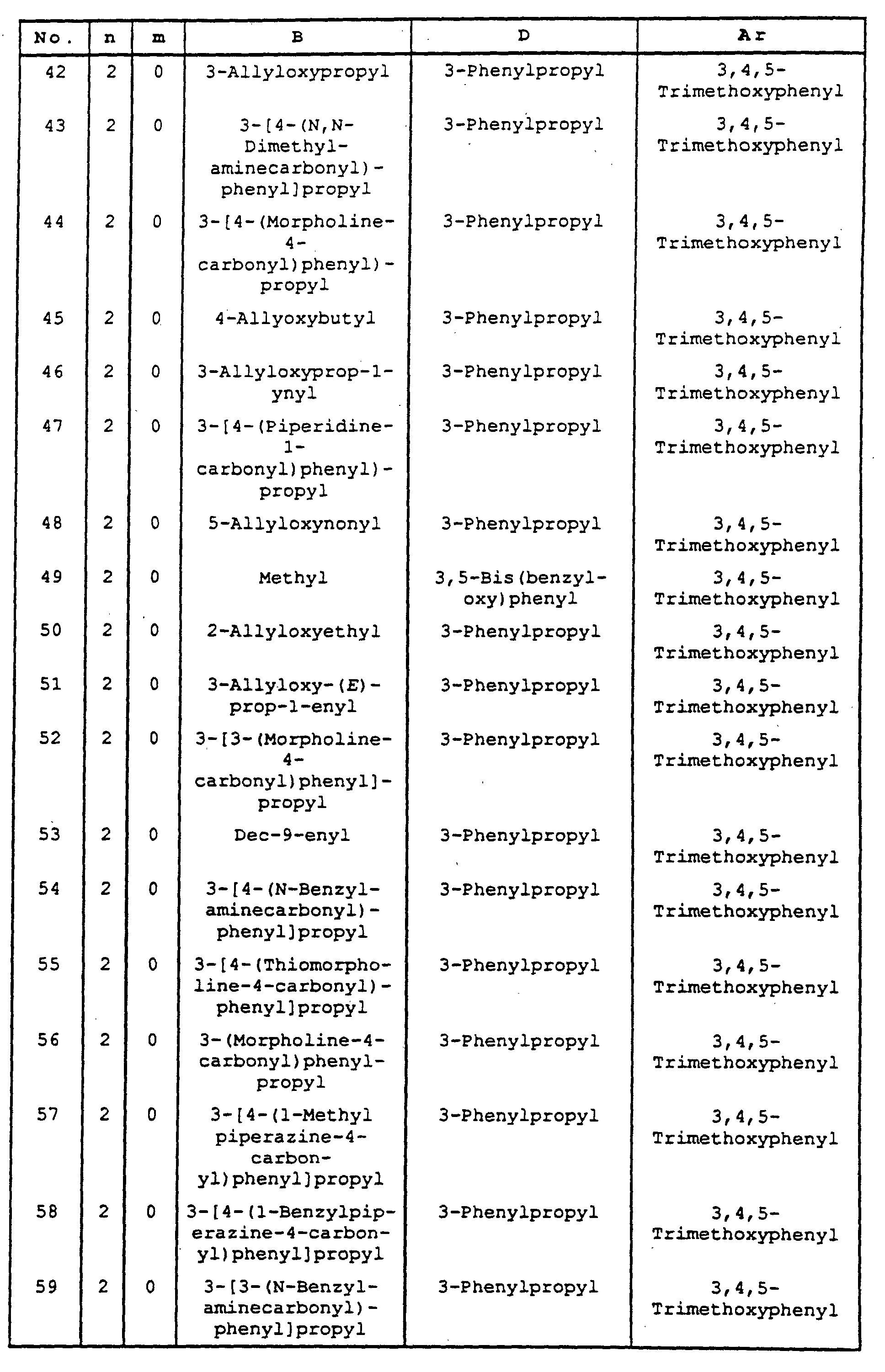

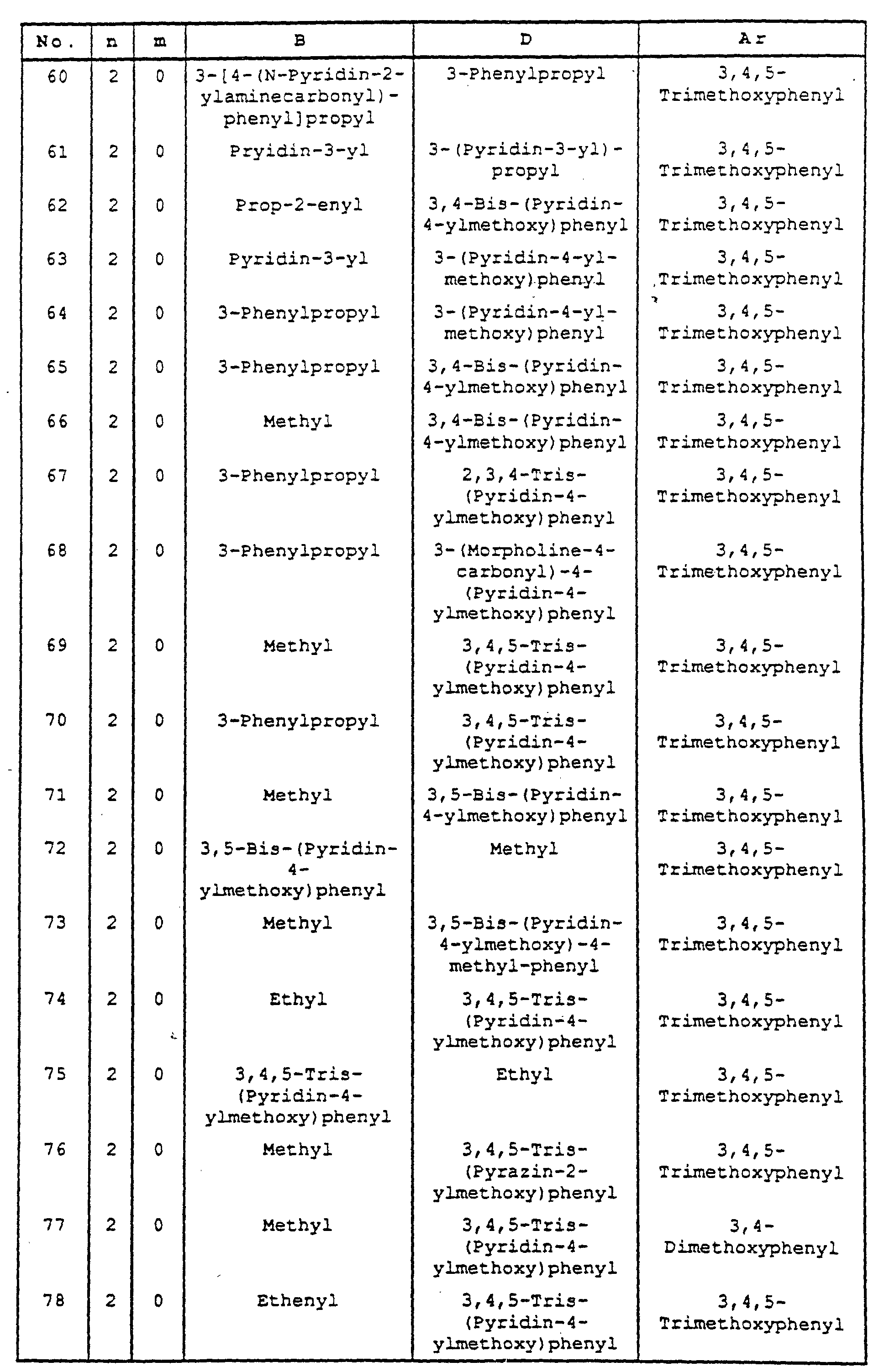

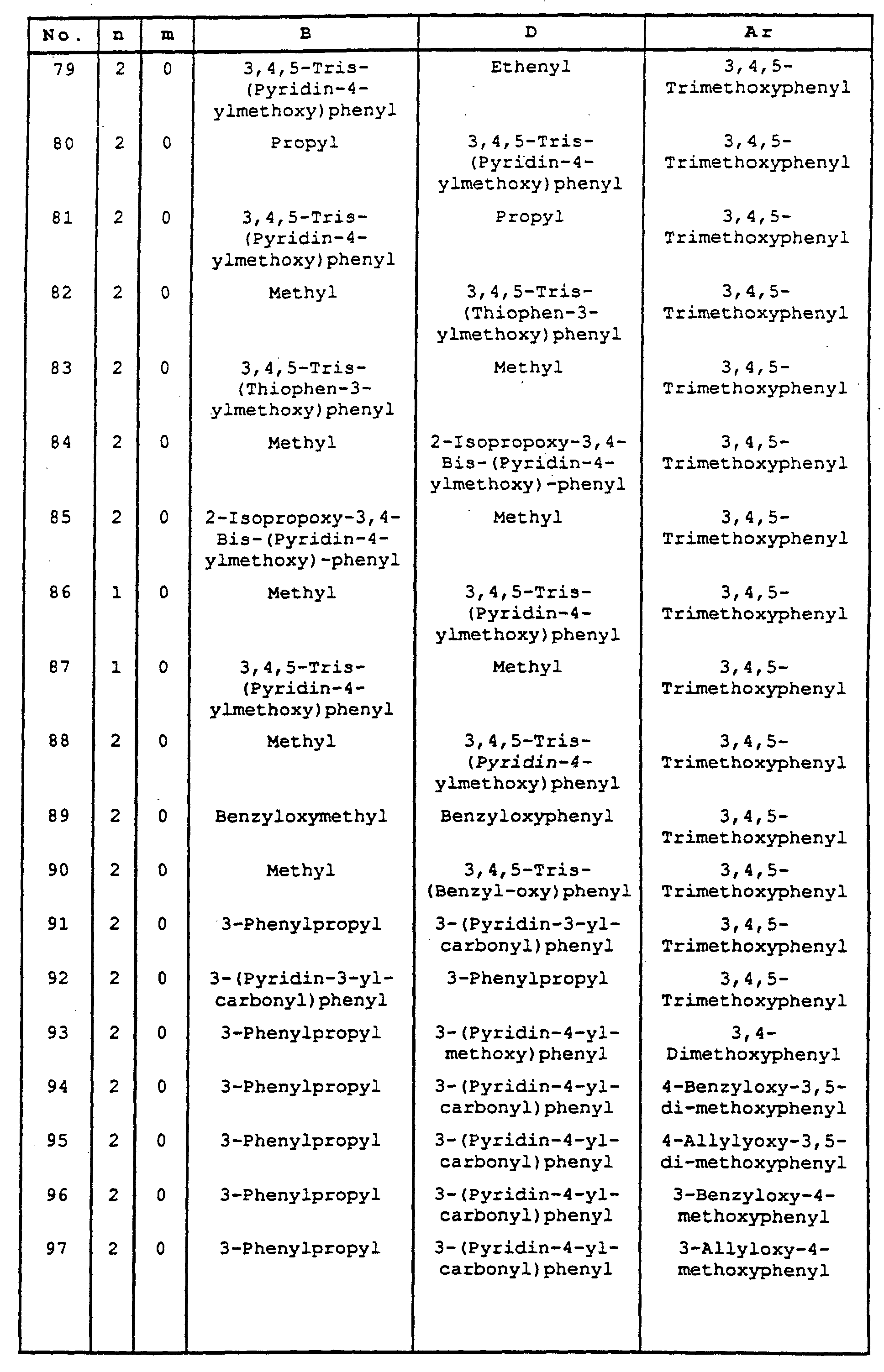

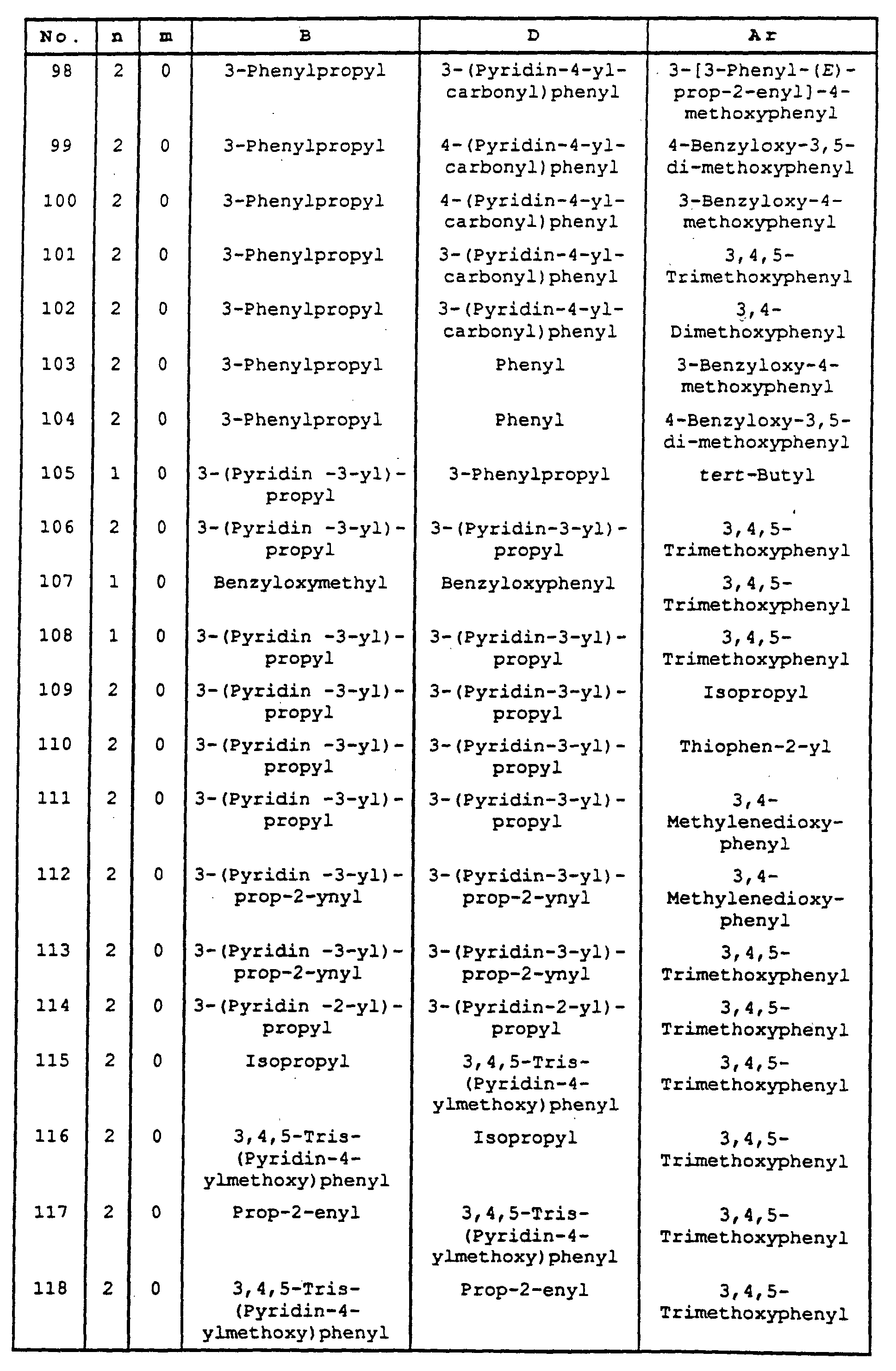

* It should be understood that with respect to the aspects of this invention relating to the use of compounds described herein in compositions or methods for treating or preventing multi-drug resistance, those compounds are represented by formula (I), as defined above. With respect to the aspect of this invention relating to the novel compounds described herein, those compounds are represented by formula (I), as defined above, except that B and D can not be hydrogen.

The most preferred compounds of this invention are (S)-1-(2-oxo-2-(3,4,5-trimethoxyphenyl) acetyl)piperidine-2-carboxylic acid-4-pyridin-3-yl-1-(3-pyridin-3-yl)propylbutyl ester, and (R)-1-(2-oxo-2-(3,4,5-trimethoxyphenyl)acetyl) piperidine-2-carboxylic acid-4-pyridin-3-yl-1-(3-pyridin-3-yl) propylbutyl ester, pharmaceutically acceptable derivatives thereof and mixtures thereof.

As used herein, the compounds of this invention, including the compounds of formula (I), are defined to include pharmaceutically acceptable derivatives thereof. A "pharmaceutically acceptable derivative" denotes any pharmaceutically acceptable salt, ester, or salt of such ester, of a compound of this invention or any other compound which, upon administration to a patient, is capable of providing (directly or indirectly) a compound of this invention, or a metabolite or residue thereof, characterized by the ability to maintain, increase or restore sensitivity of MDR cells to therapeutic or prophylactic agents or to prevent development of multi-drug resistance.

Compounds of this invention represented by formula (I) may be obtained using any conventional technique. Preferably, these compounds are chemically synthesized from readily available starting materials, such as alpha-amino acids. Modular and convergent methods for the synthesis of these compounds are also preferred. In a convergent approach, for example, large sections of the final product are brought together in the final stages of the synthesis, rather than by incremental addition of small pieces to a growing molecular chain.

Scheme 1 illustrates a representative example of a convergent process for the synthesis of compounds of formula (I'), a preferred subset of compounds of formula (I), wherein A is oxygen. The process comprises esterification of a protected alpha-amino acid of formula (X), wherein P is a protecting group, with an alcohol of formula (XI). Protected alpha-amino acids are well known in the art and many are commercially available. For example, common protecting groups and convenient methods for the protection of amino acids are described in T. W. Greene, P. G. M. Wuts, Protective Groups in Organic Chemistry, 2nd Ed., John Wiley and Sons, New York (1991). Alkoxycarbonyl groups are preferred for protection of the nitrogen atom in compounds of formula (X), with t-butoxycarbonyl (Boc), benzyloxycarbonyl (Cbz), allyloxycarbonyl (Alloc), and trimethylsilylethoxycarbonyl (Teoc) being more preferred.

After esterification, compounds of formula (XII) are deprotected under suitable deprotection conditions (see Greene, supra), and the free amino group of (XIII) is then acylated with a compound of formula (XIV), or an activated derivative thereof, to yield a compound of formula (I'). Methods for activation of carboxyl functionalities in carboxylic acids such as compounds of formula (XIV) are well known and many activating agents are commercially available.

Alcohols of formula (XI) wherein m is 0 (XI') can also be conveniently prepared, for example, as illustrated in Schemes 2 and 3. Reaction of an organometallic reagent of formula (XV) and an aldehyde of formula (XVI) provides alcohols of formula (XI') (Scheme 2).

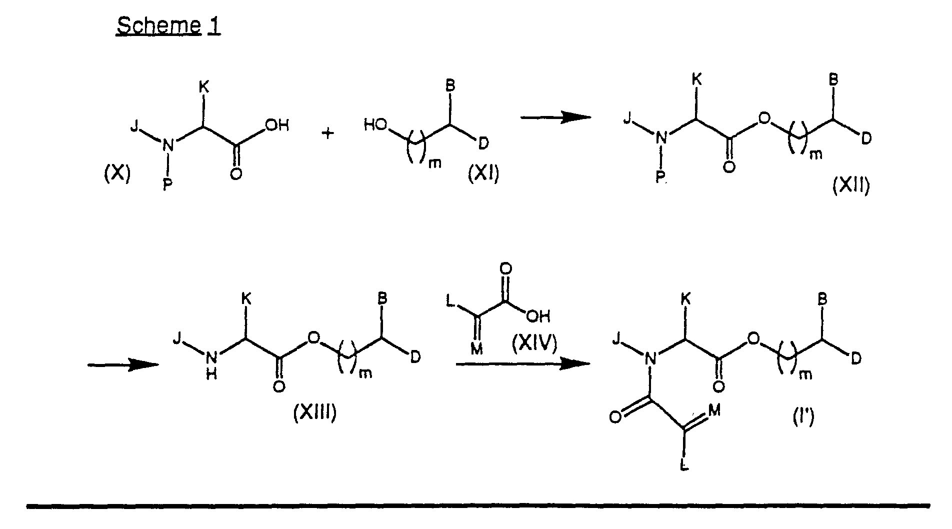

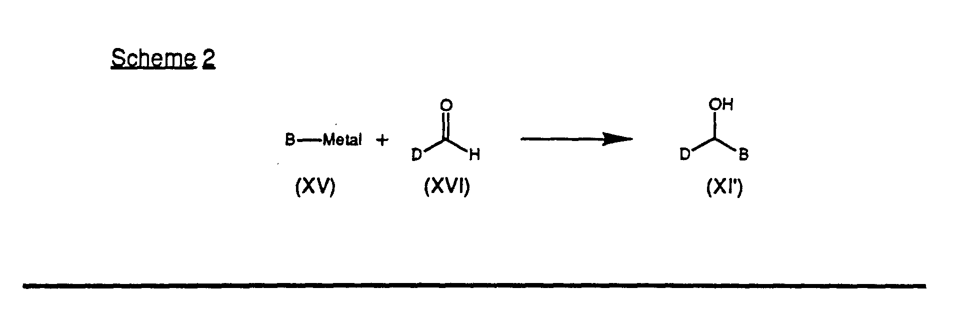

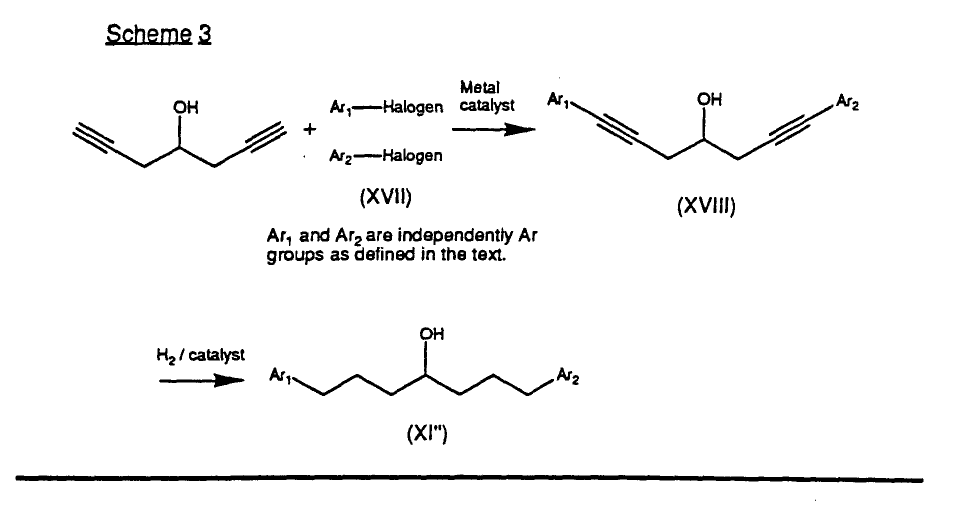

Alternatively (Scheme 3), 1,6-heptadiyn-4-ol can be coupled via a metal-catalyzed reaction to aromatic halides of formula (XVII) to give an alcohol of formula (XVIII). Subsequent hydrogenation provides an alcohol of formula (XI''), a preferred subset of alcohols of formula (XI).

Thus, this invention also provides a method for preparing compounds of formula (I') comprising the steps of:
(a) esterifying a protected amino acid of formula (X) with an alcohol of formula (XI) to give an intermediate of formula (XII) ;
(b) deprotecting the amino protecting group in the intermediate of formula (XII) to give an amino ester of formula (XIII); and
(c) acylating the free amino group in the compound of formula (XIII) with a compound of formula (XIV) or an activated derivative thereof.

It should be appreciated by those of ordinary skill in the art that a large variety of compounds of formula (I) may be readily prepared, according to the processes illustrated in synthetic Schemes 1, 2 and 3. The same processes may be used for the synthesis of many different end-products, by altering the variables in the starting materials.

For example, compounds of formula (I'') (not shown) wherein A is NH or N-(C1-C4 alkyl) can be synthesized by a peptide coupling reaction between a carboxylic acid of formula (X) and an amine of formula (XI''') (not shown) to give an amide of formula (XII'). This step is analogous to the first esterification reaction of Scheme 1. The steps leading from (XII') to (I'') are also analogous to those from (XII) to (I') shown in Scheme 1.

Optically active compounds of formula (I) may also be prepared using optically active starting materials, thus obviating the need for resolution of enantiomers or separation of diastereomers at a late stage in the synthesis.

It will also be appreciated by those of ordinary skill in the art that the above synthetic schemes are not intended to comprise a comprehensive list of all means by which the compounds or the intermediates of this invention may be synthesized. Further methods or modifications of the above general schemes will be evident to those of ordinary skill in the art.

The compounds of this invention may be modified by appending appropriate functionalities to enhance selective biological properties. Such modifications are known in the art and include those which increase biological penetration into a given biological system (e.g., blood, lymphatic system, central nervous system), increase oral availability, increase solubility to allow administration by injection, alter metabolism and alter rate of excretion.

The compounds of this invention are characterized by the ability to increase, restore or maintain the sensitivity of MDR cells to cytotoxic compounds, such as, for example, those typically used in chemotherapy. Based on that ability, the compounds of this invention are advantageously used as chemosensitizing agents, to increase the effectiveness of chemotherapy in individuals who are afflicted with drug-resistant cancers, tumors, metastases or disease. In addition, the compounds of this invention are capable of maintaining sensitivity to therapeutic or prophylactic agents in non-resistant cells. Therefore, the compounds of this invention are useful in treating or preventing multi-drug resistance in a patient. The term "patient" as used herein refers to mammals, including humans, and the term "cell" refers to mammalian cells, including human cells.

As used herein, the terms "sensitizing agent", "sensitizer", "chemosensitizing agent", "chemosensitizer" and "MDR modifier" denote a compound having the ability to increase or restore the sensitivity of an MDR cell, or to maintain the sensitivity of a non-resistant cell, to one or more therapeutic or prophylactic agents. The term "MDR sensitization" and "sensitization" and "resensitization" refer to the action of such a compound in maintaining, increasing, or restoring drug sensitivity.

According to one embodiment of this invention, compounds of this invention that are useful in increasing, restoring or maintaining drug sensitivity are also capable of binding to the protein FKBP-12 or other related FK-506 binding proteins such as FKBP-13, FKBP-26 and FKBP-52. In vitro tests (data not shown) of these compounds demonstrate that the agents bind to FKBP-12. Thus, this invention also comprises a class of chemosensitizing agents other than FK-506, characterized by the ability to bind to the FK binding protein-12 or related FK binding proteins, pharmaceutical compositions including such agents and a physiologically acceptable adjuvant, carrier or vehicle, and methods of using those compositions for treating or preventing multi-drug resistance in a patient.

Preferred compounds suitable for use in preventing or modulating multi-drug resistance are those which are not significantly immunosuppressive at clinically useful or prophylactically or therapeutically active levels -- i.e., the effect, if any, of immunosuppression does not outweigh the value of sensitization activity of the compound to the patient. Such immunosuppressive capabilities can be ascertained by the in vitro assays set forth in United States patent application Serial Nos. 07/547,814 (now United States patent 5,192,773), 07/704,734, 07/697,785 and 07/881,152, the disclosures of which are incorporated herewith.

The compounds of the present invention may be used in the form of pharmaceutically acceptable salts derived from inorganic or organic acids and bases. Included among such acid salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate. Base salts include ammonium salts, alkali metal salts, such as sodium and potassium salts, alkaline earth metal salts, such as calcium and magnesium salts, salts with organic bases, such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The compounds of the present invention may be administered orally, parenterally, by inhalation spray, topically, rectally, nasally, buccally, vaginally or via an implanted reservoir in dosage formulations containing conventional non-toxic pharmaceutically-acceptable carriers, adjuvants and vehicles. The term "parenteral" as used herein includes subcutaneous, intravenous, intramuscular, intra-articular, intra-synovial, intrasternal, intrathecal, intrahepatic, intralesional and intracranial injection or infusion techniques.

The pharmaceutical compositions of this invention comprise any of the compounds of the present invention, or pharmaceutically acceptable salts thereof, with any pharmaceutically acceptable carrier, adjuvant or vehicle. Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

According to this invention, the pharmaceutical compositions may be in the form of a sterile injectable preparation, for example a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as do natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

The pharmaceutical compositions of this invention may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These can be prepared by mixing the agent with a suitable non-irritating excipient which is solid at room temperature but liquid at the rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of active ingredient that may be combined with the carrier materials to produce a single dosage form will vary depending upon the host treated and the particular mode of administration. It should be understood, however, that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredient may also depend upon the therapeutic or prophylactic agent, if any, with which the ingredient is co-administered. As used herein, the term "pharmaceutically effective amount" refers to an amount effective to prevent multi-drug resistance or maintain, increase or restore drug sensitivity in MDR cells.

Dosage levels of between about 0.01 and about 100 mg/kg body weight per day, preferably between about 0.5 and about 50 mg/kg body weight per day of the active ingredient compound are useful. A typical preparation will contain between about 5% and about 95% active compound (w/w). Preferably, such preparations contain between about 20% and about 80% active compound.

When the compounds of this invention are administered in combination therapies with other agents, they may be administered sequentially or concurrently to the patient. Alternatively, pharmaceutical or prophylactic compositions according to this invention may comprise a combination of a compound of this invention and another therapeutic or prophylactic agent.

For example, the compounds may be administered either alone or in combination with one or more therapeutic agents, such as chemotherapeutic agents, (e.g., actinomycin D, doxorubicin, vincristine, vinblastine, etoposide, amsacrine, mitoxantrone, tenipaside, taxol and colchicine) and/or a chemosensitizing agent (e.g., cyclosporin A and analogs, phenothiazines and thioxantheres), in order to increase the susceptibility of the MDR cells within the patient to the agent or agents.

In order that this invention may be more fully understood, the following examples are set forth. These examples are for the purpose of illustration only and are not to be construed as limiting the scope of the invention in any way.

### Examples

### General Methods

Proton nuclear magnetic resonance (¹H NMR) spectra were recorded at 500 MHz on a Bruker AMX 500. Chemical shifts are reported in parts per million (δ) relative to Me₄Si (δ 0.0). Analytical high performance liquid chromatography was performed on either a Waters 600E or a Hewlett Packard 1050 liquid chromatograph.

### Example 1

### Synthesis of (S)-1,7-Diphenyl-4-heptanyl N-(3,4,5-trimethoxyphenylglyoxyl)pipecolate (3)

4-Phenyl-1-butyraldehyde (119). To a solution of 3.2 mL (20.8 mmol) of 4-phenyl-1-butanol (Aldrich Chemical Co.) in 20 mL of CH₂Cl₂ at 0 °C was added 3.2 g of powdered 3 Å molecular sieves and then 5.37 g (24.9 mmol) of pyridinum chlorochromate (PCC). The resulting suspension was stirred at 0 °C for 1 h at which time an additional 2.16 g (10.0 mmol) of PCC was added and the reaction mixture was warmed to room temperature. After stirring at ambient temperature for 0.5 h, the reaction mixture was diluted with ether and filtered through celite to give 2.5 g of the crude product. Flash chromatography (elution with 5% ethyl acetate in hexane) yielded 700 mg of the aldehyde 119. ¹H NMR was consistent with the structure.

3-Phenyl-1-propylmagnesium bromide (120). To a suspension of 736 mg (30.3 mmol) of magnesium turnings in 50 mL of THF at room temperature was added 50 µL of 1,2-dibromoethane followed by the dropwise addition of 5.5 g (25.1 mmol) of 1-bromo-3-phenylpropane (Aldrich Chemical Co.). After stirring at room temperature for 0.5 h, the supernatant was transfered via cannula to a 100 mL storage vessel and subsequently used as a 0.5 M THF solution of the Grignard reagent 120.

1,7-Diphenyl-4-heptanol (121). To a solution of 700 mg (4.7 mmol) of 4-phenyl-1-butanal (119) in 5.0 mL of THF at 0 °C was added 10.0 mL (5.0mmol) of 3-phenyl-1-propylmagnesium bromide (120) and the resulting mixture was stirred at 0 °C for 0.5 h. The mixture was then quenched by the dropwise addition of saturated NH₄Cl and diluted with ether. The phases were separated and the organic layer was washed with water and brine and then dried over MgSO₄. Concentration gave 1.12 g of the alcohol 121 as an oil. ¹H NMR spectrum was consistent with the structure.

CS)-Boc-1-Pipecolyl-1,7-dipenyl-4-heptanyl ester (122). To a solution of 164 mg (0.72 mmol) of Boc-L-Pipecolic acid in 5.0 mL of CH₂Cl₂ at room temperature was added 174 mg (0.65 mmol) of alcohol 121, 140 mg (0.72 mmol) of 1-(3-dimethylaminopropyl)-3-ethylcarbodiimide hydrochloride (EDC) and a catalytic amount of N,N-dimethylaminopyridine (DMAP). The reaction mixture was stirred at ambient temperature for 0.5 h and then applied directly to a silica gel column. Elution with 10% ethyl acetate in hexane afforded 76.2 mg of the ester 122 as an oil. ¹H NMR spectrum was consistent with the structure.

(*S*)-1,7-Diphenyl-4-heptanylpipecolate (123). To a solution of 47 mg (0.10 mmol) of the ester 122 in 1.0 mL of CH₂Cl₂ at ambient temperature was added 1.0 mL of trifluoroacetic acid. After stirring at room temperature for 0.5 h, the resulting solution was neutralized by the dropwise addition of saturated K₂CO₃. The layers were separated and the organic phase was washed with water, dried over MgSO₄ and concentrated to yield 23 mg of the amine 123 as an oil. ¹H NMR consistent with structure.

3,4,5-Trimethoxybenzoylformic acid (124). To a solution of 9.2 g (43.4 mmol) of 3,4,5-trimethoxyacetophenone (Aldrich Chemical Co.) in 35 mL of pyridine was added 6.3 g (56.7 mmol) of selenium dioxide and the resulting solution was heated at reflux overnight. The reaction mixture was cooled to room temperature, filtered through celite and concentrated to yield a dark brown oil which was dissolved into ethyl acetate and washed with 1.0 N HCl and then with saturated NaHCO₃. The basic aqueous layer was diluted with ether and acidified with concentrated HCl. The layers were separated and the organic phase was washed with brine and then dried over Na₂SO₄ to give 8.4 g of the acid 124 as a pale yellow solid. ¹H NMR consistent with structure.

(*S*)-1,7-Diphenyl-4-heptanyl N-(3,4,5-trimethoxyphenylglyoxyl)pipecolate (3). To a solution of 23 mg (0.06 mmol) of the amine 123 in 1.0 mL of CH₂Cl₂ at room temperature was added 21.8 mg (0.09 mmol) of the acid 124 and then 17.9 mg(0.09 mmol) of EDC and the resulting solution was stirred at room temperature for 0.5 h and applied directly to a silica gel column. Elution with 15% ethyl acetate in hexane gave 8.4 mg of the amide 3 as a mixture of rotamers. ¹H NMR (500MHz CDCl₃ δ 7.35-7.06(m), 5.32 (br s), 5.00 (br s), 4.88 (br s), 4.58 (d), 4.31 (br s), 3.95 (s), 3.89 (s), 3.44 (d), 3.21 (t), 3.04 (t), 2.54 (br s), 2.51 (br s), 2.42 (br s), 2.30 (d), 2.15 (d), 1.83-1.21 (m).

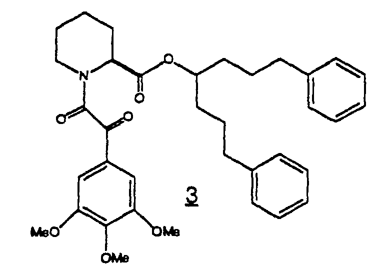

### Example 2

### Synthesis of (R and S)-1-(3-Phenoxy)phenyl-4-phenyl-1-butyl (S)-N-(3,4,5-trimethoxyphenylglyoxyl)pipecolate (4).

3-Phenoxybenzaldehyde (125). To a solution of 1.8 mL (10.3 mmol) of 3-phenoxybenzyl alcohol (Aldrich Chemical Co.) in 20 mL of CH₂Cl₂ at room temperature was added 1.5 g of powdered 4 Å molecular sieves and 2.5 g of activated MnO₂. The resulting suspension was stirred at room temperature for 0.5 h, at which time an additional 2.5 g of MnO₂ was added. After stirring at room temperature for 0.5 h the reaction mixture was filtered through celite to give 1.84 g of the aldehyde 125 as an oil. ¹H NMR consistent with structure.

(*R* and *S*)-1-(3-Phenoxy)phenyl-4-phenyl-1-butanol (126). The alcohol 126 was prepared from 190 mg (0.96 mmol) of aldehyde 125 and 2.0 mL (1.0 mmol) of the Grignard reagent 120 in 2.0 mL of THF as described above for the synthesis of the alcohol 121 in Example 1. Flash chromatography (elution with 10% ethyl acetate in hexane) afforded 108 mg of the racemic alcohol 126. ¹H NMR consistent with structure.

(*S*)-N-3,4,5-(Trimethoxyphenyl)glyoxyl pipecolic acid (127). To a slurry of 953.3 mg (3.4 mmol) of the tartrate salt of (*S*)-pipecolic acid (Egbertson, M. and Danishefsky, S. J. J. Org. Chem. 1989, 54, 11) in 7.0 mL of CH₂Cl₂ at 0 °C was added 3.9 mL (22.39 mmol) of diisopropylethylamine and 2.4 mL (18.9 mmol) of chlorotrimethylsilane and the resulting solution was allowed to stir at 0 °C for 0.5 h. In a separate reaction flask 450 µL (5.2 mmol) of oxalyl chloride and three drops of DMF were added to a solution of 820 mg (3.4 mmol) of acid 124 in 7.0 mL of CH₂Cl₂. After the evolution of gas ceased, the entire contents of the second flask were added to the first reaction vessel and the resulting mixture was allowed to stir at room temperature for 1 h. The reaction mixture was concentrated, dissolved into ether and washed with 0.5 N HCl and then saturated NaHCO₃. The basic aqueous phase was acidified with concentrated HCl and extracted with ether. The ethereal extracts were washed with water, brine, dried over MgSO₄ and concentrated to give 490 mg of the acid 127. ¹H NMR consistent with structure.

(*R* and *S*)-1-(3-Phenoxy)phenyl-4-phenyl-1-butyl (*S*)-N-(3,4,5-trimethoxyphenylglyoxyl)pipecolate (4). To a solution of 29.4 mg (0.08 mmol) of acid 127 in 2.0 mL of CH₂Cl₂ at room temperature was added 11 µL (0.13 mmol) of oxalyl chloride and three drops of DMF and the reaction mixture was allowed to stir at room temperature for 0.5 h and was then concentrated and suspended in 1.0 mL of benzene. To this suspension was added 32.0 mg (0.1 mmol) of alcohol 126 and 13.4 mg (0.1 mmol) of silver cyanide. The resulting mixture was heated at reflux overnight, cooled to room temperature and concentrated. Flash chromatography (elution with 10% ethyl acetate in hexane) gave 8.8 mg of the ester 4 as a mixture of diastereomers. ¹H NMR (500MHz CDCl₃) δ7.34-7.19 (m), 7.18-7.03 (m) 7.02-6.84 (m), 6.83-6.72 (m), 5.73 (q), 5.69-5.55 (m), 5.38 (t), 4.55 (br d), 4.35 (dd), 3.94 (s), 3.92 (s), 3.89 (s), 3.83 (s), 3.73 (s), 3.63 (s), 3.48-3.35 (m), 3.20 (t), 23.10 (t), 2.60 (q), 2.40 (dd), 1.95-1.91 (m), 1.90-1.45 (m).

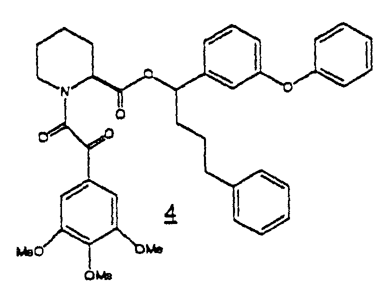

### Example 3

### Synthesis of (R and S)-6-Phenyl-1-(3-pyridyl)-3-hexyl (S)-N-(3,4,5-trimethoxyphenylglyoxyl)-pipecolate (7).

3-(3-Pyridyl)-1-propylaldehyde (128). To a solution of 2.3 g (5.46 mmol) of the Dess-Martin periodinane (Dess, D.B.; Martin, J.C. *J. Org. Chem.* 1983, *48,* 4155) in 10 mL of CH₂Cl₂ at 0 °C was added 470 µL (3.65 mmol) of 3-(3-pyridyl)-1-propanol and the resulting mixture was allowed to warm from 0 °C to ambient temperature over a 1.5 h period. To this solution was added 6.0 g (38.22 mmol) of Na₂S₂O₃ in saturated NaHCO₃ and the reaction mixture was allowed to stir at room temperature for 15 min. The reaction was extracted with CH₂Cl₂, dried over MgSO₄ and concentrated. Flash chromatography (elution with 3:1 hexane:acetone) yielded the product aldehyde 128 as an oil. ¹H NMR consistent with structure.

(*R* and *S*)-6-Phenyl-1-(3-pyridyl)-3-hexanol (129). The alcohol 129 was prepared from 125 mg (0.92 mmol) of aldehyde 128 and 2.0 mL (1.0 mmol) of 120 in 2.0 mL of THF as described above for the synthesis of alcohol 121 in Example 1 to give 221 mg of the crude alcohol 129. ¹H NMR consistent with structure.

(*S*)-Boc-Pipecolyl-(*R* and *S*)-6-Phenyl-1-(3-pyridyl)-3-hexyl ester (130). The ester 130 was prepared from 125 mg (0.49 mmol) of alcohol 129, 93 mg (0.41 mmol) of Boc-pipecolic acid, 94 mg (0.49 mmol) of EDC and a catalytic amount of DMAP in 1.0 mL of CH₂Cl₂ and 1.0 mL of DMF as described above for the synthesis of 122 in Example 1. Flash chromatography (elution with 2:1 hexane: ethyl acetate) gave 105 mg of the diastereomeric ester 130 as an oil. ¹H NMR consistent with structure.

(*R* and *S*)-6-Phenyl-1-(3-pyridyl)-3-hexyl (*S*)-pipecolate (131). The amine 131 was synthesized by treating 95 mg (0.20 mmol) of the ester 130 with 1.0 mL of trifluoroacetic acid in 3.0 mL of CH₂Cl₂ as described above for the preparation of amine 123 in Example 1, giving 58 mg of the diastereomeric amine 131 as an oil. ¹H NMR consistent with structure.

(*R* and *S*)-6-Phenyl-1-(3-pyridyl)-3-hexyl (*S*)-N-(3,4,5-trimethoxyphenylglyoxy)pipecolate (7). The ester 7 was prepared from 54 mg (0.15 mmol) of the amine 131, 50 mg (0.22 mmol) of the acid 124 and 42 mg (0.22 mmol) of EDC in 3.0 mL of CH₂Cl₂ as described above in the synthesis of ester 3 in Example 1. Flash chromatography (elution with 1:1 ethyl acetate:hexane) gave 73 mg of the diasteromeric ester 7 as a mixture of rotamers. ¹H NMR (500MHz CDCl₃) δ 8.48-8.42 (m), 7.50-7.41 (m), 7.32 (d), 7.27-7.03 (m), 5.38 (d), 5.31 (d), 5.06-5.01 (m), 4.97-4.93 (m), 4.60 (br d), 3.92 (s), 3.88 (s), 3.86 (s), 3.84 (s), 3.82 (s), 3.79 (s), 3.46 (br d), 3.27 (br t), 2.73-2.68 (m), 2.38-2.29 (m), 1.98-1.76 (m), 1.75-1.60 (m), 1.56-1.51 (m), 1.38-1.20 (m).

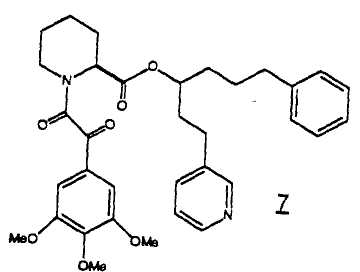

### Example 4

### Synthesis of (R and S)-(E)-1-[trans-(4-Hydroxycyclohexyl)]-2-methyl-6-phenyl-3-hex-1-enyl (S)-N-(3,4,5-trimethoxyphenylglyoxyl)pipecolate (8).

*cis*-and *trans*-4-(*tert*-Butyldimethylsilyloxy)-cyclohexan-1-ol (132) and (133). To a solution of 3.43 g (21.7 mmol) of *cis-* and *trans*-methyl 4-hydroxycyclohexane carboxylate (Noyce, D.S.; Denney, D.B. J. Am. Chem. Soc. Vol. *74,* 5912 (1952)) in 45 mL of methylene chloride at 0 °C was added 3.0 mL (26.0 mmol) of 2,6-lutidine followed by 5.5 mL (23.0 mmol) of *tert*butyldimethylsilyl trifluoromethanesulfonate. The ice bath was removed and the reaction mixture was allowed to stir at 25 °C for 2 h, at which time the solution was poured into saturated sodium bicarbonate. The layers were partitioned and the organic layer was washed with saturated copper sulfate and water and then dried over MgSO₄ to give 5.9 g of the crude methyl esters. A solution of 5.72 g (21.0 mmol) of this mixture in 45 mL of anhydrous THF was treated with 400 mg (10.5 mmol) of lithium aluminum hydride. The reaction mixture was stirred at 25 °C for 0.5 h and was then quenched by the slow addition of a saturated solution of Rochelle's salt. The mixture was diluted with ether, the layers were partitioned and the aqueous layer was washed twice with ethyl acetate. The combined organic extracts were dried over MgSO₄ and concentrated to give 4.9 g of the diastereomeric alcohols. Flash chromatography (elution with 1:5 ethyl acetate-hexane) gave 650 mg of 132, 1.10 g of 133 and 2.40 g of a mixture of the two. Data for 132: ¹H NMR (300 MHz, CDCl₃) δ 3.99-3.92(m), 3.46(d), 1.72-1.58 (m), 1.57-1.36(m), 0.86(s), 0.08(s). Data for 133: ¹H NMR (300 MHz, CDCl₃) δ 3.47(dddd), 3.38(d), 1.86-1.67(m), 1.47-1.16(m), 1.05-0.77 (m), 0.72(s), 0.02(s).

(*E*)-Ethyl 3-[*trans*-(4*-tert*-Butyldimethylsilyloxycyclohexyl)]-2-methylprop-2-enoate (134). To a -78 °C solution of oxalyl chloride (785 µL, 9.0 mmol) in 10 mL of methylene chloride was added dimethylsulfoxide (1.3 mL, 18.0 mmol). The resulting solution was stirred for 5 min and then 1.1 g (4.5 mmol) of the alcohol 133 was added in 10 mL of methylene chloride. The reaction mixture was stirred at -78 °C for 45 min at which time 3.8 mL (27.0 mmol) of triethylamine was added and the solution was allowed to warm to ambient temperature. The reaction was quenched with 1.0 N HCl and the aqueous layer was extracted with three portions of methylene chloride. The combined organic extracts were dried over MgSO₄ and evaporated to dryness to give 1.0 g of the intermediate aldehyde. A solution of this aldehyde (450 mg, 1.86 mmol) was treated directly with 710 mg (1.95 mmol) of (carbethoxyethylidene)triphenylphosphorane in 5.0 mL of methylene chloride. The resulting reaction mixture was stirred at ambient temperature overnight and was then poured into water. The layers were partitioned and the aqueous layer was extracted twice with methylene chloride. The combined organic layers were dried over MgSO₄ and concentrated to yield the enoate 134 containing a minor amount of the *Z* isomer. ¹H NMR consistent with structure.

(*E*)-3-[*trans*-(4-*tert*-Butyldimethylsilyloxycyclohexyl]-2-methylprop-2-en-1-ol (135). To a solution of 860 mg (2.6 mmol) of enoate 134 in 5.0 mL of anhydrous tetrahydrofuran at 25 °C was added 50 mg (1.3 mmol) of lithium aluminum hydride and the resulting mixture was allowed to stir for 30 min. The reaction was quenched by the slow addition of saturated Rochelle's salt and diluted with ethyl acetate. The layers were separated and the aqueous layer was extracted with two portions of ethyl acetate. The combined organic extracts were washed with both water and brine and then dried over MgSO₄. Evaporation and flash chromatography (elution with 15% ethyl acetate in hexane) gave 370 mg of the allylic alcohol 135. ¹H NMR consistent with structure.

(*E*)-3-[*trans*-(4-*tert*-Butyldimethylsilyloxycyclohexyl)]-2-methylprop-2-en-1-al (136). To a -78 °C solution of oxalyl chloride (105 µL, 1.2 mmol) in 1.0 mL of methylene chloride was added dimethylsulfoxide (170 µL, 2.4 mmol). The resulting solution was stirred for 5 min and then 170 mg (0.6 mmol) of the alcohol 135 was added in 1.0 mL of methylene chloride. The reaction mixture was stirred at -78 °C for 45 min at which time 500 µL (3.6 mmol) of triethylamine was added and the solution was allowed to warm to ambient temperature. The reaction was quenched with 1.0 N HCl and the aqueous layer was extracted with three portions of methylene chloride. The combined organic extracts were dried over MgSO₄ and evaporated to dryness to give the crude aldehyde 136 which was used directly in the next reaction. ¹H NMR consistent with structure.

(*R* and *S*)-(*E*)-1-[*trans*-(4-*tert*-Butyldimethylsilyloxycyclohexyl)]-2-methyl-6-phenylhex-1-en-3-ol (137). The alcohol 137 was prepared from the crude aldehyde 136 and 1.5 mL (0.75 mmol) of 120 in 2.0 mL of THF as described above for the synthesis of alcohol 121 in Example 1 to give 220 mg of the crude diastereomeric alcohol 137. Flash chromatography (elution with 20% ethyl acetate in hexane) afforded 146 mg of the alcohol 137 as an oil. ¹H NMR consistent with structure.

(*R* and *S*)-(*E*)-1-[*trans*-(4-*tert*-Butyldimethylsilyloxycyclohexyl)]-2-methyl-6-phenyl-3-hex-1-enyl (*S*)-N-(3,4,6-trimethoxyphenylglyoxyl)pipecolate (138). To a solution of 75.7 mg (0.22 mmol) of acid 127 in 2.5 mL of CH₂Cl₂ at room temperature was added 30 µL (0.34 mmol) of oxalyl chloride and three drops of DMF and the reaction mixture was allowed to stir at room temperature for 0.5 h and was then concentrated and suspended in 1.0 mL of benzene. To this suspension was added 43.4 mg (0.11 mmol) of alcohol 137 and 28.8 mg (0.22 mmol) of silver cyanide. The resulting mixture was heated at reflux overnight, cooled to room temperature and concentrated. Flash chromatography (elution with 4% acetone in hexane) gave 17.5 mg of the ester 138 as a mixture of diastereomers. ¹H NMR consistent with structure.

(*R* and *S*)-(*E*)-1-[*trans*-(4-Hydroxycyclohexyl)]-2-methyl-6-phenyl-3-hex-1-enyl (*S*)-N-(3,4,5-trimethoxyphenylglyoxyl)pipecolate (8). To a solution of 17.5 mg (0.02 mmol) of the ester 138 in 1.0 mL of CH₃CN at room temperature was added 10 drops of a 95:5 solution of CH₃CN:5% HF and the resulting mixture was stirred at room temperature for 0.5 h. The reaction mixture was neutralized with saturated K₂CO₃ and extracted into ether. The ether layers were washed with water, dried over MgSO₄ and concentrated to yield 7.2 mg of crude material. Flash chromatography (elution with 15% acetone in hexane) gave 4.9 mg of the diastereomeric alcohol 8 as a mixture of rotamers. ¹H NMR (500MHZ, CDCl₃) δ 7.38-7.02(m), 5.35-5.01(m), 4.62-4.53(m), 4.28(t), 3.95(s), 3.89(s), 3.87(s), 3.86(s), 3.85(s), 3.81(s), 3.55(m), 3.45(m), 3.20(m), 3.10-2.90(m), 2.60-2.45(m), 2.32(t), 2.10(t), 1.95(d), 1.85-1.40(m), 1.39-1.02(m).

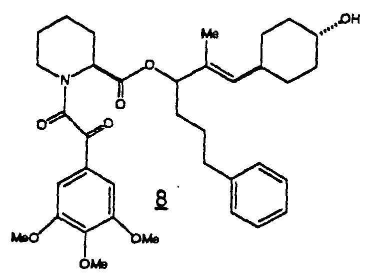

### Example 5

### Synthesis of (R and S)-5-(3-indolyl)-1-phenyl-2-pentyl (S)-N-(3,4,5-tri-methoxyphenylglyoxyl)pipecolate (11).

N-Methyl-N-Methoxy-4-(3-indolyl)butyramide (139). To a slurry of 1.75 g (8.61 mmol) of 3-indolebutyric acid (Aldrich Chemical Co.) in acetonitrile at room temperature was added 7.0 mL (40.2 mmol) of N,N-diisopropylethylamine, 3.8 g (21.5 mmol) of N,N-dimethylhydroxylamine hydrochloride and 4.19 g (9.5 mmol) of benzotriazol-1-yloxy-tris(dimethylamino)-phosphonium hexafluorophosphate (BOP reagent) and the resulting mixture was allowed to stir at room temperature overnight and was than concentrated to dryness. The residue was dissolved into ethyl acetate and washed with water, 0.5 N HCl, saturated NaHCO₃ and brine and then dried over MgSO₄ and concentrated. Flash chromatography (elution with a gradient of 2-10% ether in methylene chloride) provided 2.0 g of the amide 139. ¹H NMR consistent with structure.

Benzyl-3-(3-indolyl)propyl ketone (140). To a solution of 147 mg (0.60 mmol) of amide 139 in 4.0 mL of THF at -78 °C was added 1.31 mL (1.31 mmol) of benzylmagnesium chloride (1.0 M in Et₂O) and the reaction mixture was allowed to warm to room temperature and stir for 3 h. The reaction was quenched with 5% KHSO₄ and extracted into ether. The combined ethereal layers were washed with brine and dried over MgSO₄. Flash chromatography (elution with 25% ether in hexane) gave 108 mg of the ketone 140. ¹H NMR consistent with structure.

(*R* and *S*)-5-(3-indolyl)-1-phenyl-2-pentanol (141). To a slurry of 105 mg (0.38 mmol) of ketone 140 in 3.0 mL of MeOH at 0 °C was added 30 mg (0.79 mmol) of solid NaBH₄ and the resulting suspension was allowed to stir for 3 h. The reaction mixture was quenched with 5% KHSO₄ and extracted into ethyl acetate. The combined organic extracts were washed with brine and dried over MgSO₄. Flash chromatography (elution with 4% ether in methylene chloride) gave 81 mg of the alcohol 141 as a white solid. ¹H NMR consistent with structure.

(*S*)-Boc-Pipecolyl-(*R* and *S*)-5-(3-indolyl)-1-phenyl-2-pentyl ester (142). The ester 142 was prepared from 80 mg (0.29 mmol) of alcohol 141, 82 mg (0.36 mmol) of (S)-Boc-pipecolic acid, 66 mg (0.34 mmol) of EDC and a catalytic amount of 4. pyrrolidinopyridine in 2.0 mL of CH₂Cl₂ (mixture was allowed to stir overnight at room temperature) as described above for the synthesis of ester 122 in Example 1. Flash chromatography (elution with 4:10:26 ether: methylene chloride: hexane) gave 108 mg of the diastereomeric ester 142 as a white foam. ¹H NMR consistent with structure.

(*R* and *S*)-5-(3-indolyl)-1-thenyl-2-pentyl (*S*)-pipecolate hydrochloride salt (143). Anhydrous HCl was bubbled into a solution of 103 mg (0.21 mmol) of the ester 142 in 10 mL of EtOAc at -20 °C for 10 min and then the reaction mixture was purged with N₂. Concentration gave 108 mg of the crude amine 143 as the hydrochloride salt. ¹H NMR consistent with structure.

(*R* and *S*)-5-(3-indolyl)-1-phenyl-2-pentyl (*S*)-N-(3.4,5-trimethoxyphenylglyoxyl)pipocolate (11). To a slurry of 108 mg of the crude amino hydrochloride 143 in CH₃CN at room temperature was added 91 µL (0.52 mmol) of N,N-diisopropylethylamine, 76 mg (0.31 mmol) of acid 124, and 111 mg (0.25 mmol) of the BOP reagent and the resulting mixture was stirred at room temperature for two days and then was concentrated to dryness. The residue was reconstituted into 75 mL of ethyl acetate and then sequentially washed with water, 5% KHSO₄, saturated NaHCO₃ and brine and then dried over MgSO₄ and concentrated. Flash chromatography (elution with 4% ether in methylene chloride) gave 56.7 mg of the diastereomeric amide 11 as a rotameric mixture. ¹H NMR (500 MHz, CDCl₃) δ 7.98(d), 7.56(t), 7.38-6.73(m), 5.38-5.14(m), 3.90(m), 3.38(brt), 3.10 (brt), 2.97-2.60(m), 2.31(d), 2.10(d), 1.98-1.17(m), 0.8(m). R_{f} 0.51 (10% ether in methylene chloride).

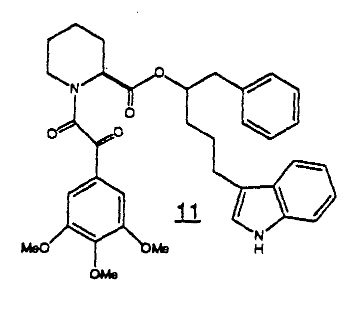

### Example 6

### Synthesis of (R and S)-2-Benzyl-4-phenyl-1-butyl (S)-N-(3,4,5-trimethoxyphenylglyoxyl)pipecolate (16).

(*R* and *S*)-2-Benzyl-4-phenyl-1-butyric acid (144). To a solution of 1.06 g (6.43 mmol) of 4-phenylbutyric acid in 20 mL of THF at 0 °C was added 193 mg (6.43 mmol) of solid NaH (60% in mineral oil). After stirring at 0 °C for 0.5 h, 3.2 mL (6.43 mmol) of lithium diisopropyl amide-THF complex (2.0 M) was added and the resulting red solution was stirred at 0 °C for 45 min. To this mixture was added 765 µL (6.43 mmol) of benzyl bromide and the solution was then allowed to stir overnight at room temperature. The reaction mixture was quenched by the slow addition of saturated NaHCO₃ and then washed with ether. The basic extracts were acidified with solid KHSO₄ and partitioned with ethyl acetate. The combined organic extracts were washed with brine, dried over MgSO₄ and concentrated to give 484 mg of the acid 144. ¹H NMR consistent with structure.

(*R* and *S*)-2-Benzyl-4-phenyl-1-butanol (145). To a solution of 469 mg (1.84 mmol) of acid 144 in 3.0 mL of THF at -78° 0 was added 2.03 mL (2.03 mmol) of lithium aluminum hydride (1.0 M in THF) and the resulting solution was allowed to warm to room temperature and stir overnight. The reaction mixture was quenched by the slow addition of Rochelle's salt and partitioned with ether. The combined ether extracts were washed with water and brine and dried over MgSO₄ and concentrated. Flash chromatography (elution with 2% ether in methylene chloride) afforded 264 mg of the alcohol 145. ¹H NMR consistent with structure.

(*S*)-Boc-Pipecolyl-(*R* and *S*)-2-Benzyl-4-phenyl-1-butyl ester (146). The ester 146 was prepared from 264 mg (1.10 mmol) of alcohol 145, 302 mg (1.32 mmol) of (S)-Boc-L-pipecolic acid, 253 mg (1.32 mmol) of EDC and a catalytic amount of 4-pyrrolidinopyridine in 2.0 mL of CH₂Cl₂ (mixture was allowed to stir at room temperature for 3 days) as described above for the synthesis of ester 122 in Example 1. Flash chromatography (elution with 1:5:14 ether: methylene chloride: hexane) gave 375 mg of the diastereomeric ester 146. ¹H NMR consistent with structure.

(*R* and *S*)-2-Benzyl-4-phenyl-1-butyl (*S*)-pipecolate hydrochloride salt (147). Anhydrous HCl was bubbled into a solution of 375 mg (0.83 mmol) of the ester 146 in 10 mL of EtOAc at -20 °C for 10 min and then the reaction mixture was purged with N₂. Concentration gave 352 mg of the crude amine 147 as the hydrochloride salt. ¹H NMR consistent with structure.

(*R* and *S*)-2-Benzyl-4-phenyl-1-butyl (*S*)-N-(3,4,5-trimethoxyphenylglyoxyl)pipecolate (16). To a slurry of 54 mg (0.14 mmol) of the crude amine hydrochloride 147 in 2.0 mL of CH₃CN at room temperature was added 60 µL (0.35 mmol) of N,N-diisopropylethylamine, 50 mg (0.21 mmol) of acid 124, and 73 mg (0.16 mmol) of the BOP reagent and the resulting mixture was stirred for 3 days at room temperature and was then concentrated to dryness. The residue was reconstituted into 75 mL of ethyl acetate and then sequentially washed with water, 5% KHSO₄, saturated NaHCO₄ and brine and then dried over MgSO₄ and concentrated. Flash chromatography (elution with 2% ether in methylene chloride) gave 52.7 mg of the diastereomeric amide 16 as a rotameric mixture. ¹H NMR (500 MHZ, CDCl₃ δ 7.21-7.01 (m), 5.41 (brs), 4.21 (dd), 4.08 (dd), 4.12 (d), 3.88 (d), 3.95 (s), 3.91 (s), 3.49 (d), 3.39 (dt), 2.80-2.62 (m), 2.38 (brt), 2.09 (br s), 1.87-1.20 (m). R_{f} 0.9 (1:3:26 Methanol: ether:methylene chloride).

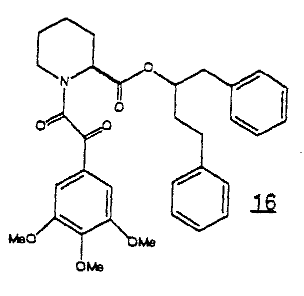

### Example 7

### Synthesis of (R and S)-1-Phenyl-7-(2-pyridyl)-4-heptyl (S)-N-(tert-butylglyoxyl)pipecolate (21).

(*E* and *Z*)-3-(1,3-Dioxan-2-yl)-1-(2-pyridyl)-1-propene (148 and 149). To a suspension of 4.6 g (10.2 mmol) of [2-(1,3-dioxan-2-yl)ethyl]triphenylphosphonium bromide (Aldrich Chemical Co.) in 50 mL of THF at 0 °C was added 6.4 mL (10.2 mmol) of n-butyl lithium (1.6 M in hexanes) and the resulting red solution was allowed to stir at 0 °C for 0.5 h. To this solution was added 880 µL (9.3 mmol) of 2-pyridinecarboxaldehyde (Aldrich Chemical Co.). The reaction mixture was allowed to stir at room temperature for 1 h and was then poured into water and partitioned with ether. The combined either extracts were dried over MgSO₄ and concentrated. Flash chromatography (elution with 3:1 hexane:ethyl acetate) gave 0.43 g of *E*-3-(1,3-dioxan-2-yl)-1-(2-pyridyl)-1-propene (148) and 1.12 g of *Z*-3-(1,3-dioxan-2-yl)-1-(2-pyridyl)-1-propene (149). ¹H NMRs consistent with structures.

1-(1,3-Dioxan-2-yl)-3-(2-pyridyl)propane (150). Through a suspension of 800 mg (4.2 mmol) of olefin 149 and 100 mg of 10% palladium on carbon was bubbled a steady stream of hydrogen gas for a period of 10 min. The reaction mixture was then filtered through celite and concentrated to give 805 mg of the acetal 150 as a colorless oil. ¹H NMR consistent with structure.

4-(2-Pyridyl)-1-butyraldehyde (151). A solution of 420 mg (2.2 mmol) of acetal 150 in 4.0 mL of THF and 3.0 mL of 4N HCl was stirred at room temperature for 1.5 h and was then neutralized by the slow addition of solid NaHCO₃. The reaction mixture was extracted with ethyl acetate, dried over MgSO₄ and concentrated to yield 288 mg of the aldehyde 151. ¹H NMR consistent with structure.

(*R* and *S*)-1-Phenyl-7-(2-pyridyl)-4-heptanol (152). The alcohol 152 was prepared from 288 mg (1.93 mmol) of aldehyde 151 and 2.3 mL (2.3 mmol) of 120 in 3.0 mL of THF as described above for the synthesis of alcohol 121 in Example 1 to give 520 mg of the crude alcohol 152. ¹H NMR consistent with structure.

(*S*)-Boc-Pipecolyl-(*R* and *S*)-1-Phenyl-7-(2-pyridyl)-4-heptyl ester (153). The ester 153 was prepared from 520 mg (1.93 mmol) of alcohol 152, 442 mg (1.93 mmol) of (*S*)-Boc-L-pipecolic acid, 370 mg (1.93 mmol) of EDC and a catalytic amount of DMAP in 4.0 mL of CH₂Cl₂ and 4.0 mL of DMF as described above for the synthesis of 122 in Example 1. Flash chromatography (elution with 3:1 hexane: ethyl acetate) gave 740 mg of the diastereomeric ester 153 as an oil. ¹H NMR consistent with structure.

(*R* and *S*)-1-Phenyl-7-(2-pyridyl)-4-heptyl (*S*)-pipecolate (154). The amine 154 was synthesized by treating 740 mg (1.54 mmol) of the ester 153 with 2.0 mL of trifluoroacetic acid in 5.0 mL of CH₂Cl₂ as described above for the preparation of 123 in Example 1 giving 580 mg of the diastereomeric amine 154 as an oil. ¹H NMR consistent with structure.

(*R* and *S*)-1-Phenyl-7-(2-pyridyl)-4-heptanol (*S*)-N-methyloxalylpipecolate (155). To a solution of 48 mg (0.13 mol) of the amine 154 in 1.0 mL of CH₂Cl₂ at 0 °C was added 33 µL (0.19 mmol) of N,N-diisopropylethylamine and 14 µL (0.15 mmol) of methyloxalyl chloride and the resulting solution was warmed to room temperature and allowed to stir overnight. The reaction mixture was diluted with ethyl acetate, washed with saturated NH₄Cl and brine, dried over MgSO₄ and then concentrated. Flash chromatography (elution with 25-30% ethyl acetate in hexane) gave 49 mg of the diastereomeric amide 155 as a mixture of rotamers. ¹H NMR consistent with structure.

(*R* and *S*)-1-Phenyl-7-(2-pyridyl)-4-heptanol (*S*)-N-(*tert*-butylglyoxyl)-pipecolate (21). To a solution of the amide 155 in 1.2 mL of THF at -78 °C was added tert-butyl lithium dropwise until TLC showed the consumption of the starting material. The reaction mixture was quenched with saturated NH₄Cl and partitioned with ethyl acetate. The combined organic extracts were washed with brine, dried over MgSO₄ and concentrated. Flash chromatography (elution with 30% ethyl acetate in hexane) gave the diasteromeric amide 21 as a mixture of rotamers. ¹H NMR (500 Mhz, CDCl₃) δ 8.50 (5), 7.57 (5), 7.20-7.05 (m), 5.23 (d), 5.18 (d), 4.56 (d), 4.44 (br d), 4.13 (d), 3.69 (br d), 3.37-3.28 (m), 3.13-3.00(m), 2.85-2.70 (m), 2.65-2.54 (m), 2.38-2.15 (m), 1.82-1.65(m), 1.56-1.44(m), 1.55-1.30(m), 1.27(s), 1.21 (s).

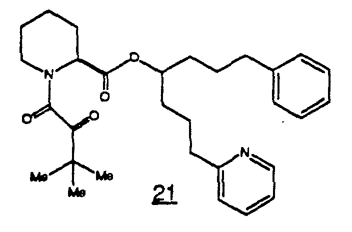

### Example 8

### Synthesis of (S)-1-[2-Oxo-2-(3,4,5-trimethoxyphenyl)acetyl]piperidine-2-carboxylic acid (R and S)-1-(3-phenylpropyl)-4-pyridin-3-yl-butyl ester (9).

(*E* and *Z*)-3-(1,3-Dioxan-2-yl)-1-(3-pyridyl)-1-propene (156). To a suspension of 9.9 g (22.4 mmol) of [2-(1,3-dioxan-2-yl)ethyl]triphenylphosphonium bromide (Aldrich Chemical Co.) in 50 mL of THF at 0 °C was added 14.0 mL (22.4 mmol) of butyl lithium (1.6 M in hexanes) and the resulting red solution was allowed to stir at 0 °C for 0.5 h. To this solution was added 1.8 mL (18.7 mmol) of 3-pyridinecarboxaldehyde (Aldrich Chemical Co.) and the reaction mixture was allowed to stir at room temperature for 1.5 h and was then poured into water and partitioned with ether. The combined ether extracts were dried over MgSO₄ and concentrated. Flash chromatography (elution with 2:1 hexane:ethyl acetate) gave 3.3 g of the alkene 156 as a mixture of olefin isomers. ¹H NMR consistent with structure.

1-(1,3-Dioxan-2-yl)-3-(3-pyridyl)propane (157). Through a solution of 3.2 g (16.7 mmol) of olefin 156 and 300 mg of 10% palladium on carbon was bubbled a steady stream of hydrogen gas for a period of 10 min. The reaction mixture was then filtered through celite and concentrated to give 2.8 g of the acetal 157 as a colorless oil. ¹H NMR consistent with structure.

4-(3-Pyridyl)-1-butyraldehyde (158). A solution of 1.5 g (7.8 mmol) of acetal 157 in 10.0 mL of THF and 10.0 mL of 4N HCl was stirred overnight at room temperature and was then neutralized by the slow addition of solid NaHCO₃. The reaction mixture was extracted with ethyl acetate, dried over MgSO₄ and concentrated to yield 1.1 g of the aldehyde 158. ¹H NMR consistent with structure.

(*R* and *S*)-1-Phenyl-7-(3-pyridyl)-4-heptanol (159). The alcohol 159 was prepared from 1.1 g (7.4 mmol) of aldehyde 158 and 8.1 mL (8.1 mmol) of 120 in 30.0 mL of THF as described above for the synthesis of 121 in Example 1 to give 1.9 g of the crude alcohol 159. ¹H NMR consistent with structure.

(*S*)-Boc-Pipecolyl-(*R* and *S*)-1-Phenyl-7-(3-pyridyl)-4-heptyl ester (160). The ester 160 was prepared from 1.65 g (6.12 mmol) of alcohol 159, 1.54 g (6.73 mmol) of (*S*)-Boc-pipecolic acid, 1.29 g (6.73 mmol) of EDC and a catalytic amount of DMAP in 8.0 mL of CH₂Cl₂ and 8.0 mL of DMF as described above for the synthesis of 122 in Example 1. Flash chromatography (elution with 2:1 hexane:ethyl acetate) gave 1.42 g of the diastereomeric ester 160 as an oil. ¹H NMR consistent with structure.

(*R* and *S*)-1-Phenyl-7-(3-pyridyl)-4-heptyl (*S*)-pipecolate (161). The amine 161 was synthesized by treating 1.42 g (2.95 mmol) of the ester 160 with 2.0 mL of trifluoroacetic acid in 8.0 mL of CH₂Cl₂ as described above for the preparation of 123 in Example 1 giving 1.02 g of the diastereomeric amine 161 as an oil. ¹H NMR consistent with structure.

(*R* and *S*)-1-Phenyl-7-(3-pyridyl)-4-heptyl (*S*)-N-(3,4,5-trimethoxy-phenylglyoxyl)pipecolate (9). The ester 9 was prepared from 995 mg (2.61 mmol) of the amine 161, 645 mg (2.87 mmol) of the acid 124 and 551 mg (2.87 mmol) of EDC in 6.0 mL of CH₂Cl₂ as described above in the synthesis of ester 3 in Example 1. Flash chromatography (elution with 3:1 acetone:hexane) gave 976 mg of the diasteromeric amide 9 as a mixture of rotamers. ¹H NMR consistent with structure.

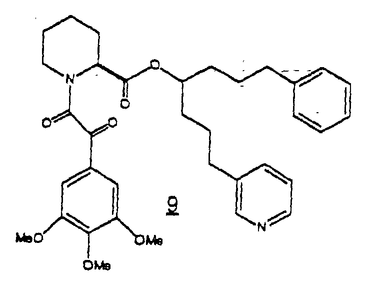

### Example 9

### Synthesis of (R and S)-1-Phenyl-7-(3-pyridyl)-4-heptyl (S)-N-(3,4,5-trimethoxyphenylglyoxyl)pipecolate N-oxide (22).

(*R* and *S*)-1-Phenyl-7-(3-pyridyl)-4-heptyl (*S*)-N-(3,4,5-trimethoxyphenylglyoxyl)pipecolate N-oxide (22). To a solution of 15 mg (0.02 mmol) of the amide 9 in 2.0 mL of CH₂Cl₂ at room temperature was added 9.3 µL (0.03 mmol) of 55% 3-chloroperoxybenzoic acid and the resulting solution was allowed to stir overnight at room temperature. Flash chromatography (elution with 100% acetone) gave 12.6 mg of the N-oxide 22 as a mixture of rotamers. ¹H NMR (500MHz CDCl₃) δ 8.10 (m), 7.46-7.02 (m), 5.88 (d), 5.80 (d), 5.06-5.00 (m), 4.95-4.89 (m), 4.61 (m), 4.31 (dd), 3.87 (s), 3.84 (s), 3.83 (s), 3.81 (s), 3.78 (s), 3.50 (br d), 3.27 (ddd), 3.12 (ddd), 3.00 (ddd), 2.67-2.49 (m), 2.32 (br d), 1.86-1.78 (m), 1.55-1.50 (m), 1.39-1.22 (m).

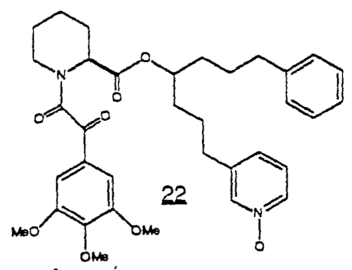

### Example 10

### Synthesis of (R and S)-1-Phenyl-7-purinyl-4-heptyl (S)-N-(3,4,5-trimethoxyphenylglyoxyl)pipecolate (25).

4-Chlorobutyraldehyde (162). To a solution of 19.1 g (0.16 mol) of 4-chloro-1-butanol (Aldrich Chemical Co.) in 50 mL of CH₂Cl₂ at 0 °C was added 1.0 g of powdered 4 Å molecular sieves and 38.7 g (0.18 mol) of pyridinium dichromate and the resulting suspension was stirred at 0 °C for 45 min. The reaction mixture was diluted with ether, filtered through celite and concentrated. The residue was vacuum distilled (bp 45-55 °C) to yield 5.0 g of the aldehyde 162 as an oil. ¹H NMR consistent with structure.

(*R* and *S*)-1-Chloro-7-phenyl-4-heptanol (163). The alcohol 163 was prepared from 182 mg (1.7 mmol) of aldehyde 162 and 1.9 mL (1.9 mmol) of 120 in 20.0 mL of THF as described above for the synthesis of 121 in Example 1 to give 128 mg of the alcohol 163 (flash chromatography in 100% methylene chloride). ¹H NMR consistent with structure.

(*S*)-Boc-Pipecolyl-(*R* and *S*)-1-Chloro-7-phenyl-4-heptyl ester (164). The ester 164 was prepared from 128 mg (0.56 mmol) of alcohol 163, 156 mg (0.68 mmol) of (*S*)-Boc-pipecolic acid, 1380 mg (0.68 mmol) of EDC and a catalytic amount of 4-pyrrolidinopyridine in 2.0 mL of CH₂Cl₂ as described above for the synthesis of 122 in Example 1. Flash chromatography (elution with 1:5:14 ether: methylene chloride: hexane) gave 159 mg of the diastereomeric ester 164. ¹H NMR consistent with structure.

(*S*) -Boc-Pipecolyl-(*R* and *S*)-1-Phenyl-7-purinyl-4-heptyl ester (165). To a solution of 34 mg (0.28 mmol) of purine in 3.0 mL of DMF at room temperature was added 8.4 mg (0.28 mmol) of solid NaH (80% in mineral oil) and the resulting solution was allowed to stir at room temperature for 10 min. To this reaction mixture was added 62 mg (0.14 mmol) of the ester 164 and 10 mg of NaCl and this mixture was stirred overnight at room temperature and then concentrated to dryness. The residue was dissolved into ethyl acetate, washed sequentially with water, saturated NaHCO₃, and brine and then dried over MgSO₄ and concentrated. Flash chromatography (elution with 15% 5:10:85 NH₄OH:MeOH:CH₂Cl₂ in CH₂Cl₂) gave 56 mg of the substituted purine 165 as an oil. ¹H NMR consistent with structure.

(*R* and *S*)-1-Phenyl-7-purinyl-4-heptyl (*S*)-pipecolate hydrochloride salt (166). Anhydrous HCl was bubbled into a solution of 53.7 mg (0.10 mmol) of the ester 165 in 10 ml of EtOAc at -20 °C for 10 min and then the reaction mixture was purged with N₂.
Concentration gave the crude amine 166 as the hydrochloride salt. ¹H NMR consistent with structure.

(*R* and *S*)-1-Phenyl-7-purinyl-4-heptyl (*S*)-N-(3,4,5-trimethoxyphenylglyoxyl)pipecolate (25). To a slurry of the crude amine hydrochloride 166 in CH₃CN at room temperature was added 45 µL (0.26 mmol) of N,N-diisopropylethylamine, 37 mg (0.15 mmol) of acid 124, and 54 mg (0.12 mmol) of the BOP reagent and the resulting mixture was stirred at room temperature for two days and then was concentrated to dryness. The residue was reconstituted into 75 mL of ethyl acetate and then sequentially washed with water, 5% KHSO₄, saturated NaHCO₃ and brine and then dried over MgSO₄ and concentrated. Flash chromatography (elution with 1:4:36 MeOH:Et₂O:CH₂Cl₂) gave 26.5 mg of the diastereomeric amide 25 as a rotameric mixture. ¹H NMR (500 MHz, CDCl₃) δ 9.11 (s), 8.95 (m), 8.09 (m), 7.36-7.05 (m), 5.31 (m), 4.28 (m), 3.90 (m), 3.46 (br t), 3.20 (m), 2.58 (m), 2.28 (br d), 2.17-1.18 (m), R_{f} 0.1 (30% ether in methylene chloride).

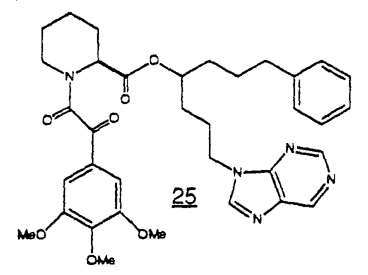

### Example 11

### Synthesis of (S)-1-[2-Oxo-2-(3,4,5-trimethoxyphenyl)-acetyl]piperidine-2-carboxylic acid (R and S)-4-[4-(morpholine-4-carbonyl)phenyl)-1-(3-phenylpropyl)butyl ester (44).

4-Formylbenzoic acid methyl ester (167). To a suspension of 9.6 g (63.6 mmol) of 4-carboxybenzaldehyde (Aldrich Chemical Co.) in 100 mL of CH₂Cl₂ at 0 °C was added excess trimethylsilyldiazomethane and the resulting mixture was allowed to stir at 0 °C for 1 h. The mixture was poured into saturated aqueous NaHCO₃ and extracted three times with ethyl acetate. The combined organic extracts were dried over MgSO₄, filtered and concentrated to give 4.3 g of the ester 167 as an oil. ¹H NMR consistent with the product.

(*E* and *Z*)-4-[3-[1,3]-Dioxolan-2-yl-propenyl]-benzoic acid methyl ester (168). The olefin was prepared from 4.3 g (26.2 mmol) of the aldehyde 167 13.94 g of [1-(1,3-dioxan-2-yl)ethyl]triphenylphosphonium bromide and 12.6 mL (32.0 mmol) of *n*-BuLi in 75 mL of THF as described for the synthesis of 156 in Example 8. Flash chromatography (elution with 10% ethyl acetate in hexane) gave 3.27 g of the olefin 168. ¹H NMR consistent with the product.

4-[3-[1,3]-Dioxolan-2-yl-propyl]benzoic acid methyl ester (169). The olefin 169 (3.21 g, 12.9 mmol) was hydrogenated over 328 mg of 10% Pd/C in 50 mL of EtOH as described for compound 157 in Example 8. Filtration and evaporation gave 2.85 g of 169 as an oil. ¹H NMR consistent with the product.

[4-(3-[1,3]-Dioxan-2-yl-propyl)phenyl]-methanol (170). To a solution of 2.85 g (11.4 mmol) of ester 169 in 25 mL of THF at 0 °C was added 4.4 mL (24.7 mmol) of diisobutylaluminum hydride and the resulting mixture was allowed to stir at 0 °C for 15 min. The reaction was quenched with saturated potassium sodium tartrate and extracted three times with ethyl acetate. The combined organic extracts were dried over MgSO₄, filtered and concentrated to yield 2.58 g of the crude alcohol 170 as an oil. ¹H NMR consistent with the product.

2-[3-(4-*tert*-Butyldiphenylsilyloxymethylphenyl)propyl]-[1,3]-dioxolane (171). To a solution of 2.58 g (11.6 mmol) of alcohol 170 and 1.19 g (17.5 mmol) of imidazole in 50 mL of CH₂Cl₂ was added 3.4 mL (13.1 mmol) of tert-butylchlorodiphenyl silane and the resulting mixture was allowed to stir at room temperature for 1 h. The mixture was then diluted with ethyl acetate and washed with 0.5 N HCl. The organic layer was dried over MgSO₄, filtered and concentrated. Flash chromatography (elution with 5% ethyl acetate in hexane) afforded 5.5 g of 171. ¹H NMR consistent with the product.

4-(4-*tert*-Butyldiphenylsilyloxymethylphenyl) butyraldehyde (172). To a solution of 5.5 g (11.9 mmol) of the dioxolane 171 in 40 mL of THF at room temperature was added 40 mL of 4.ON HCl and the resulting solution was allowed to stir for 1 h. The mixture was neutralized with solid K₂CO₃, extraced with ethyl acetate and concentrated. The crude mixture was dissolved into 25 mL of CH₂Cl₂ to which was added 600 mg (8.8 mmol) of imidazole and 1.9 mL (7.3 mmol) of tert-butylchlorodiphenyl silane. The resulting mixture was allowed to stir overnight at room temperature and was then poured into 0.5 N HCl and extracted with ethyl acetate. The organics were dried over MgSO₄, filtered and concentrated. Flash chromatography (elution with 8% ethyl acetate in hexane) gave 2.12 g of the aldehyde 172 as an oil. ¹H NMR consistent with the product.

1-(4-*tert*-Butyldiphenylsilyloxymethylphenyl)-7-phenyl-heptan-4-ol (173). The alcohol 173 was prepared from 2.12 g (5.0 mmol) of 172 and 9.0 mL (9 mmol) of 120 in 50 mL of THF as described for the synthesis of 121 in Example 1. Flash chromatography (elution with 10% ethyl acetate in hexane) gave 3.3 g of the alcohol 173. ¹H NMR consistent with the product.

(*S*)-Piperidine-1,2-dicarboxylic acid (*R* and *S*)-2-[4-(4-*tert*-butyldiphenylsilyloxymethylphenyl)-1-(3-phenylpropyl)butyl] ester 1-*tert*-butyl ester (174). The ester 174 was prepared from 3.3 g (6.15 mmol) of alcohol 173, 1.7 g (7.4 mmol) of (5)-Boc-pipecolic acid, 1.4 g (7.3 mmol) of EDC and a catalytic amount of DMAP in 35 mL of CH₂Cl₂ as described above for the synthesis of 122 in example 1. Flash chromatography (elution with 5% ethyl acetate in hexane) provided 2.4 g of the ester 174. ¹H NMR consistent with the product.

(*S*)-Piperidine-1,2-dicarboxylic acid 1-*tert*-butyl ester (*R* and *S*)-2-[4-(4-hydroxymethylphenyl)-1-(3-phenylpropyl)butyl] ester (175). To a solution of 750 mg (1.0 mmol) of the ester 174 in 10 mL of THF was added 1.1 mL (1.1 mmol) of a solution of tetrabutylammonium fluoride (1.0 M in THF) and the resulting mixture was allowed to stir at room temperature for 15 min. The mixture was diluted with ethyl acetate, washed with 5% KHSO₄, dried over MgSO₄ and concentrated. Flash chromatography (elution with 20% ethyl acetate in hexane) gave 308 mg of the alcohol 175. ¹H NMR consistent with the product.

(*S*)-Piperidine-1,2-dicarboxylic acid 1-*tert*-butyl ester (*R* and *S*)-2-[4-(4-carboxyphenyl)-1-(3-phenylpropyl) butyl] ester (176). To a solution of 326 mg (0.64 mmol) of the alcohol 175 in 3.0 mL of acetone was added 0.5 mL (1.27 mmol) of the Jones reagent and the resulting mixture was allowed to stir at room temperature for 1 h, and was then filtered through a pad of celite and concentrated. Flash chromatography (elution with 2% MeOH in CH₂Cl₂) gave 155 mg of the acid 176. ¹H NMR consistent with the product.

(*S*)-Piperidine-2-carboxylic acid (*R* and *S*)-4-(4-carboxyphenyl)-1-(3-phenylpropyl)butyl ester Trifluoroacetate salt (177). To a solution of 155 mg (0.3 mmol) of the acid 176 in 3.0 mL of CH₂Cl₂ was added 500 µL of trifluoroacetic acid and the resulting solution was allowed to stir at room temperature for 3 h at which time the volatiles were removed *in vacuo.* The crude residue was suspended in 5.0 mL of dry benzene and the volatiles were removed to yield an anhydrous sample of the salt 177.

(*S*)-1-[2-Oxo-2-(3,4,5-trimethoxyphenyl)-acetyllpiperidine-2-carboxylic acid (*R* and *S*)-4-(4-carboxyphenyl)-1-(3-phenylpropyl)butyl ester (178). To a suspension of 159 mg (0.3 mmol) of the salt 177 in 2.5 mL of CH₂Cl₂ at 0 °C was added 110 µL (0.63 mmol) of N,N-diisopropylethylamine and then 40 µL (0.31 mmol) of chlorotrimethylsilane and the resulting mixture was allowed to stir at 0 °C for 30 min. To this solution was added 85 mg (0.44 mmol) EDC and 106 mg (0.44 mmol) of the acid 124 and the reaction mixture was allowed to stir at room temperature overnight. The mixture was diluted with ethyl acetate and washed with 0.5N HCl, water, brine, dried over MgSO₄ and concentrated. Flash chromatography (elution with 30% MeOH in CH₂Cl₂) gave 97 mg of the product 178 as a mixture of rotamers. ¹H NMR consistent with the product.

(*S*)-1-[2-Oxo-2-(3,4,5-trimethoxyphenyl)-acetyl]piperidine-2-carboxvlic acid (*R* and *S*)-4-[4-(morpholine-4-carbonyl)phenyl]-1-(3-phenylpropyl)butyl ester (44). To a solution of 11.2 mg (17 µmol) of the acid 178 in 1.0 mL of CH₂Cl₂ was added 4.1 mg (21.4 µmol) of EDC and 1.8 mg (20.7 µmol) of morpholine and the resulting solution was allowed to stir overnight at room temperature. Flash chromatography (elution with 20% MeOH in CH₂Cl₂) gave 7.6 mg of the amide 44 as a mixture of rotamers. ¹H NMR (500MHz CDCl₃) δ 7.32 (d), 7.30 (d), 7.26 (s), 7.21-7.08 (m), 5.33 (m), 5.01 (m), 4.92 (m), 3.92 (s), 3.89 (s), 3.88 (s), 3.87 (s), 3.86 (s), 3.85 (s), 3.81-3.53 (m), 3.42 (brd), 3.29-3.21 (m), 3.05 (m), 2.61 (m), 2.42 (dd), 2.31 (d), 2.12 (m), 1.83 (m), 1.73-1.42 (m) , 1.42-1.20 (m).

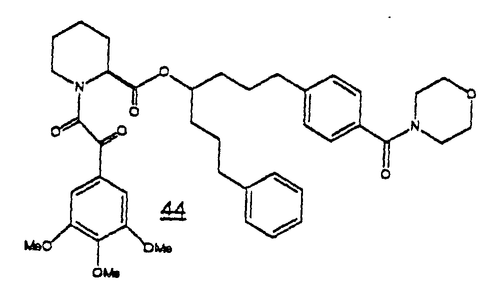

### Example 12 -- NMR DATA

We have prepared other compounds of formula (I) by methods substantially similar to those described in the above Examples 1-11 and those illustrated in Schemes 1-3. The NMR spectral data for these compounds are summarized below. Compounds are numbered according to the numbering scheme of Table 1.

Compound 2: ¹H NMR(500MHz CDCl₃), (mixture of diastereomers, mixture of rotamers) δ 8.42-8.33(m), 7.51(d), 7.42(d), 7.38(s), 7.31(d), 7.29-7.05(m), 5.01(s,br), 4.8(m), 4.71(m), 4.62(m), 3.92-3.83(m) 3.81(d), 3.60-3.51(m), 3.50-3.45(m), 2.65-2.51(m), 2.50-2.39(m), 2.38-2.22(m), 2.05(m), 1.95(m), 1.81-1.68(m), 1.67-1.49(m), 1.48-1.31(m), 1.22(s).

Compound 5: ¹H NNR(SOOMHz CDCl₃), (mixture of diastereomers, mixture of rotamers) δ 7.39-6.80(m), 6.75(d), 5.47(m), 4.55(m), 4.45(m), 3.95-3.78(m), 3.49-3.40(m), 3.22-3.11(m), 2.49-2.38(m), 1.88-1.67(m), 1.61-1.42(m), 1.37-1.14(m).

Compound 6: ¹H NMR(500MHz CDCl₃), (mixture of diastereomers, mixture of rotamers) δ 7.36-7.19(m), 7.18-7.02(m), 5.77(t), 5.65(m), 5.39(m), 4.60-4.52(m), 4.35(m), 3.93-3.82(m), 3.71-3.63(m), 3.48-3.42(m), 3.41-3.34(m), 3.28-3.19(m), 3.12-3.07(m), 2.65-2.58(m), 2.57-2.48(m), 2.42-2.31(m), 2.02-1.94(m), 1.91-1.21(m), 1.11-1.02(m)

Compound 10: ¹H NMR(500MHz CDCl₃), (mixture of diastereomers, mixture of rotamers) δ 7.35-6.98(m), 5.35(d), 5.3-5.14(m), 4.52(bd), 4.24(bs), 3.97-3.87(m), 3.49(t), 3.12(q), 3.00-2.56(m), 2.46(t), 2.32(d), 2.18(d), 2.11(d), 1.93(d), 1.83-1.56(m), 1.55-1.38(m), 1.32-1.18(m), 0.94-0.72(m).

Compound 12: ¹H NMR(500MHz CDCl₃), (mixture of diastereomers, mixture of rotamers) δ 7.37(s), 7.31-7.06(m), 6.98(d), 5.39(dd), 5.09-5.00(m), 4.99-4.93(m), 4.73(d), 4.38(m), 3.98-3.86(m), 3.91(s), 3.50(d), 3.34-3.24(m), 3.09(t), 2.73-2.16(m), 2.02-1.24(m).

Compound 13: ¹H NMR(500MHz CDCl₃), (mixture of diastereomers, mixture of rotamers) δ 7.38(s), 7.29-7.21(m), 7.20-7.03(m), 6.99(d), 6.88(d), 6.82-6.73(m), 5.40-5.32(m), 5.04-4.98(m), 4.97-4.91(m), 4.61(d), 4.37(d), 3.93-3.83(m), 3.81-3.74(m), 3.53-3.47(d,br), 3.32-3.22(m), 3.11-3.04(m), 2.65-2.12(m), 1.97-1.21(m).

Compound 14: ¹H NMR (500MHz CDCl₃), (mixture of diastereomers, mixture of rotamers) δ 8.04(d), 7.97(t), 7.59-7.48(m), 7.47-7.41(m), 7.31-7.22(m), 7.21-7.02(m), 6.98-6.91(m), 6.82-6.76(m), 5.43-5.38(m), 5.12-5.03(m), 4.93(m), 4.65-4.60(m), 4.38(m), 3.79(m), 3.53-3.48(m), 3.23(q), 3.11-2.99(m), 2.68-2.29(m), 2.19(t), 1.98-1.31(m).

Compound 15: ¹H NMR (500MHz CDCl₃), (mixture of diastereomers, mixture of rotamers) δ 8.05-7.92(m), 7.78(d), 7.47-7.03(m), 6.42(bs), 5.33(d), 5.01(m), 4.94(m), 4.59(bd), 4.32-4.14(m), 4.08-4.00(m), 3.97-3.84(m), 3.77-3.68(m), 3.45(bd), 3.17-3.08(m), 2.97(t), 2.60(t), 2.48(t), 2.35-2.21(m), 2.11(d), 2.05-1.10(m), 0.91-0.79(m).

Compound 17: ¹H NMR(500MHz CDCl₃), (mixture of diastereomers, mixture of rotamers) δ 7.38-6.92(m), 6.82-6.71(m), 5.38-5.29(m), 5.06-4.85(m), 4.60(d), 4.31(d), 3.94-3.81(m), 3.79-3.70(m), 3.51-3.41(m), 3.23(t,br), 3.06(t), 2.62-2.22(m), 2.15(d), 1.82-1.29(m).

Compound 18: ¹H NMR (500MHz CDCl₃), (mixture of diastereomers, mixture of rotamers) δ 8.55-8.38(m), 8.08-8.00(m), 7.98(d), 7.68(t), 7.59(t), 7.50-7.45(m), 7.45-7.41(m), 7.29-7.25(m), 7.25-7.08(m), 5.40(m), 5.11(m), 4.93(m), 4.61(brd), 4.38(m), 3.61(m), 3.51-3.46(m), 3.26-3.15(m), 3.08-2.96(m), 2.70-2.61(m), 2.58-2.49(m), 2.38(brd), 2.19(brd), 1.83-1.78(m), 1.78-1.59(m), 1.56-1.43(m), 1.41-1.24(m).

Compound 19: ¹H NMR(500MHz CDCl₃), (mixture of diastereomers, mixture of rotamers) δ 8.52-8.49(m), 8.04(d), 7.96(d), 7.64(t), 7.61-7.57(m), 7.52(t), 7.46-7.41(m), 7.26-7.22(m), 7.17(t), 7.12-7.08(m), 5.41(d), 5.12(m), 4.93(m), 4.61(brd), 4.38(d), 3.89-3.83(m), 3.67-3.61(m), 3.53-3.48(m), 3.28-3.19(m), 3.06-3.00(m), 2.83(brt), 2.72(brt), 2.65(brt), 2.52(brt), 2.48(brd), 2.21(brd), 1.89-1.73(m), 1.73-1.70(m), 1.70-1.48(m), 1.48-1.33(m).

Compound 20: ¹H NMR(500MHz CDCl₃), (mixture of diastereomers, mixture of rotamers) δ 8.50(d), 7.61(dd), 7.28-7.25(m), 7.21-7.16(m), 7.12(dd), 5.38(brd), 5.09-5.02(m), 4.93-4.90(m), 4.62(brd), 4.34(m), 3.94(s), 3.92(s), 3.91(s), 3.90(s), 3.89(s), 3.49(brddd), 3.28(ddd), 3.09(dd), 2.83(t), 2.74(m), 2.63(brd), 2.49(dd), 2.36(brd), 2.19(brd), 1.86-1.70(m), 1.70-1.62(m), 1.59-1.52(m), 1.48-1.23(m).

Compound 23: ¹H NMR(500MHz CDCl₃), (mixture of diastereomers, mixture of rotamers) δ 8.30(d), 8.28(d), 7.79(d), 7.34(s), 7.31-7.00(m), 6.43(s), 5.33(d), 5.06(d), 4.94(m), 4.59(d), 4.42-4.10(m), 4.04(s), 3.96(s), 3.94(s), 3.91(s), 3.81(s), 3.77(s), 3.48(d), 3.27(dt), 3.05(dt), 2.67-2.47(m), 2.32(d), 2.14(d), 2.03-1.22(m), 0.94-0.81(m).

Compound 26: ¹H NMR(500MHz CDCl₃), (mixture of diastereomers, mixture of rotamers) δ 7.32(d), 7.27-6.99(m), 5.34-5.28(m), 5.00(s,br), 4.61(d), 4.30(d), 3.92-3.81(m), 3.02(t), 2.54-2.48(m), 2.47-2.39(m), 2.34-2.22(m), 2.14(d), 1.82-1.14(m).

Compound 27: ¹H NMR(500MHz CDCl₃), (mixture of diastereomers, mixture of rotamers) δ 8.46-8.38(m), 7.68-7.50(m), 7.49-7.30(m), 7.29-7.08(m), 5.48(m), 5.16-5.02(m), 4.98-4.90(m), 4.60(d), 4.32(d), 3.51-3.42(m), 3.26-3.12(m), 3.11-2.98(m), 2.65-2.42(m), 2.32(d,br), 2.14(d,br), 1.83-1.22(m).

Compound 28: ¹H NMR (500MHz CDCl₃), (mixture of diastereomers, mixture of rotamers) δ 8.45-8.32 (m), 7.62-7.53(m), 7.52-7.43(m), 7.42-7.05(m), 6.09-5.98(m), 5.44-5.25(m), 5.09(s,br), 4.92(s,br), 4.64-4.51(m), 4.31(d), 3.50-3.41(m), 3.24-3.12(m), 3.07-2.94(m), 2.68-2.45(m), 2.32(d,br), 2.14(d,br), 1.83-1.26(m).

Compound 29: ¹H NMR(500MHz CDCl₃), (mixture of diastereomers, mixture of rotamers) δ 8.44-8.37(m), 7.58-7.51(m), 7.50-7.08(m), 5.35(t,br), 5.10(s,br), 4.93(s,br), 4.68-4.54(m), 4.32(d), 3.51-3.42(m), 3.25-3.12(m), 3.00(q), 2.69-2.45(m), 2.38-2.29(m), 2.14(d,br), 1.82-1.20(m).

Compound 30: ¹H NMR(500MHz CDCl₃), (mixture of diastereomers, mixture of rotamers) δ 7.35(s), 7.29-7.20(m), 7.19-7.02(m), 6.89(m), 6.77(m), 5.34(d), 5.03(m), 4.91(m), 4.61(d), 4.33(d), 3.95-3.88(m), 3.48(d), 3.31-3.21(m), 3.05(t,br), 2.87-2.43(m), 2.32(d,br), 2.18(d,br), 1.87-1.21(m).

Compound 31: ¹H NMR(500MHz CDCl₃), (mixture of diastereomers, mixture of rotamers) δ 8.00(s,br), 7.34(s,br), 7.31-7.02(m), 5.34(s,br), 5.31(s,br), 5.03(s,br), 4.92(d,br), 4.61(d,br), 4.33(s,br), 3.96-3.84(m), 3.48(d,br), 3.24(s,br), 2.76-2.42(m), 2.32(d,br), 2.15(m), 1.87-1.20(m).

Compound 32: ¹H NMR(SOOMHz CDCl₃), (mixture of diastereomers, mixture of rotamers) δ 7.38(d), 7.30-7.08(m), 7.07-7.03(d), 5.35-5.31(m), 4.98(m), 4.88(m), 4.59(m), 4.31(m), 3.97-3.86(m), 3.46(d,br), 3.29-3.18(m), 3.04(m), 2.65-2.42(m), 2.35-2.22(m), 1.83-1.14(m), 1.10(m).

Compound 33: ¹H NMR (500MHz CDCl₃), (mixture of diastereomers, mixture of rotamers) δ 7.38(d), 7.32-7.24(m), 7.24(d), 7.21(d), 7.01(s), 7.00(s), 6.02-5.99(m), 5.92-5.88(m), 5.38(d), 5.36(d), 4.70(ABq), 4.69(ABq), 4.64(ABq), 4.32(brd), 3.91(s), 3.89(s), 3.88(s), 3.74(s), 3.73(s), 3.48(brddd), 3.36(brd), 3.20(ddd), 3.06-2.97(m), 2.62(t), 2.58(t), 2.38(brd), 2.21(brd), 2.08-2.04(m), 1.90-1.74(m), 1.73-1.46(m), 1.38-1.33(m), 1.24(t).

Compound 34: ¹H NMR(500MHz CDCl₃), (mixture of diastereomers, mixture of rotamers) δ 7.33(s), 7.30(d), 7.29(s), 7.28-7.20(m), 7.18-7.11(m), 6.95-6.90(m), 6.83(d), 6.82(d), 6.31-6.28(m), 6.02-5.91(m), 5.43-5.40(m), 5.21(dd), 4.53(d), 3.91(s), 3.89(s), 3.86(s), 3.85(s), 3.84(s), 3.76(s), 3.71(s), 3.45(brddd), 3.40(brddd), 3.28(ddd), 3.15(ddd), 3.02(ddd), 2.62(dd), 2.40(brd), 1.94-1.89(m), 1.87-1.67(m), 1.65-1.50(m).

Compound 35: ¹H NMR(500MHz CDCl₃), (mixture of diastereomers, mixture of rotamers) δ 7.34-7.29(m), 7.28-7.11(m), 7.10-6.93(m), 5.35-5.28(m), 5.09-4.98(m), 4.90(m), 4.64-4.44(m), 4.30(m), 3.95-3.81(m), 3.46(t,br), 3.31-3.19(m), 3.03(m), 2.66-2.38(m), 2.34-2.25(m), 2.16(m), 1.85-1.19(m).

Compound 36: ¹H NMR(500MHz CDCl₃), (mixture of diastereomers, mixture of rotamers) δ 7.93-7.81(m), 7.78(s), 7.41-7.01(m), 5.32(s,br), 5.02(s,br), 4.90(m), 4.58(d), 4.31(s,br), 3.95-3.80(m), 3.45(d), 3.22(t), 3.05(m), 2.72-2.48(m), 2.47(d), 1.83-1.43(m), 1.42-1.18(m).

Compound 37: ¹H NMR(500MHz CDCl₃), (mixture of diastereomers, mixture of rotamers) δ 7.38(s), 7.30(s), 7.30-7.02(m), 7.01(s), 5.80-5.83(m), 5.68(dd), 5.62(dd), 5.38(d), 5.36(d), 4.66(s), 4.65(ABq), 4.54(s), 4.32(brd), 4.28(brd), 3.90(s), 3.88(s), 3.86(s), 3.85(s), 3.84(s), 3.78(s), 3.76(s), 3.43(brddd), 3.39(brddd), 3.24(ddd), 3.12(ddd), 3.06(ddd), 2.97(ddd), 2.62(t), 2.57(t), 2.48(brd), 2.24(brd), 2.01-1.94(m), 1.89-1.73(m), 1.72-1.65(m), 1.65-1.58(m), 1.52-1.49(m), 1.40-1.33(m), 1.12-1.08(m).

Compound 40: ¹H NMR(500MHz CDCl₃), (mixture of diastereomers, mixture of rotamers) δ 7;36(s), 7.29-7.19(m), 7.18-7.06 (m) , 6.89(m), 6.75(s), 5.32(s,br), 4.94(t), 3.95-3.84(m), 3.46(d,br), 3.22(m), 2.82(t), 2.61(t), 2.30(m)1 1.82-1.19(m).

Compound 41: ¹H NMR(500MHz CDCl₃), (mixture of diastereomers, mixture of rotamers) δ 7.37(d), 7.29-7.08(m), 7.04(d), 5.34(m), 4.97(m), 4.61(d), 4.33(m), 3.96-3.88(t), 3.86(d), 3.48(d), 3.25(m), 3.09(m), 2.65-2.52(m), 2.48(m), 2.32(d), 2.18(d), 1.86-1.49(m), 1.48-1.15(m).

Compound 42: ¹H NMR(500MHz CDCl₃), (mixture of diastereomers, mixture of rotamers) δ 7.34(d), 7.2(m), 7.13(m), 7.0-7.1(m), 5.87(m), 5.32(m), 5.22(dd), 5.12(dd), 5.0(m), 4.89(bm), 4.57(bd), 4.30(bm), 3.80-3.95(m), 3.45(bd), 3.40(m), 3.32(m), 3.22(dt), 3.05(bm), 2.60(m), 2.52(bm), 2.44(m), 2.30(m), 2.15(bm), 1.75(m), 1.60(m), 1.54(m), 1.20-1.45(bm).

Compound 43: ¹H NMR(500MHz CDCl₃), (mixture of diastereomers, mixture of rotamers) δ 7.36-7.30(m), 7.29-7.20(m), 7.19-7.04(m), 5.34(m), 5.01(s,br), 4.91(m), 4.59(d), 4.31(s,br), 3.95-3.86(m), 3.47(d,br), 3.25(t,br), 3.14-2.90(m), 2.68-2.52(m), 2.45(t), 2.32(d), 2.18(d), 1.85-1.46(m), 1.45-1.18(m).

Compound 45: ¹H NMR(500MHz CDCl₃), (mixture of diastereomers, mixture of rotamers) δ 7.35(d), 7.25(m), 7.15(m), 7.10(d), 7.05(d), 5.87(m), 5.38(bd), 5.34(m), 5.22(dd), 5.14(dd), 4.95(bm), 4.88(bm), 4.58(bd), 4.32(m), 3.82-3.95(m), 3.45(bd), 3.40(t), 3.25(m), 3.05(bm), 2.60(bm), 2.44(m), 2.34(bd), 2.18(bd), 1.78(m), 1.48-1.70(m), 1.20-1.45(m).

Compound 46: ¹H HMR(500MHz CDCl₃), (mixture of diastereomers, mixture of rotamers) δ 7.32(s), 7.25(m), 7.16(m), 7.10(t), 5.85(m), 5.50(dt), 5.38(dd), 5.25(dd), 5.18(d), 4.58(bm), 4.35(bm), 4.15(s), 4.06(d), 4.02(d), 3.85-3.95(m), 3.46(bd), 3.25(m), 3.08(bt), 2.98(bt), 2.65(t), 2.58(t), 2.53(t), 2.35(bt), 2.20(bd), 1.70-1.88(m), 1.50-1.70(m), 1.20-1.42(m).

Compound 47: ¹H NMR(500MHz CDCl₃), (mixture of diastereomers, mixture of rotamers) δ 7.44(d), 7.42-7.06(m), 5.45-5.30(m), 5.12-4.91(m), 4.03-3.83(m), 3.82-3.19(m), 2.72-2.26(m), 1.91-1.22(m)

Compound 48: ¹H NMR(500MHz CDCl₃), (mixture of diastereomers, mixture of rotamers) δ 7.34(d), 7.25(m), 7.20(d), 7.15(m), 7.10(d), 7.05(d), 5.88(m), 5.32(bt), 5.24(dd), 5.14(dd), 4.96(m), 4.86(m), 4.58(bd), 4.30(bm), 3.85-3.95(m), 3.45(bd), 3.38(t), 3.32(t), 3.25(m), 3.05(m), 2.60(m), 2.32(bd), 2.16(bd), 1.78(m), 1.48-1.72(m), 1.20-1.45(m).

Compound 49: ¹H NMR(500MHz CDCl₃), (mixture of diastereomers, mixture of rotamers) δ 7.28-7.42, 6.57-6.61(m), 6.45-6.51(m), 5.80-5.87(dd), 5.70-5.77(dd), 5.37-5.41(brd), 5.34-5.37(brd), 4.94-5.07(dd), 4.53-4.60(brd), 4.35-4.38(m), 3.80-3.95(m), 3.74(s), 3.38-3.50(brdd), 3.22-3.31(ddd), 3.15-3.22(ddd), 2.96-3.08(m), 2.32-2.44(brdd), 1.73-1.85(m), 1.48-1.75(m), 1.54-1.56(d), 1.15-1.48(m).

Compound 50: ¹H NMR(500MHz CDCl₃), (mixture of diastereomers, mixture of rotamers) δ 7.34(d), 7.24(m), 7.15(m), 7.10(d), 7.04(d), 5.85(m), 5.32(m), 5.22(dd), 5.15(m), 5.00(m), 4.58(bd), 4.30(bs), 3.74-3.95(m), 3.44(m), 3.25(bt), 3.04(bm), 2.62(m), 2.45(t), 2.30(bd), 2.18(bd), 1.88(m), 1.78(m), 1.46-1.72(m), 1.22-1.45(m).

Compound 51: ¹H NMR(500MHz CDCl₃), (mixture of diastereomers, mixture of rotamers) δ 7.34(s), 7.25(m), 7.20(d), 7.14(m), 7.10(d), 7.06(d), 5.87(m), 5.78(dt), 5.68(m), 5.45-5.60(m), 5.35(d), 5.24(m), 5.15(d), 4.58(bd), 3.85-3.96(m), 3.45(m), 3.24(m), 3.04(m), 2.62(m), 2.56(t), 2.49(dt), 2.34(dt), 2.18(bm), 1.48-1.82(m), 1.24-1.40(m).

Compound 52: ¹H NMR (500MHz CDCl₃), (mixture of diastereomers, mixture of rotamers) δ 7.40-7.03(m), 5.38-5.28(m), 5.02(s,br), 4.90(m), 4.60(d), 4.32(s,br), 3.99-3.87(m), 3.86-3.31(m), 3.30-3.21(t,br), 3.11-3.02(q,br), 2.69-2.50(m), 2.47(m), 2.32(d), 2.14(d), 1.89-1.48(m), 1.47-1.21(m).

Compound 53: ¹H NMR(500MHz CDCl₃), (mixture of diastereomers, mixture of rotamers) δ 7.40(d), 7.35(d), 7.30(d), 7.28(s), 6.60(d), 6.55(d), 6.52(t), 6.49(t), 5.86(q), 5.78(q), 5.42(d), 5.08(s), 4.64(bd), 4.35(m), 3.88-3.98(m), 3.46(bd), 3.21(dt), 3.05(dt), 2.36(bd), 2.18(bd), 1.80(m), 1.74(bd), 1.64(s), 1.56(d), 1.48-1.55(m), 1.40(d), 1.15-1.30(m).

Compound 54: ¹H NMR(500MHz CDCl₃), (mixture of diastereomers, mixture of rotamers) δ 8.52(m), 7.82-7.71(m), 7.70-7.62(m), 7.55-7.42(m), 7.38-7.01(m), 5.36-5.29(m), 5.01(m), 4.90(m), 4.79-4.67(m), 4.59(d), 4.39-4.11(m), 3.96-3.73(m), 3.44(d), 3.22(t), 3.09-3.00(q,br), 2.72-2.41(m), 2.30(d), 2.14(d), 1.86-1.43(m), 1.42-1.02(m), 0.98-0.73(m).

Compound 55: ¹H NMR(500MHz CDCl₃), (mixture of diastereomers, mixture of rotamers) δ 7.38(d), 7.33(d), 7.29-7.02 (m) , 5.32(m), 5.01(m), 4.90(m), 4.59(m), 4.30(m), 4.08-3.51(m), 3.46(d),3.29-3.18(m), 3.11-2.98(q,br), 2.81-2.32(m), 2.30(d), 2.14(d), 1.84-1.19(m).

Compound 56: ¹H NMR(500MHz CDCl₃), (single diastereomer, mixture of rotamers) δ 7.39-7.30(m), 7.27-7.20(brs), 7.20-7.15(brt), 7.14-7.06(brd), 5.81-5.78(brt), 5.77-5.72(brt), 5.34-5.30(brd), 5.28(s), 4.60-4.55(brd), 5.33(brs), 3.91(s), 3.88(s), 3.80(brs), 3.79-3.48(m), 3.47-3.30(brd), 3.28-3.20(brt), 3.01-2.94(brt), 2.66-2.60(t), 2.59-2.54(t), 2.42-2.35(brd), 2.25-2.19(brd), 2.04-1.93(m), 1.89-1.73(m), 1.72-1.65(m), 1.64-1.57(m), 1.54(brs), 1.39-1.25(m), 1.20(brs).

Compound 57: ¹H NMR(500MHz CDCl₃), (mixture of diastereomers, mixture of rotamers) δ 7.32(d), 7.31-7.01(m), 5.31(m), 5.00(m), 4.90(m), 4.59(m), 4.30(m), 3.93-3.83(m), 3.82-3.63(m), 3.49-3.38(m), 3.22(t), 3.10-2.98(t), 2.68-2.21(m), 2.12(m), 1.82-1.21(m).

Compound 58: ¹H NMR(500MHz CDCl₃), (mixture of diastereomers, mixture of rotamers) δ 7.33-7.01(m), 5.31(m), 4.99(m), 4.89(m), 4.59(d), 4.29(m), 3.92-3.84(m), 3.83-3.64(m), 3.55-3.28(m), 3.22(t), 3.04(m), 2.63-2.22(m), 2.14(d), 1.81-1.21(m).

Compound 59: ¹H NMR(500MHz CDCl₃), (mixture of diastereomers, mixture of rotamers) δ 7.71-7.52(m), 7.42(m), 7.39-7.04(m), 6.72-6.59(m), 5.32(m), 5.22(m), 5.11(m), 5.01(m), 4.99-4.90(m), 4.69-4.52(m), 4.39-4.26(m), 3.99-3.79(m), 3.46(t), 3.22(t), 3.11-2.94(m), 2.72-2.40(m), 2.29(t), 2.20-2.11(m), 1.88-1.19(m), 0.89(m).

Compound 60: ¹H NMR(500MHz CDCl₃), (mixture of diastereomers, mixture of rotamers) δ 8.53(m), 7.80(m), 7.72-7.53(m), 7.39-7.03(m), 5.36-5.28(dd), 5.12-4.98(m), 4.92(m), 4.79-4.52(m), 4.31(m), 3.98-3.81(m), 3.45(m), 3.31-3.19(q,br), 3.11-3.00(m), 2.72-2.43(m), 2.31(d), 2.20-2.11(m), 1.88-1.22(m).

Compound 61: ¹H NMR(500MHz CDCl₃), (mixture of diastereomers, mixture of rotamers) δ 8.45(s,br), 7.60-7.49(m), 7.38-7.21(m), 5.38-5.31(m), 5.03-4.98(m), 3.99-3.88(m), 3.50(d,br), 3.29(q), 2.65(m), 2.38-2.31(m), 1.88-1.13(m), 0.92-0.74(m).

Compound 62: ¹H NMR(500MHz CDCl₃), (mixture of diastereomers, mixture of rotamers) δ 8.55-8.65(m), 7.32-7.40(m), 6.80-7.00(m), 5.74-5.78(m), 5.62-5.71(m), 5.85-5.89(brd), 5.80-5.84(brd), 5.13-5.21(m), 5.03-5.10(m), 4.77-4.81(dd), 3.87-3.94(m), 3.80(s), 3.79(s), 3.72(s), 3.38-3.46(brdd), 3.14-3.28(m), 2.66-2.83(m), 2.48-2.58(m), 2.28-2.48(m), 1.32-1.18(m).

Compound 63: ¹H NMR(500MHz CDCl₃), (mixture of diastereomers, mixture of rotamers) δ 8.62(d), 8.61-8.58(m), 7.64(dd), 7.59(dd), 7.32-7.24(m), 7.12(d), 6.92(dd), 6.89-6.83(m), 6.82(d), 6.79(d), 6.74(d), 5.48(d), 5.07(d), 4.60(m), 4.44(brdd), 3.91(s), 3.90(s), 3.86(S), 3.84(s), 3.83(s), 3.78(s), 3.44(brd), 3.18(ddd), 2.92(ddd), 2.40(brt), 2.32(brt), 1.89-1.70(m), 1.62-1.48(m).

Compound 64: ¹H NKR(500MHz CDCl₃), (mixture of diastereomers, mixture of rotamers) δ 8.59(d), 8.58(d), 7.32-7.04(m), 6.99-6.80(m), 5.62(dd), 5.61(dd), 5.38(dd), 5.06(s), 5.02(d), 4.99(d), 4.53(m), 4.36(m), 3.91(s), 3.90(s), 3.89(s), 3.88(s), 3.84(s), 3.69(s), 3.61(s), 3.46(brd), 3.41(brd), 3.24(dd), 3.12(dd), 2.62(t), 2.58(t), 2.34(brt), 1.99-1.92(m), 1.86-1.42(m).

Compound 66: ¹H NMR(500MHz CDCl₃), (mixture of diastereomers, mixture of rotamers) δ 8.56-8.51(m), 7.35-7.28(m), 7.27-7.22(m), 7.14(s), 7.07(s), 6.93-6.88(m), 6.87-6.80(m), 6.79-6.71(m), 6.65-6.62(m), 5.81(q), 5.71(q), 5.32-5.27(m), 5.20-4.98(m), 4.57-4.47(m), 4.28-4.23(m), 3.92-3.70(m), 3.40(brd), 3.20(brd), 3.11(ddd), 3.00-2.89(m), 2.33(d), 2.26(d), 2.20(d), 2.07(d), 1.80-1.57(m), 1.56-1.25(m), 1.24-1.17(m), 1.13-1.00(m).

Compound 67: ¹H NMR(500MHz CDCl₃), (mixture of diastereomers, mixture of rotamers) δ 8.63-8.54(m), 8.53-8.44(m), 7.38-7.11(m), 7.10-6.99(m), 6.78(d), 6.72(dd), 6.63(dd), 6.53(d), 6.44(d), 6.14(dd), 6.08(dd), 6.00(dd), 5.88(dd), 5.39(d), 5.31(d), 5.23-4.95(m), 4.61-4.50(m), 4.32-4.29(m), 3.91(s), 3.90(s), 3.88-3.74(m), 3.71(s), 3.64-3.58(m), 3.47-3.38(m), 3.37-3.32(m), 3.24(ddd), 3.13(ddd), 3.07(ddd), 2.94(ddd), 2.62-2.45(m), 2.38-2.29(m), 2.20-2.11(m), 2.00-1.88(m), 1.87-1.40(m), 1.39-1.08(m).

Compound 68: ¹H NMR(500MHz CDCl₃), (single diastereomer, mixture of rotamers) 6 8.61(d), 7.38(d), 7.31(s), 7.28-7.22(m), 7.14(dd), 7.10(d), 7.04(d), 6.83(d), 5.23(dd), 5.14(dd), 5.36(d), 5.11(brs), 4.58(m), 4.31(m), 3.91(s), 3.90(s), 3.89(s), 3.88(s), 3.82-3.79(m), 3.78-3.64(m), 3.51-3.44(m), 3.40(brd), 3.26-3.10(m), 2.63(dd), 2.32(brd), 2.00-1.92(m), 1.88-1.40(m), 1.08-1.00(m).

Compound 69: ¹H NMR(500MHz CDCl₃), (mixture of diastereomers, mixture of rotamers) δ 8.60-8.57(m), 8.56-8.53(m), 7.38-7.35(m), 7.32-7.17(m), 6.53(s), 6.52(s), 5.83(q), 5.76(q), 5.38-5.32(m), 5.17-5.05(m), 4.67-4.60(m), 4.30-4.28(m), 4.13-4.08(m), 3.96-3.82(m), 3.80(s), 3.45(brd), 3.28(ddd), 2.97(ddd), 2.77-2.72(m), 2.53-2.43(m), 2.36-2.22(m), 2.15-1.92(m), 1.86-0.79(m).

Compound 70: ¹H NMR(500MHz CDCl₃), (mixture of diastereomers, mixture of rotamers) δ 8.59-8.43(m), 7.38-6.98(m), 6.65(s), 6.57(s), 6.53(m), 6.43(m), 5.88-5.84(m), 5.68-5.64(m), 5.63-5.59(m), 5.58-5.54(m), 5.35-5.28(m), 5.15-5.00(m), 4.99(d), 4.92(d), 4.58(d), 4.51(d), 4.33(d), 4.26(d), 3.89(s), 3.87(s), 3.83(s), 3.79(s), 3.72(s), 3.65(s), 3.45-3.37(m), 3.21(ddd), 3.10(ddd), 2.95-2.83(m), 2.62-2.42(m), 2.28(d), 2.21(d), 1.92-1.26(m), 1.17-1.12(m), 1.11-1.01(m).

Compound 71: ¹H NMR(500MHz CDCl₃), (single diastereomer, mixture of rotamers) δ 8.64(d), 7.35(d), 7.28(s), 6.60(d), 6.55(d), 6.52(t), 6.49(t), 5.86(q), 5.78(q), 5.42(d), 5.08(s), 4.64(bd), 4.35(m), 3.88-3.98(m), 3.46(bd), 3.21(dt), 3.05(dt), 2.36(bd), 2.18(bd), 1.80(m), 1.74(bd), 1.64(s), 1.56(d), 1.48-1.55(m), 1.40(d), 1.15-1.30(m).

Compound 72: ¹H NMR(500MHz CDCl₃), (single diastereomer, mixture of rotamers) δ 8.62(d), 7.35(d), 7.28(s), 6.60(d), 6.50(d), 6.45(t), 6.42(t), 5.85(q), 5.73(q), 5.40(d), 5.10(d), 5.04(d), 4.58(bd), 4.38(m), 3.92(s), 3.88(s), 3.82(s), 3.72(s), 3.50(bd), 3.30(dt), 3.01(dt), 2.40(bd), 2.30(bd), 1.85(m), 1.64(bs), 1.56(d), 1.48(d), 1.35-1.45(m).

Compound 73: ¹H NMR(500MHz CDCl₃), (mixture of diastereomers, mixture of rotamers) δ 8.55-8.65(brd), 7.32-7.42(brdd), 7.28(s), 7.20(s), 6.50-6.55(m), 5.72-5.87(m), 5.32-5.39(m), 5.05-5.17(m), 4.58-4.64(brd), 4.53-4.58(brd), 4.34-4.36(brd), 4.25-4.29(brd), 3.71-3.96(ms), 3.40-3.48(m), 3.23-3.30(ddd), 3.13-3.22(ddd), 2.17-2.37(m), 1.10-1.86(m), 1.48-1.52(d).

Compound 74: ¹H NMR(500MHz CDCl₃), (single diastereomer, mixture of rotamers) δ 8.62-8.58(d), 8.57-8.51(d), 7.38-7.35(d), 7.33-7.28(m), 7.27(s), 7.18(s), 6.61(s), 6.59(s), 5.65-5.60(t), 5.55-5.50(t), 5.40-5.36(d), 5.18-5.05(m), 4.67-4.63(brd), 4.33-4.30(d), 3.96(s), 3.93(s), 3.92(s), 3.87(s), 3.50-3.43(brd), 3.25-3.16(dt), 3.05-2.97(dt), 2.32-2.28(brd), 2.14-2.08(brd), 1.95-1.85(m), 1.84-1.64(m), 1.63-1.56(brd), 1.55-1.42(m), 1.35-1.23(m), 1.22-1.12(m), 0.92-0.83(t), 0.73-0.68(t).

Compound 75: ¹H NMR(500MHz CDCl₃), (single diastereomer, mixture of rotamers) δ 8.62-8.58(m), 8.57-8.53(d), 7.41-7.39(d), 7.38-7.35(d), 7.27(s), 7.23(s), 7.13(s), 6.61(s), 6.51(s), 5.60-5.55(t), 5.54-5.50(t), 5.39-5.35(d), 5.15(s), 5.14-5.10(m), 5.09(s), 5.07(s), 5.01(s), 5.00(s), 4.60-4.55(brd), 4.51-4.49(t), 4.40-4.38(brd), 3.90(s), 3.85(s), 3.80(s) 3.73(s), 3.48-3.43(brd), 3.30-3.22(dt), 2.95-2.88(dt), 2.38-2.32(brd), 2.27-2.22(brd), 1.90-1.70(m), 1.69-1.62(brd), 1.59-1.50(m), 1.46-1.35(m), 1.26(s), 0.90-0.85(t), 0.82-0.78(t).

Compound 76: ¹H NMR(500MHz CDCl₃), (mixture of diastereomers, mixture of rotamers) δ 8.95(s), 8.80(d), 8.55(m), 8.50(m), 7.34(s), 7.30(s), 7.28(s), 6.76(s), 6.73(s), 5.85(q), 5.77(q), 5.40(m), 5.20-5.35(m), 4.60(m), 4.35(m), 3.85-3.98(m), 3.80(s), 3.48(bt), 3.18-3.30(m), 3.00(m), 2.40(bd), 2.32(bd), 2.26(bd), 1.65-1.90(m), 1.60(s), 1.55(dd), 1.48(d), 1.40(m), 1.12(m).

Compound 77: ¹H NMR(500MHz CDCl₃), (mixture of diastereomers, mixture of rotamers) δ 8.43-8.53(m), 7.20-7.56(m), 7.04(s), 7.01(s), 6.75-6.92(m), 6.62(brs), 5.78-5.85(m), 5.68-5.77(m), 5.80-5.84(brd), 5.02-5.12(m), 3.76-4.00(m), 3.64-3.76(m), 3.49-3.60(m), 3.38-3.49(m), 3.32-3.34(d), 3.21-3.27(m), 3.02-3.18(m), 2.73-2.82(m), 2.37-2.53(m), 2.24-2.32(m), 2.20(s), 2.15(s), 1.27-1.72(m), 1.07-1.22(m), 0.92-0.97(dd), 0.82-0.86(dd).

Compound 78: ¹H NMR(500MHz CDCl₃), (single diastereomer, mixture of rotamers) δ 8.65-8.56(d), 8.55-8.51(d), 7.40-7.35(d), 7.34-7.20(m), 7.16(s), 6.70-6.60(m), 6.21-6.18(d), 6.15-6.11(d), 5.97-5.88(m), 5.83-5.75(m), 5.45-5.40(d), 5.32(s), 5.28(s), 5.27(s), 5.21-5.18(m), 5.13(s), 5.11(s), 4.67-4.61(brd), 4.51-4.49(d), 4.35-4.33(d), 4.05-4.00(m), 3.95(s), 3.94(s), 3.90(s), 3.84-3.82(d), 3.81(s), 3.66-3.60(q), 3.50-3.45(brd), 3.40(s), 3.30(s), 3.23-3.17(dt), 3.03-2.97(brt), 3.86-3.80(brt), 2.60-2.55(brt), 2.50-2.40(m), 2.30-2.25(brd), 2.20(s), 2.15-2.10(brd), 1.90-1.65(m), 1.64-1.60(brd), 1.56-1.43(m), 1.36-1.27(m), 1.26-1.11(m).

Compound 79: ¹H NMR(500MHz CDCl₃), (single diastereomer, mixture of rotamers) δ 8.65-8.59(d), 8.58-8.52(d), 7.40-7.35(d), 7.32-7.28(d), 7.25-7.24(d), 7.13(s), 6.65(s), 6.60(s), 6.20-6.18(d), 6.12-6.10(d), 5.97-5.90(m), 5.89-5.75(m), 5.43-5.38(d), 5.33-5.20(m), 5.16(s), 5.15(s), 5.10(s), 4.60-4.58(brd), 4.51-4.49(d), 4.40-4.38(d), 4.05-4.00(m), 3.93-3.85(m), 3.83(s), 3.82(s), 3.79(s), 3.65-3.60(q), 3.50-3.45(brd), 3.39(s), 3.30-3.18(m), 2.95-2.80(m), 2.61-2.55(m), 2.39-2.32(brd), 2.20(s), 1.90-1.75(m), 1.74-1.66(m), 1.65-1.60(m), 1.59-1.48(m), 1.47-1.31(m), 1.27-1.22(m), 1.20-1.18(d).

Compound 80: ¹H NMR(500MHz CDCl₃), (single diastereomer, mixture of rotamers) δ 8.62-8.58(d), 8.56-8.52(d), 7.40-7.35(d), 7.30(brs), 7.26(s), 7.18(s), 6.62(s), 6.60(s), 5.72-5.68(t), 5.62-5.58(t), 5.40-5.36(d), 5.30(s), 5.18(s), 5.17-5.13(d), 5.10(s), 4.66-4.61(br d), 4.60-4.58(m), 4.31-4.29(br d), 3.96(s), 3.95(s), 3.92(s), 3.87(s), 3.49-3.43(br d), 3.24-3.16(dt), 3.04-2.96(brt), 2.32-2.28(br d), 2.17(s), 2.13-2.06(m), 2.91-2.85(m), 2.81-1.64(m), 1.63-1.55(m), 1.54-1.40(m), 1.36-1.00(m), 0.93-0.87(t), 0.83-0.77(t).

Compound 81: ¹H NMR(500MHz CDCl₃), (single diastereomer, mixture of rotamers) δ 8.62-8.58(d), 8.56-8.52(d), 7.41-7.39(d), 7.38-7.35(d), 7.33-7.28(d), 7.27(s), 7.23(s), 7.11(s), 6.60(s), 6.50(s), 5.65-5.61(t), 5.60-5.97(t), 5.38-5.35(d), 5.30(s), 5.15(s), 5.13-5.10(d), 5.08(s), 5.06(s), 5.01(s), 4.59-4.54(brd), 4.40-4.38(brd), 3.91(s), 3.85(s), 3.80(s), 3.74(s), 3.48-3.42(brd), 3.30-3.23(dt), 2.95-2.90(brt), 2.38-2.32(brd), 2.18(s), 1.90-1.75(m), 1.74-1.46(m), 1.44-1.10(m), 0.94-0.88(t), 0.87-0.82(t).

Compound 82: ¹H NMR(500MHz CDCl₃), (single diastereomer, mixture of rotamers) δ 7.28-7.35(m), 7.26(s), 7.24(m), 7.14(d), 7.10(d), 6.65(s), 6.57(s), 5.85(q), 5.78(q), 5.40(d), 5.13(s), 5.07(q), 5.04(s), 4.60(bd), 4.38(d), 3.92(s), 3.88(s), 3.80(s), 3.48(bd), 3.26(dt), 2.95(dt), 2.40(bd), 2.25(bd), 1.82(m), 1.64(bd), 1.56(s), 1.54(d), 1.46(d), 1.38(m).

Compound 83: ¹H NMR(500MHz CDCl₃), (single diastereomer, mixture of rotamers) δ 7.36(s), 7.34(m), 7.27(m), 7.22(d), 7.13(dd), 7.08(dd), 6.65(s), 5.85(q), 5.75(q), 5.40(d), 5.10(d), 5.04(s), 4.63(bd), 4.34(d), 3.95(s), 3.92(s), 3.88(s), 3.46(bd), 3.22(dt), 3.04(dt), 2.33(bd), 2.15(bd), 1.80(m), 1.70(dt), 1.55(d), 1.46-1.58(m), 1.36(d), 1.14(m).

Compound 84: ¹H NMR(500MHz CDCl₃), (single diastereomer, mixture of rotamers) δ 8.53(d), 8.52(d), 7.42(d), 7.31(s), 7.27(d), 7.17(s), 6.52(ABq), 5.81(q), 5.74(q), 5.10(d), 5.04(s), 5.03(s), 4.58-4.50(m), 4.31(m), 3.91(s), 3.88(s), 3.87(s), 3.85(s), 3.41(brd), 3.18(ddd), 3.00(ddd), 2.29(brd), 2.12(brd), 1.78-1.72(m), 1.68(brd), 1.52(d), 1.36(d), 1.32(d), 1.31(d), 1.11(m).

Compound 85: ¹H NMR(500MHz CDCl₃), (single diastereomer, mixture of rotamers) δ 8.51(d), 7.42(d), 7.31(s), 7.28(d), 7.25(s), 7.13(s), 6.58(s), 5.80(q), 5.76(q), 5.33(d), 5.10(s), 5.02(s), 4.56-4.50(m), 4.31(brd), 3.90(s), 3.88(s), 3.81(s), 3.79(s), 3.46(brd), 3.24(ddd), 2.90(ddd), 2.33(brd), 2.21(brd), 1.85-1.74(m), 1.62(m), 1.51(d), 1.47(d), 1.31(d), 1.29(d).

Compound 86: ¹H NMR(500MHz CDCl₃), (single diastereomer, mixture of rotamers) δ 8.61-8.45(m), 7.38-7.28(m), 6.68(s), 6.49(s), 5.79(q), 5.61(q), 5.19-5.01(m), 4.72-4.63(m), 3.89-3.67(m), 3.65-3.45(m), 2.85(t), 2.58(1), 2.39-2.23(m), 2.11-1.92(m), 1.72-1.45(m), 1.39-1.16(m), 0.89(m).

Compound 87: ¹H NMR(500MHz CDCl₃), (single diastereomer, mixture of rotamers) δ 8.60-8.46(m), 7.38-7.15(m), 6.74-6.63(m), 6.62(s), 6.52-6.47(m), 5.75(q), 5.61(m), 5.32-5.25(m), 5.15-5.01(m), 4.72-4.59(m), 3.93-3.80(m), 3.75(m), 3.62-3.43(m), 2.39-1.55(m), 1.50(dd), 1.36-1.21(m).

Compound 88: ¹H NNR(500MHz CDCl₃), (mixture of diastereomers, mixture of rotamers) δ 9.16(d), 8.74(d), 8.70(d) 7.85(d), 7.50(t), 7.27(d), 6.68(s), 5.80(m), 5.70(m), 5.38(bd), 5.31(bd), 5.24(s), 5.20(d), 4.60(m), 4.34(dd), 3.88-3.95(m), 3.84(s), 3.75(s), 3.45(bd), 3.24(dt), 3.19(dt), 2.98(bt), 2.34(bd), 2.30(bd), 2.22(bd), 1.10-1.90(m), 1.52(d), 1.45(d).

Compound 89: ¹H NMR(500MHz CDCl₃), (single diastereomer, mixture of rotamers) δ 7.36-7.22(m), 5.43(d), 5.36(quintet), 5.25(quintet), 4.60-4.35(m), 3.95(s), 3.91(s), 3.88(s), 3.03(d), 3.67(d), 3.47-3.40(brd), 3.24(dt), 3.07(dt), 2.38(brd), 2.22(br d), 1.85-1.60(m), 1.58-1.25(m).

Compound 91: ¹H NMR(500MHz CDCl₃), (single diastereomer, mixture of rotamers) δ 9.01-8.93(m), 8.78(m), 8.06(m), 7.75(s), 7.68(t), 7.61(m), 7.57(d), 7.51-7.41(m), 7.28-7.19(m), 7.15(t), 7.12-7.05(m), 7.03(s), 5.82(q), 5.73(t), 5.33(d), 4.55(d), 4.33(d), 3.93-3.78(m), 3.73(s), 3.43(d,br), 3.21(dt), 3.01(t), 2.63(t), 2.58(t), 2.39(d,br), 2.22(d), 2.09-1.94(m), 1.92-1.43(m), 1.41-1.14(m).

Compound 92: ¹H NMR(500MHz CDCl₃), (single diastereomer, mixture of rotamers) δ 8.94(d), 8.81(m), 8.08(m), 7.75(s), 7.69(t), 7.55(d), 7.48(t), 7.42(m), 7.31(s), 7.29-7.07(m), 7.02(d), 5.81(t), 5.71(t), 5.40(d), 4.56(d), 4.34(d), 3.92-3.79(m), 3.40(d,br), 3.11(dt), 2.96(t), 2.61(t), 2.50(m), 2.22-1.91(m), 1.90-1.35(m), 1.20(s), 1.02(m), 0.83(t).

Compound 93: ¹H NMR(500MRz CDCl₃), (mixture of diastereomers, mixture of rotamers) δ 8.62-8.55(m), 7.66-7.58(m), 7.57-7.56(m), 7.52-7.46(m), 7.40-7.30(m), 7.29-7.20(m), 7.19-7.04(m), 6.96-6.79(m), 6.77-6.69(m), 5.85-5.77(m), 5.70-5.62(m), 5.43-5.38(m), 5.10-4.98(m), 4.64-4.52(m), 4.39-4.35(m), 4.08-4.06(m), 4.02-3.99(m), 3.98-3.90(m), 3.89-3.84(m), 3.83-3.68(m), 3.48-3.40(m), 3.18(ddd), 3.14(ddd), 2.96(ddd), 2.92(ddd), 2.68-2.58(m), 2.57-2.51(m), 2.37(dd), 2.24-2.11(m), 2.05-1.94(m), 1.89-1.41(m), 1.40-1.23(m), 1.22-1.10(m).

Compound 94: ¹H NMR(500MHz CDCl₃), (mixture of diastereomers, mixture of rotamers) δ 8.61-8.55(m), 7.47-7.40(m), 7.38-7.02(m), 6.92-6.88(m), 6.87-6.82(m), 6.81-6.71(m), 6.68-6.64(m), 5.77-5.72(m), 5.65-5.59(m), 5.40-5.36(m), 5.11-5.04(m), 5.02(s), 4.97(s), 4.58-4.52(m), 4.36-4.33(m), 3.87(s), 3.83(s), 3.77(s), 3.70(s), 3.57-3.52(m), 3.48-3.36(m), 3.24(ddd), 3.12(ddd), 2.99(ddd), 2.81(ddd), 2.66-2.53(m), 2.41-2.31(m), 2.28-2.22(m), 2.02-1.92(m), 1.88-1.45(m), 1.44-1.21(m).

Compound 95: ¹H NMR(500MHz CDCl₃), (mixture of diastereomers, mixture of rotamers) δ 8.91-8.75(m), 7.38-7.29(m), 7.28-7.02(m), 6.92-6.80(m), 6.79-6.76(m), 6.74-6.71(m), 6.69-6.64(m), 6.09-5.98(m), 5.78-5.70(m), 5.65-5.60(m), 5.40-5.34(m), 5.32-5.26(m), 5.19-5.13(m), 5.09-5.00(m), 4.63-4.52(m), 4.36-4.32(m), 3.95-3.63(m), 3.46(brd), 3.41(brd), 3.24(ddd), 3.12(ddd), 3.02-2.92(m), 2.67-2.45(m), 2.41-2.30(m), 2.27-2.21(m), 2.20-2.12(m), 2.01-1.90(m), 1.89-1.04(m).

Compound 96: ¹H NMR(500MHz CDCl₃), (mixture of diastereomers, mixture of rotamers) δ 8.59-8.54(m), 7.67-7.57(m), 7.55-7.49(m), 7.47-7.38(m), 7.37-7.05(m), 6.95-6.71(m), 5.83(t), 5.78(t), 5.68(t), 5.65(t), 5.42-5.37(m), 5.28(S), 5.23-4.95(m), 4.62-4.52(m), 4.38-4.32(m), 3.93(s), 3.92(s), 3.88(s), 3.87(s), 3.47-3.38(m), 3.18-3.07(m), 2.98-2.87(m), 2.67-2.58(m), 2.57-2.50(m), 2.41-2.30(m), 2.22-2.17(m), 2.16-2.11(m), 2.03-1.92(m), 1.89-1.21(m), 1.20-1.09(m).

Compound 97: ¹H NMR(500MHz CDCl₃), (mixture of diastereomers, mixture of rotamers) δ 8.62-8.52(m), 7.64-7.54(m), 7.52-7.46(m), 7.42-7.04(m), 6.97-6.78(m), 6.77-6.70(m), 6.12-5.97(m), 5.85-5.76(m), 5.69-5.61(m), 5.46-5.35(m), 5.33-5.24(m), 5.10-5.01(m), 4.70-4.52(m), 4.39-4.33(m), 3.92(s), 3.91(s), 3.88(s), 3.87(s), 3.48-3.41(m), 3.18-3.10(m), 2.97-.2.90(m), 2.67-2.57(m), 2.56-2.50(m), 2.42-2.31(m), 2.23-2.10(m), 2.04-1.93(m), 1.89-1.10(m).

Compound 98: ¹H NMR(500MHz CDCl₃), (mixture of diastereomers, mixture of rotamers) δ 8.59-8.53(m), 7.67-7.44(m), 7.39-7.03(m), 6.94-6.78(m), 6.77-6.66(m), 6.46-6.33(m), 6.03-5.93(m), 5.83(t), 5.78(t), 5.68(t), 5.64(t), 5.42-5.37(m), 5.08-4.97(m), 4.92-4.66(m), 4.64-4.52(m), 4.40-4.33(m), 3.94(s), 3.92(s), 3.90(s), 3.88(s), 3.87-3.84(m), 3.48-3.40(m), 3.20-3.08(m), 2.98-2.88(m), 2.64-2.57(m), 2.56-2.50(m), 2.41-2.31(m), 2.23-2.17(m), 2.16-2.10(m), 2.03-1.92(m), 1.88-1.08(m).

Compound 99: ¹H NMR(500MHz CDCl₃), (mixture of diastereomers, mixture of rotamers) δ 8.67-8.58(m), 8.54-8.48(m), 7.49-7.03(m), 6.95-6.87(m), 6.86-6.82(m), 6.72-6.68(m), 5.78-5.68(m), 5.63-5.57(m), 5.40-5.31(m), 5.14-4.93(m), 4.59-4.51(m), 4.35-4.30(m), 3.90-3.78(m), 3.73(s), 3.71(s), 3.45(brd), 3.38(brd), 3.22(ddd), 3.11(ddd), 2.99-2.91(m), 2.67-2.48(m), 2.42-2.39(m), 2.26-2.18(m), 2.17-2.11(m), 2.05-1.92(m), 1.89-1.18(m), 1.09-0.98(m).

Compound 100: ¹H NMR(500MHz CDCl₃), (mixture of diastereomers, mixture of rotamers) δ 8.63-8.56(m), 7.68-7.59(m), 7.57-7.40(m), 7.39-7.20(m), 7.19-7.04(m), 7.03-6.98(m), 6.97-6.81(m), 6.78-6.71(m), 5.80(s), 5.77(s), 5.67(t), 5.62(t), 5.40-5.34(m), 5.27-4.94(m), 4.62-4.52(m), 4.38-4.32(m), 3.94(s), 3.92(s), 3.91(s), 3.88(s), 3.87(s), 3.82(s), 3.81(s), 3.47-3.37(m), 3.18-3.05(m), 3.00-2.90(m), 2.68-2.50(m), 2.43-2.29(m), 2.22-2.09(m), 2.07-1.95(m), 1.90-1.63(m), 1.62-1.20(m), 1.14-1.02(m).

Compound 101: ¹H NMR(500MHz CDCl₃), (mixture of diastereomers, mixture of rotamers) δ 8.64-8.58(m), 7.43-7.30(m), 7.29-7.19(m), 7.18-7.02(m), 6.98-6.94(m), 6.93-6.87(m), 6.86-6.83(m), 6.77-6.73(m), 5.73(t), 5.71(t), 5.62(t), 5.60(t), 5.41-5.32(m), 5.10-5.05(m), 4.58-4.52(m), 4.35-4.30(m), 3.94(s), 3.93(s), 3.91(s), 3.90(s), 3.88(s), 3.84(s), 3.83(s), 3.78(s), 3.76(s), 3.45(brd), 3.38(brd), 3.22(ddd), 3.10(ddd), 3.06-2.92(m), 2.67-2.53(m), 2.52-2.48(m), 2.42-2.29(m), 2.28-2.11(m), 2.04-1.94(m), 1.88-1.20(m), 1.08-0.98(m).

Compound 102: ¹H NMR(500MHz CDCl₃), (mixture of diastereomers, mixture of rotamers) δ 8.63-8.57(m), 7.66-7.60(m), 7.58-7.54(m), 7.53-7.47(m), 7.41-7.31(m), 7.27-7.20(m), 7.19-7.03(m), 6.92-6.70(m), 5.80(t), 5.77(t), 5.67(t), 5.61(t), 5.40-5.36(m), 5.09-5.02(m), 4.70-4.52(m), 4.37-4.33(m), 3.92(s), 3.91(s), 3.89(s), 3.88(s), 3.87(s), 3.86(s), 3.85(s), 3.82-3.77(m), 3.48-3.40(m), 3.18-3.09(m), 2.98-2.88(m), 2.66-2.42(m), 2.40-2.10(m), 2.04-1.94(m), 1.89-1.62(m), 1.61-1.18(m), 1.14-1.13(m).

Compound 103: ¹H NMR(500MHz CDCl₃), (mixture of diastereomers, mixture of rotamers) δ 7.76-7.59(m), 7.50-7.40(m), 7.38-7.18(m), 7.17-7.05(m), 6.93-6.87(m), 6.77-6.73(m), 6.18-6.15(m), 5.85(t), 5.79(t), 5.20(t), 5.16(t), 5.41-5.38(m), 5.21-5.08(m), 4.60-4.52(m), 4.37-4.32(m), 3.92(s), 3.91(s), 3.88(s), 3.87(s), 3.47-3.37(m), 3.17-3.03(m), 2.97-2.91(m), 2.64-2.58(m), 2.57-2.50(m), 2.42-2.33(m), 2.05-1.95(m), 1.90-1.80(m), 1.79-1.62(m), 1.61-1.31(m), 1.13-1.08(m).

Compound 104: ¹H NMR(500MHz CDCl₃), (mixture of diastereomers, mixture of rotamers) δ 7.47-7.41(m), 7.37-7.02(m), 5.78-5.72(m), 5.18(t), 5.12(t), 5.40-5.37(m), 5.10 (s), 5.08(s), 5.07(s), 5.05(s), 4.59-4.51(m), 4.37-4.31(m), 3.87(s), 3.85(s), 3.77(s), 3.73(s), 3.45(brd), 3.37(brd), 3.24(ddd), 3.10(ddd), 3.02-2.94(m), 2.65-2.59(m), 2.58-2.53(m), 2.52-2.46(m), 2.43-2.35(m), 2.27-2.22(m), 2.21-2.15(m), 2.05-1.94(m), 1.89-1.30(m), 1.10-1.01(m).

Compound 105: ¹H NMR(500MHz CDCl₃), (mixture of diastereomers, mixture of rotamers) δ 8.39(d), 7.64(q), 7.52(q), 7.43(m), 7.29-7.03(m), 5.02-4.88(m), 4.60(q), 4.46(q), 3.62(m), 3.52-3.38(m), 2.68-2.49(m), 2.31-2.13(m), 2.09-1.75(m), 1.74-1.44(m), 1.29-1.16(m).

Compound 106: ¹H NMR(500MHz CDCl₃), (single diastereomer, mixture of rotamers) δ 8.43-8.34(m), 7.46(ddt), 7.39(ddt), 7.32(s), 7.19-7.15(m), 5.32(br d), 5.28(s), 5.04-4.98(m), 4.92-4.88(m), 4.85(br d), 3.92(s), 3.90(s), 3.88(s), 3.87(s), 3.45(br d), 3.23(dt), 3.05(dt), 2.64-2.02(m), 2.29(br d), 2.13(br d), 1.82-1.48(m).

Compound 107: ¹H NMR(500MHz CDCl₃), (single diastereomer, mixture of rotamers) δ 7.34-7.23(m), 5.31(quintet), 5.12(quintet), 4.74(dd), 4.69(dd), 4.52(dq), 4.41(dq), 3.93(s), 3.90(s), 3.82(s), 3.70(m), 3.56-3.43(m), 2.34-1.88(m).

Compound 108: ¹H NMR(500MHz CDCl₃), (single diastereomer, mixture of rotamers) δ 8.50-8.31(m), 7.62(d), 7.57(d), 7.46(d), 7.44-7.31(m), 7.30(s), 7.19(q), 7.10(q), 5.00(m), 4.80(m), 4.69(m), 4.56(m), 3.97-3.71(m), 3.61-3.43(m), 2.68-2.41(m), 2.34-2.12(m), 2.08-1.84(m), 1.83-1.72(m), 1.71-1.42(m), 1.29-1.13(m).

Compound 109: ¹H NMR(500MHz CDCl₃), (single diastereomer, mixture of rotamers) δ 8.48-8.32(m), 7.53(dd), 7.47(m), 7.25-7.14(m), 5.02-4.89(m), 4.79(m), 4.49(m), 3.73-3.55(m), 3.48(quintet), 3.30(quintet), 2.69-2.44(m), 2.32-1.41(m), 1.32-1.04(m), 1.01(m).

Compound 110: ¹H NMR(500MHz CDCl₃), (single diastereomer, mixture of rotamers) δ 8.63-8.51(m), 8.50-8.31(m), 8.06(m), 7.93-7.85(m), 7.84-7.76(m), 7.69(d), 7.51-7.40(m), 7.23-7.11(m), 7.09(t), 5.32(d), 5.20(m), 5.08(m), 4.95(m), 4.61-4.52(m), 3.80(m), 3.61(m), 3.39(t), 3.21(dt), 2.94(dt), 2.74-2.44(m), 2.40(d), 2.31(m), 2.22-2.14(m), 2.13-1.91(m), 1.90-1.13(m).

Compound 110: ¹H NMR(500MHz CDCl₃), (single diastereomer, mixture of rotamers) δ 8.46-8.36(m), 7.61(dd), 7.52(dd), 7.50-7.40(m), 7.22-7.15(m), 6.87(dd), 6.83(dd), 6.07(s), 6.04(dd), 5.35(d), 5.10-5.06(m), 4.98-4.92(m), 4.6(br d), 4.34(d), 3.4(br d), 3.15(dt), 2.98(dt), 2.68-2.50(m), 2.24(br d), 1.8-1.46(m), 1.37-1.24(m).

Compound 112: ¹H NMR(500MHz CDCl₃), (single diastereomer, mixture of rotamers) δ 8.7(d), 8.6(d), 7.7.-7.6(dd), 7.45(s), 7.3-7.2(m), 6.9(d), 6.1(d), 5.3(m), 4.6(d), 4.4(d), 3.45(dd), 3.4-3.3(m), 3.1-2.9(m), 2.85-2.8(m), 2.4(dd), 1.97-1.7(m), 1.6-1.35(m).

Compound 112: ¹H NMR(500MHz CDCl₃), (single diastereomer, mixture of rotamers) δ 8.7(d), 8.6(d), 8.5(m), 7.7-7.6(dd), 7.3(s), 7.2(m), 5.4(d), 5.3(m), 4.6(brd), 4.4(brd), 3.95(s), 3.90(s), 3.85(s), 3.45(dd), 3.3-3.2(dd), 3.1-2.9(m), 2.4(dd), 1.95(s), 1.9-1.7(m), 1.6-1.35(m).

Compound 114: ¹H NMR(500MHz CDCl₃), (single diastereomer, mixture of rotamers) δ 8.49(d), 7.52(q), 7.31(s), 7.18(s), 7.12-6.99(m), 5.31(d), 4.99(m), 4.54(d), 3.92-3.79(m), 3.42(d,br), 3.22(dt), 3.02(dt), 2.81-2.62(m), 2.60(t), 2.30(d,br), 2.13(d), 1.82-1.19(m).

Compound 115: ¹H NMR(500MHz CDCl₃), (single diastereomer, mixture of rotamers) δ 8.63-8.53(m), 7.43-7.37(d), 7.35-7.23(m), 7.17(s), 6.56(s), 6.54(s), 5.48-5.42(d), 5.41-5.38(d), 5.32-5.29-(d), 5.20-5.10(m), 4.68-4.62(brd), 4.32-4.30(d), 4.00-3.90(m), 3.86(s), 3.53-3.47(brd), 3.25-3.20(dt), 3.05-3.00(dt), 2.37-2.21(brd), 2.10-2.00(m), 1.92-1.87(m), 1.80-1.70(m), 1.69-1.59(m), 1.57-1.43(m), 1.34-1.15(m), 0.97-0.92(d), 0.85-0.78(d), 0.77-0.75(d), 0.66-0.64(d).

Compound 116: ¹H NMR(500MHz CDCl₃), (single diastereomer, mixture of rotamers) δ 8.65-8.55(m), 7.42-7.40(d), 7.39-7.37(d), 7.33-7.30(d), 7.26(s), 7.22(s), 7.10(s), 6.60(s), 6.42(s), 5.42-5.40(d), 5.39-5.37(d), 5.34-5.32(d), 5.16(s), 5.15-5.11(m), 5.10(s), 5.07-4.94(q), 4.60-4.55(brd), 4.41-4.39(brd), 3.93(s), 3.84(s), 3.80(s), 3.70(s), 3.48-3.43(brd), 3.30-3.22(dt), 2.96-2.90(dt), 2.39-2.35(brd), 2.29-2.25(brd), 2.05-2.00(m), 1.90-1.75(m), 1.65-1.60(m), 1.59-1.48(m), 1.47-1.33(m), 0.95-0.87(d), 0.86-0.83(d), 0.82-0.78(d), 0.73-0.69(d).

Compound 117: ¹H NMR(500MHz CDCl₃), (single diastereomer, mixture of rotamers) δ 8.65-8.60(d), 8.59-8.52(d), 7.45-7.39(d), 7.38-7.23(m), 7.21(s), 6.67(s), 6.66(s), 5.83-5.79(t), 5.78-5.75(t), 5.74-5.63(m), 5.53-5.48(m), 5.45-5.41(brd),5.20-5.05(m), 5.04(s), 5.01(s), 4.99(s), 4.72-4.68(brd), 4.35-4.32(brd), 3.98(s), 3.97(s), 3.93(s), 3.90(s), 3.85(s), 3.55-3.48(brd), 3.32-3.24(dt), 3.10-3.03(dt), 2.70-2.62(m), 2.61-2.56(m), 2.55-2.45(m), 2.39-2.32(brd), 2.20-2.15(brd), 1.97-1.70(m), 1.69-1.60(m), 1.59-1.47(m), 1.40-1.20(m), 0.93-0.90(m).

Compound 118: ¹H NMR(500MHz CDCl₃), (single diastereomer, mixture of rotamers) δ 8.66-8.62(d), 8.61-8.59(d), 7.46-7.44(d), 7.43-7.40(d), 7.39-7.33(d), 7.31(s), 7.28(s), 7.16(s), 6.68(s), 6.57(s), 5.80-5.75(t), 5.74-5.67(m), 5.43-5.40(d), 5.20-5.05(m), 4.64-4.60(brd), 4.43-4.41(brd), 3.96(s), 3.90(s), 3.85(s), 3.78(s), 3.53-3.49(brd), 3.35-3.28(dt), 3.02-2.96(brt), 2.70-2.50(m), 2.42-2.36(brd), 2.32-2.29(brd), 1.91-1.78(m), 1.73-1.68(brd), 1.63-1.55(m), 1.50-1.40(m).

### Example 13 -- MDR SENSITIZATION ASSAYS

To assay the ability of the compounds according to this invention to increase the antiproliferative activity of a drug, cell lines which are known to be resistant to a particular drug may be used. These cell lines include, but are not limited to, the L1210, P388D, CHO and MCF7 cell lines. Alternatively, resistant cell lines may be developed. The cell line is exposed to the drug to which it is resistant, or to the test compound; cell viability is then measured and compared to the viability of cells which are exposed to the drug in the presence of the test compound.

We have carried out assays using L1210 mouse leukemia cells transformed with the pHaMDR1/A retrovirus carrying a MDR1 cDNA, as described by Pastan et al., Proc. Natl. Acad. Sci., Vol. 85, 4486-4490. (1988). The resistant line, labelled L1210VMDRC.06, was obtained from Dr. M. M. Gottesman of the National Cancer Institute. These drug-resistant transfectants had been selected by culturing cells in 0.06 mg/ml colchicine.

Multi-drug resistance assays were conducted by plating cells (2 x 10³, 1 x 10⁴, or 5 x 10⁴ cells/well) in 96 well microtiter plates and exposing them to a concentration range of doxorubicin (50 nM-10 µM) in the presence or absence of multi-drug resistance modifier compounds ("MDR inhibitors") of this invention (1, 2.5 or 10 µM) as described in Ford et al., Cancer Res., Vol. 50, 1748-1756. (1990). After culture for 3 days, the viability of cells was quantitated using MTT (Mossman) or XTT dyes to assess mitochondrial function. All determinations were made in replicates of 4 or 8. Also see, Mossman T., J. Immunol. Methods, Vol. 65, 55-63 (1983).

Results were determined by comparison of the IC₅₀ for doxorubicin alone to the IC₅₀ for doxorubicin + MDR inhibitor. An MDR ratio was calculated (IC₅₀ Dox/IC₅₀ Dox + Inhibitor) and the integer value used for comparison of compound potencies.

In all assays, compounds according to this invention were tested for intrinsic antiproliferative or cytotoxic activity. The results are summarized in Table 2 below. As demonstrated in Table 2, the compounds generally caused <10% cytotoxicity at concentrations of 10 µM or greater.

Compounds of formula (I) have also been assayed for MDR sensitization activity with other MDR cell lines including several human cell lines (e.g., myeloma cells (8226/DOX6, 8226/DOX40, MDR10V, MR 20), melanoma cells (VCR 4.5, VBL 3.0, COL-1), GM3639 T cells, MCF-7 breast carcinoma, A549 bronchogenic adenocarcinoma, LOX melanoma, P388/ADR, and P388 VMDRC.04), and different chemotherapeutic drugs (e.g., doxorubicin, vincristine, vinblastine, taxol, colchicine, and etoposide). Results similar to those shown in Table 2 were obtained in these assays (data not shown), further demonstrating the effectiveness of the compounds of this invention in multi-drug resistance sensitization.

**Table 2:**

| Evaluation of Compounds for Reversal of Multidrug Resistance | | | | | | | |
|---|---|---|---|---|---|---|---|
| Cmpd | IC₅₀Dox Alone | IC₅₀ + 1 µM | IC₅₀Dox + 2.5 µM | IC₅₀Dox + 10 µM | MDR Ratio 1 µM | MDR Ratio 2.5 µM | MDR Ratio 10 µM |
| 2 | 900nM | 400 | | <60 | 2.25 | | >15 |
| 4 | 800 | 400 | | <60 | 2 | | 2.7 |
| 6 | 900 | 300 | | <60 | 3 | | >15 |
| 8 | 800 | 500 | | 100 | 1.6 | | 8 |
| 10 | 6500 | | 625 | | | 10.4 | |
| 11 | 700 | 200 | | <60 | 3.5 | | >12 |
| 12 | 6500 | | 350 | | | 18.6 | |
| 15 | 800 | 400 | | <60 | 2 | | >13 |
| 21 | 1000 | 700 | | 90 | 1.4 | | 11.1 |
| 27 | 1200 | 900 | | 200 | 1.3 | | 6 |
| 31 | 1300 | 900 | | 500 | 1.4 | | 2.6 |
| 43 | 6500 | | 600 | | | 10.8 | |
| 44 | 400 | 200 | | <60 | 2 | | >6 |
| 47 | 900 | 800 | | 100 | 1.1 | | 9 |
| 48 | 1400 | 800 | | 100 | 1.75 | | 14 |
| 49 | 5000 | | 700 | | | 7.1 | |
| 52 | 900 | 500 | | <60 | 1.8 | | >15 |
| 53 | 1600 | 700 | | 200 | 2.3 | | 8 |
| 54 | 6500 | | 510 | | | 12.7 | |
| 55 | 900 | 400 | | <60 | 2.25 | | >15 |
| 56 | 400 | 300 | | <60 | 1.3 | | >7 |
| 64 | 1500 | 700 | | 400 | 2.1 | | 3.75 |
| 66 | 1600 | 1300 | | 400 | 1.3 | | 4 |
| 69 | 800 | 400 | | <60 | 2 | | >13 |
| 84 | 6000 | | 350 | | | 17.1 | |
| 98 | 6000 | | 2000 | | | 3 | |
| 105 | 9000 | 2800 | | 500 | 3.2 | | 18 |
| CsA | 1800 | 80 | | | 22.5 | | |
| FK506 | 400 | 400 | | 100 | 1 | | 4 |

While we have described a number of embodiments of this invention, it is apparent that our basic constructions may be altered to provide other embodiments which utilize the products, processes and methods of this invention. Therefore, it will be appreciated that the scope of this invention is to be defined by the appended claims, rather than by the specific embodiments which have been presented by way of example.

## Claims

1. A compound of formula (I): 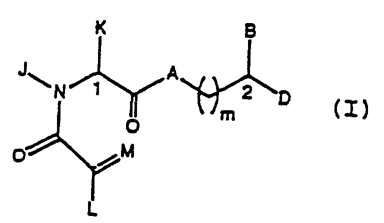 wherein A is CH₂, oxygen, NH or N-(C1-C4 alkyl);
wherein B and D are independently
(i) Ar, (C1-C10)-straight or branched alkyl, (C2-C10)-straight or branched alkenyl or alkynyl, (C5-C7)-cycloalkyl substituted (C1-C6)-straight or branched alkyl, (C2-C6)-straight or branched alkenyl or alkynyl, (C5-C7)-cycloalkenyl substituted (C1-C6)-straight or branched alkyl, (C2-C6)-straight or branched alkenyl or alkynyl, or Ar substituted (C1-C6)-straight or branched alkyl, (C2-C6)-straight or branched alkenyl or alkynyl wherein, in each case, any one of the CH₂ groups of said alkyl, alkenyl or alkynyl chains may be optionally replaced by a heteroatom selected from the group consisting of O, S, SO, SO₂, N, and NR, wherein R is selected from the group consisting of hydrogen, (C1-C4)-straight or branched alkyl, (C2-C4)-straight or branched alkenyl or alkynyl, and (C1-C4) bridging alkyl wherein a bridge is formed between the nitrogen and a carbon atom of said heteroatom-containing chain to form a ring, and wherein said ring is optionally fused to an Ar group; or 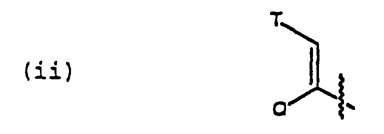
wherein Q is hydrogen, (C1-C6)-straight or branched alkyl or (C2-C6)-straight or branched alkenyl or alkynyl;
wherein T is Ar or substituted 5-7 membered cycloalkyl with substituents at positions 3 and 4 which are independently selected from the group consisting of oxo, hydrogen, hydroxyl, O-(C1-C4)-alkyl or O-(C2-C4)-alkenyl;
provided that at least one of B or D is independently selected from the group consisting of (C2-C10)-straight or branched alkynyl, (C5-C7)-cycloalkyl substituted (C2-C6)-straight or branched alkynyl, (C5-C7)-cycloalkenyl substituted (C2-C6)-straight or branched alkynyl, and Ar substituted (C2-C6)-straight or branched alkynyl;
wherein Ar is a carbocyclic aromatic group selected from the group consisting of phenyl, 1-naphthyl, 2-naphthyl, indenyl, azulenyl, fluorenyl, and anthracenyl; or a
heterocyclic aromatic group selected from the group consisting of 2-furyl, 3-furyl, 2-thienyl, 3-thienyl, 2-pyridyl, 3-pyridyl, 4-pyridyl, pyrrolyl, oxazolyl, thiazolyl, imidazolyl, pyrazolyl, 2-pyrazolinyl, pyrazolidinyl, isoxazolyl, isotiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indoliziriyl, indolyl, isoindolyl, 3H-indolyl, indolinyl, benzo[b]furanyl, benzo[b]thiophenyl, 1H-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, carbazolyl, acridinyl, phenazinyl, phenothiazinyl, and phenoxazinyl;
wherein Ar may contain one to three substituents which are independently selected from the group consisting of hydrogen, halogen, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl, N-(C1-C5-straight or branched alkyl or alkenyl) carboxamide, N,N-di-(C1-C5-straight or branched alkyl or C2-C5-straight or branched alkenyl)carboxamide, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, and q is 0-2;
wherein L is either hydrogen or U; M is either oxygen or CH-U, provided that if L is hydrogen, then M is CH-U or if M is oxygen, then L is U;
wherein U is hydrogen, O-(C1-C4)-straight or branched alkyl or O-(C2-C4)straight or branched alkenyl, (C1-C6)-straight or branched alkyl or (C2-C6)-straight or branched alkenyl, (C5-C7)-cycloalkyl or (C5-C7)-cycloalkenyl substituted with (C1-C4)-straight or branched alkyl or (C2-C4)-straight or branched alkenyl, [(C1-C4)-alkyl or (C2-C4)-alkenyl]-Y or Y;
wherein Y is a carbocyclic aromatic group selected from the group consisting of phenyl, 1-naphthyl, 2-naphthyl, indenyl, azulenyl, fluorenyl, and anthracenyl; or a
heterocyclic aromatic groups as defined above;
wherein Y may contain one to three substituents which are independently selected from the group consisting of hydrogen, halogen, hydroxyl, nitro, trifluoromethyl, trifluoromethoxy, (C1-C6)-straight or branched alkyl, (C1-C6)-straight or branched alkenyl, O-(C1-C4)-straight or branched alkyl, O-(C2-C4)-straight or branched alkenyl, O-benzyl, O-phenyl, 1,2-methylenedioxy, amino, and carboxyl;
wherein J is hydrogen, (C1-C2) alkyl or benzyl; K is (C1-C4)-straight or branched alkyl, benzyl or cyclohexylmethyl, or wherein J and K may be taken together to form a 5-7 membered heterocyclic ring which may contain a heteroatom selected from the group consisting of O, S, SO and SO₂; and
wherein m is 0-3.

2. A compound of formula (I): 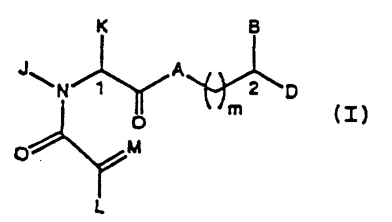 wherein A is CH₂, oxygen, NH or N-(C1-C4 alkyl);
wherein B and D are independently:
(i) Ar, (C1-C10)-straight or branched alkyl, alkenyl or alkynyl, (C5-C7)-cycloalkyl substituted (C1-C6)-straight or branched alkyl, alkenyl or alkynyl, (C5-C7)-cycloalkenyl substituted (C1-C6)-straight or branched alkyl, alkenyl or alkynyl, or Ar substituted (C1-C6)-straight or branched alkyl, alkenyl, or alkynyl; wherein, in each case, any one of the CH₂ groups of said alkyl, alkenyl or alkynyl chains may be optionally repyaced by a heteroatom selected from the group consisting of O, S, SO and SO₂; or 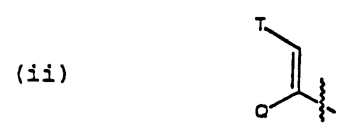
wherein Q is hydrogen, (C1-C6)-straight or branched alkyl or (C2-C6)-straight or branched alkenyl;
wherein T is Ar or substituted 5-7 membered cycloalkyl with substituents at positions 3 and 4 which are independently selected from the group consisting of oxo, hydrogen, hydroxyl, O-(C1-C4)-alkyl, and O-(C2-C4)-alkenyl;
provided that at least one of B or D is independently selected from the group consisting of (C2-C10)-straight or branched alkynyl, (C5-C7)-cycloalkyl substituted (C2-C6)-straight or branched alkynyl, (C5-C7)-cycloalkenyl substituted (C2-C6)-straight or branched alkynyl, and Ar substituted (C2-C6)-straight or branched alkynyl;
wherein Ar is selected from the group consisting of phenyl, 1-naphthyl, 2-naphthyl, 2-furyl, 3-furyl, 2-thienyl, 3-thienyl, 2-pyridyl, 3-pyridyl, 4-pyridyl, and mono and bicyclic heterocyclic ring systems with individual ring sizes being 5 or 6 which may contain in either or both rings a total of 1-4 heteroatoms independently selected from oxygen, nitrogen and sulfur;
wherein Ar may contain one to three substituents which are independently selected from the group consisting of hydrogen, halogen, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl, N-(C1-C5-straight or branched alkyl or alkenyl) carboxamide, N,N-di-(C1-C5-straight or branched alkyl or alkenyl)carboxamide, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, and q is 0-2;
wherein L is either hydrogen or U and M is either oxygen or CH-U, provided that if L is hydrogen, then M is CH-U or if M is oxygen then L is U;
wherein U is hydrogen, O-(C1-C4)-straight or branched alkyl or O-(C1-C4)straight or branched alkenyl, (C1-C6)-straight or branched alkyl or (C1-C6)-straight or branched alkenyl, (C5-C7)-cycloalkyl or (C5-C7)-cycloalkenyl substituted with (C1-C4)-straight or branched alkyl or (C2-C4)-straight or branched alkenyl, [(C1-C4)-alkyl or (C2-C4)-alkenyl]-Y or Y;
wherein Y is selected from the group consisting of phenyl, 1-naphthyl, 2-naphthyl, 2-furyl, 3-furyl, 2-thienyl, 3-thienyl, 2-pyridyl, 3-pyridyl, 4-pyridyl, and mono and bicyclic heterocyclic ring systems with individual ring sizes being 5 or 6 which may contain in either or both rings a total of 1-4 heteroatoms independently selected from oxygen, nitrogen and sulfur;
wherein Y may contain one to three substituents which are independently selected from the group consisting of hydrogen, halogen, hydroxyl, nitro, trifluoromethyl, trifluoromethoxy, (C1-C6)-straight or branched alkyl, (C1-C6)-straight or branched alkenyl, O-(C1-C4)-straight or branched alkyl, O-(C2-C4)-straight or branched alkenyl, O-benzyl, O-phenyl, 1,2-methylenedioxy, amino, and carboxyl;
wherein J is hydrogen (C1-C2) alkyl or benzyl; K is (C1-C4)-straight or branched alkyl, benzyl or cyclohexylmethyl, or wherein J and K may be taken together to form a 5-7 membered heterocyclic ring which may contain an O, S, SO or SO₂ substituent therein; and
wherein m is 0-3.

3. A compound of formula (I): 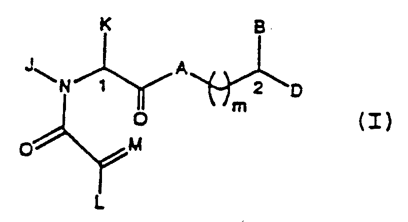 wherein A is CH₂, oxygen, NH or N-(C1-C4 alkyl);
wherein B and D are independently
(i) Ar, (C1-C10)-straight or branched alkyl, (C2-C10)-straight or branched alkenyl or alkynyl, (C5-C7)-cycloalkyl substituted (C1-C6)-straight or branched alkyl, (C2-C6)-straight or branched alkenyl or alkynyl, (C5-C7)-cycloalkeny substituted (C1-C6)-straight or branched alkyl, (C2-C6)-straight or branched alkenyl or alkynyl; or Ar substituted (C1-C6)-straight or branched alkyl, (C2-C6)-straight or branched alkenyl or alkynyl wherein, in each case, any one of the CH₂ groups of said alkyl, alkenyl or alkynyl chains may be optionally replaced by a heteroatom selected from the group consisting of O, S, SO, SO₂, N, and NR, wherein R is selected from the group consisting of hydrogen, (C1-C4)-straight or branched alkyl, (C2-C4)-straight or branched alkenyl or alkynyl, and (C1-C4) bridging alkyl wherein a bridge is formed between the nitrogen and a carbon atom of said heteroatom-containing chain to form a ring, and wherein said ring is optionally fused to an Ar group; or 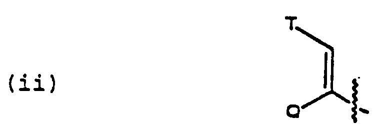
wherein Q is hydrogen, (C1-C6)-straight or branched alkyl or (C2-C6)-straight or branched alkenyl or alkynyl;
wherein T is Ar or substituted 5-7 membered cycloalkyl with substituents at positions 3 and 4 which are independently selected from the group consisting of oxo, hydrogen, hydroxyl, O-(C1-C4)-alkyl and O-(C2-C4)-alkenyl;
provided that at least one of B or D is independently selected from the group consisting of Ar', Ar'-substituted (C1-C6)-straight or branched alkyl, and Ar'-substituted (C2-C6)-straight or branched alkenyl or alkynyl;
wherein Ar' is an Ar group substituted with one to three substituents which are independently selected from the group consisting of N-(straight or branched C1-C5 alkyl or C2-C5 alkenyl) carboxamides, N,N-di-(straight or branched C1-C5 alkyl or C2-C5 alkenyl)carboxamides, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, wherein q is 0-2;
wherein Ar is a carbocyclic aromatic group selected from the group consisting of phenyl, 1-naphthyl, 2-naphthyl, indenyl, azulenyl, fluorenyl, and anthracenyl; or a
heterocyclic aromatic group selected from the group consisting of 2-furyl, 3-furyl, 2-thienyl, 3-thienyl, 2-pyridyl, 3-pyridyl, 4-pyridyl, pyrrolyl, oxazolyl, thiazolyl, imidazolyl, pyrazolyl, 2-pyrazolinyl, pyrazolidinyl, isoxazolyl, isotiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, benzo[b]thiophenyl, 1H-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, carbazolyl, acridinyl, phenazinyl, phenothiazinyl, and phenoxazinyl ;
wherein Ar may contain one to three substituents which are independently selected from the group consisting of hydrogen, halogen, hydroxyl, nitro, trifluoromethyl, trifluoromethoxy, (C1-C5)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl, N-(C1-C5-straight or branched alkyl or alkenyl) carboxamides, N,N-di-(C1-C5-straight or branched alkyl or C2-C5-straight or branched alkenyl)carboxamides, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, and q is 0-2;
wherein L is either hydrogen or U and M is either oxygen or CH-U, provided that if L is hydrogen, then M is CH-U or if M is oxygen then L is U;
wherein U is hydrogen, O-(C1-C4)-straight or branched alkyl or O-(C2-C4)straight or branched alkenyl, (C1-C6)-straight or branched alkyl or (C2-C6)-straight or branched alkenyl, (C5-C7)-cycloalkyl or (C5-C7)-cycloalkenyl substituted with (C1-C4)-straight or branched alkyl or (C2-C4)-straight or branched alkenyl, [(C1-C4)-alkyl or (C2-C4)-alkenyl]-Y or Y;
wherein Y is selected from the group consisting of phenyl, 1-naphthyl, 2-naphthyl, indenyl, azulenyl, fluorenyl, anthracenyl, and heterocyclic aromatic groups as defined above;
where Y may contain one to three substituents which are independently selected from the group consisting of hydrogen, halogen, hydroxyl, vitro, trifluoromethyl, trifluoromethoxy, (C1-C5)-straight or branched alkyl, (C1-C6)-straight or branched alkenyl, O-(C1-C4)-straight or branched alkyl, O-(C2-C4)-straight or branched alkenyl, O-benzyl, O-phenyl, 1,2-methylenedioxy, amino, and carboxyl;
wherein J is hydrogen, (C1-C2) alkyl or benzyl; K is (C1-C4)-straight or branched alkyl, benzyl or cyclohexylmethyl, or wherein J and K may be taken together to form a 5-7 membered heterocyclic ring which may contain a heteroatom selected from the group consisting of O, S, SO and SO₂; and
wherein m is 0-3.

4. A pharmaceutical composition comprising a compound according to any one of claims 1 to 3 and a pharmaceutically acceptable carrier, adjuvant or vehicle.

5. The pharmaceutical composition according to claim 4, further comprising a chemotherapeutic agent.

6. The pharmaceutical composition according to claim 4 or 5, further comprising a chemosensitizer, other than the compound according to any one of claims 1 to 3.

7. A use of a compound of formula (I) for the manufacture of a medicament for treating or preventing multi-drug resistance in a patient, **characterized in that** said compound has formula: 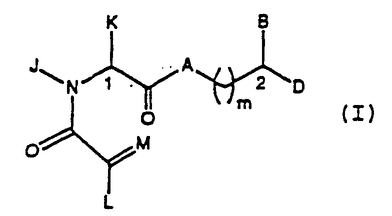 wherein A is CH₂, oxygen, NH or N- (C1-C4 alkyl);
wherein B and D are independently:
(i) hydrogen, Ar, (C1-C10)-straight or branched alkyl, (C2-C10)-straight or branched alkenyl or alkynyl, (C5-C7)-cycloalkyl substituted (C1-C6)-straight or branched alkyl, (C2-C6)-straight or branched alkenyl or alkynyl, (C5-C7)-cycloalkenyl substituted (C1-C6)-straight or branched alkyl, (C2-C6)-straight or branched alkenyl or alkynyl, or Ar substituted (C1-C6)-straight or branched alkyl, (C2-C6)-straight or branched alkenyl or alkynyl
wherein, in each case, any one of the CH₂ groups of said alkyl, alkenyl or alkynyl chains may be optionally replaced by a heteroatom selected from the group consisting of O, S, SO, SO₂, N, and NR, wherein R is selected from the group consisting of hydrogen, (C1-C4)-straight or branched alkyl, (C2-C4)-straight or branched alkenyl or alkynyl, and (C1-C4) bridging alkyl wherein a bridge is formed between the nitrogen and a carbon atom of said heteroatom-containing chain to form a ring, and wherein said ring is optionally fused to an Ar group; or 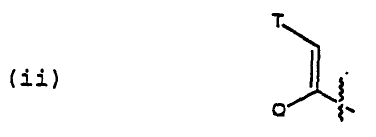 wherein Q is hydrogen, (C1-C6)-straight or branched alkyl or (C2-C6)-straight or branched alkenyl or alkynyl;
wherein T is Ar or substituted 5-7 membered cycloalkyl with substituents at positions 3 and 4 which are independently selected from the group consisting of oxo, hydrogen, hydroxyl, O-(C1-C4)-alkyl, and O-(C2-C4)-alkenyl;
wherein Ar is a carbocyclic aromatic group selected from the group consisting of phenyl, 1-naphthyl, 2-naphthyl, indenyl, azulenyl, fluorenyl, and anthracenyl ; or
a heterocyclic aromatic group selected from the group consisting of 2-furyl, 3-furyl, 2-thienyl, 3-thienyl, 2-pyridyl, 3-pyridyl, 4-pyridyl, pyrrolyl, oxazolyl, thiazolyl, imidazolyl, pyrazolyl, 2-pyrazolinyl, pyrazolidinyl, isoxazolyl, isotiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, benzo[b]thiophenyl, 1H-indazolyl, benzimidazolyl, benzthiazolyl, purinyl, 4H-quinolizinyl, quinolinyl, isoquinolinyl, cinnolinyl, phthalazinyl, quinazolinyl, quinoxalinyl, 1,8-naphthyridinyl, pteridinyl, carbazolyl, acridinyl, phenazinyl, phenothiazinyl, and phenoxazinyl;
wherein Ar may contain one to three substituents which are independently selected from the group consisting of hydrogen, halogen, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl, N-(C1-C5-straight or branched alkyl or alkenyl) carboxamides, N,N-di-(C1-C5-straight or branched alkyl or C2-C5-straight or branched alkenyl)carboxamides, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, and q is 0-2;
wherein L is either hydrogen or U; M is either oxygen or CH-U, provided that if L is hydrogen, then M is CH-U or if M is oxygen, then L is U;
wherein U is hydrogen, O-(C1-C4)-straight or branched alkyl or O-(C2-C4)straight or branched alkenyl, (C1-C6)-straight or branched alkyl or (C2-C6)-straight or branched alkenyl, (C5-C7)-cycloalkyl or (C5-C7)-cycloalkenyl substituted with (C1-C4)-straight or branched alkyl or (C2-C4)-straight or branched alkenyl, [(C1-C4)-alkyl or (C2-C4)-alkenyl]-Y or Y;
wherein Y is selected from the group consisting of phenyl, 1-naphthyl, 2-naphthyl, indenyl, azulenyl, fluorenyl, anthracenyl, 2-pyrrolinyl, 3-pyrrolinyl, pyrolidinyl, 1,3-dioxolyl, 2-imidazolinyl, imidazolidinyl, 2H-pyranyl, 4H-pyranyl, piperidyl, 1,4-dioxanyl, morpholinyl, 1,4-dithianyl, thiomorpholinyl, piperazinyl, quinuclidinyl, and heterocyclic aromatic groups as defined above;
where Y may contain one to three substituents which are independently selected from the group consisting of hydrogen, halogen, hydroxyl, nitro, trifluoromethyl, trifluoromethoxy, (C1-C6)-straight or branched alkyl, (C1-C6)-straight or branched alkenyl, O-(C1-C4)-straight or branched alkyl, O-(C2-C4)-straight or branched alkenyl, O-benzyl, O-phenyl, 1,2-methylenedioxy, amino, and carboxyl;
wherein J is hydrogen, (C1-C2) alkyl or benzyl; K is (C1-C4)-straight or branched alkyl, benzyl or cyclohexylmethyl, or wherein J and K may be taken together to form a 5-7 membered heterocyclic ring which may contain a heteroatom selected from the group consisting of O, S, SO and SO₂; and
wherein m is 0-3.

8. A use of a compound of formula (I) for the manufacture of a medicament for treating or preventing multi-drug resistance in a patient, **characterized in that** said compound has formula: 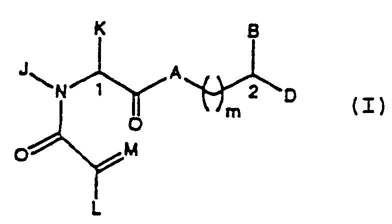 wherein A is CH₂, oxygen, NH or N-(C1-C4 alkyl);
wherein B and D are independently:
(i) Ar, (C1-C10)-straight or branched alkyl, (C2-C10)-straight or branched alkenyl or alkynyl, (C5-C7)-cycloalkyl substituted (C1-C6)-straight or branched alkyl, alkenyl or alkynyl, (C5-C7)-cycloalkenyl substituted (C1-C6)-straight or branched alkyl, alkenyl or alkynyl, or Ar substituted (C1-C6)-straight or branched alkyl, alkenyl, or alkynyl
wherein, in each case, any one of the CH₂ groups of said alkyl, alkenyl or alkynyl chains may be optionally replaced by a heteroatom selected from the group consisting of O, S, SO, SO₂; or 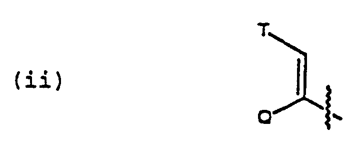 wherein Q is hydrogen, (C1-C6)-straight or branched alkyl or (C1-C6)-straight or branched alkenyl;
wherein T is Ar or substituted 5-7 membered cycloalkyl with substituents at positions 3 and 4 which are independently selected from the group consisting of oxo, hydrogen, hydroxyl, O-(C1-C4)-alkyl, or O-(C1-C4)-alkenyl;
wherein Ar is selected from the group consisting of phenyl, 1-naphthyl, 2-naphthyl, 2-furyl, 3-furyl, 2-thienyl, 3-thienyl, 2-pyridyl, 3-pyridyl, 4-pyridyl, and mono and bicyclic heterocyclic ring systems with individual ring sizes being 5 or 6 which may contain in either or both rings a total of 1-4 heteroatoms independently selected from oxygen, nitrogen and sulfur;
wherein Ar may contain one to three substituents which are independently selected from the group consisting of hydrogen, halogen, hydroxyl, nitro, trifluoromethyl, trifluoromethoxy, (C1-C6)-straight or branched alkyl, (C2-C6)-straight or branched alkenyl, O-(C1-C4)-straight or branched alkyl, O-(C2-C4)-straight or branched alkenyl, O-benzyl, O-phenyl, 1,2-methylenedioxy, amino, carboxyl, N-(C1-C5-straight or branched alkyl or alkenyl) carboxamides, N,N-di-(C1-C5-straight or branched alkyl or alkenyl)carboxamides, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, and q is 0-2;
wherein L is either hydrogen or U and M is either oxygen or CH-U, provided that if L is hydrogen, then M is CH-U or if M is oxygen then L is U;
wherein U is hydrogen, O-(C1-C4)-straight or branched alkyl or O-(C1-C4)straight or branched alkenyl, (C1-C6)-straight or branched alkyl or (C1-C6)-straight or branched alkenyl, (C5-C7)-cycloalkyl or (C5-C7)-cycloalkenyl substituted with (C1-C4)-straight or branched alkyl or (C2-C4)-straight or branched alkenyl, [(C1-C4)-alkyl or (C2-C4)-alkenyl]-Y or Y;
wherein Y is selected from the group consisting of phenyl, 1-naphthyl, 2-naphthyl, 2-furyl, 3-furyl, 2-thienyl, 3-thienyl, 2-pyridyl, 3-pyridyl, 4-pyridyl, and mono and bicyclic heterocyclic ring systems with individual ring sizes being 5 or 6 which may contain in either or both rings a total of 1-4 heteroatoms independently selected from oxygen, nitrogen and sulfur;
wherein Y may contain one to three substituents which are independently selected from the group consisting of hydrogen, halogen, hydroxyl, nitro, trifluoromethyl, trifluoromethoxy, (C1-C6)-straight or branched alkyl, (C1-C6)-straight or branched alkenyl, O-(C1-C4)-straight or branched alkyl, O-(C2-C4)-straight or branched alkenyl, O-benzyl, O-phenyl, 1,2-methylenedioxy, amino, and carboxyl;
wherein J is hydrogen (C1-C2) alkyl or benzyl; K is (C1-C4)-straight or branched alkyl, benzyl or cyclohexylmethyl, or wherein J and K may be taken together to form a 5-7 membered heterocyclic ring which may contain a heteroatom selected from the group consisting of O, S, SO and SO₂; and
wherein m is 0-3.

9. The use according to claim 7 or claim 8, wherein, in formula (I), at least one of B or D is independently selected from the group consisting of (C2-C10)-straight or branched alkynyl; (C5-C7)-cycloalkyl substituted (C2-C6)-straight or branched alkynyl; (C5-C7)-cycloalkenyl substituted (C2-C6)-straight or branched alkynyl; and Ar substituted (C2-C6)-straight or branched alkynyl.

10. The use according to claim 7 or claim 8, wherein, in formula (I), at least one of B or D is independently selected from the group consisting of Ar', Ar'-substituted (C1-C6)-straight or branched alkyl, and Ar'-substituted (C2-C6)-straight or branched alkenyl or alkynyl;
wherein Ar' is an Ar group substituted with one to three substituents which are independently selected from the group consisting of N-(straight or branched C1-C5 alkyl or C2-C5 alkenyl) carboxamides, N,N-di-(straight or branched C1-C5 alkyl or C2-C5 alkenyl)carboxamides, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, and CH=CH-X; wherein X is 4-methoxyphenyl, 2-pyridyl, 3-pyridyl, 4-pyridyl, pyrazyl, quinolyl, 3,5-dimethylisoxazoyl, isoxazoyl, 2-methylthiazoyl, thiazoyl, 2-thienyl, 3-thienyl, and pyrimidyl, wherein q is 0-2.

11. The use according to any one of claims 7 to 10, wherein, in formula (I), J and K are taken together to form a 5-7 membered ring.

12. The use according to any one of claims 7 to 11, wherein, in formula (I), at least one of B or D is independently represented by the formula -(CH₂)ᵣ-(X)-(CH₂)ₛ-Ar, wherein:
r is 0-4;
s is 0-1;
Ar is as defined in claim 1; and
each X is independently selected from the group consisting of CH₂, O, S, SO, SO₂, N, and NR, wherein R is selected from the group consisting of hydrogen, (C1-C4)-straight or branched alkyl, (C2-C4)-straight or branched alkenyl or alkynyl, and (C1-C4) bridging alkyl
wherein a bridge is formed between the nitrogen atom and the Ar group.

13. The use according to claim 7, wherein said compound of formula (I) is selected from the group consisting of:
(S)-1-(2-oxo-2-(3,4,5-trimethoxyphenyl) acetyl)piperidine-2-carboxylic acid-4-pyridin-3-yl-1-(3-pyridin-3-yl)propyl)butyl ester;
(R)-1-(2-oxo-2-(3,4,5-trimethoxyphenyl) acetyl)piperidine-2-carboxylic acid-4-pyridin-3-yl-1-(3-pyridin-3-yl)propyl)butyl ester; and
pharmaceutically acceptable derivatives thereof, and mixtures thereof.

14. The pharmaceutical composition according to any one of claims 4 to 6 for treatment or prevention of multi-drug resistance.

15. The pharmaceutical composition according to any one claims 4-6, wherein the compound is not substantially immunosuppressive at the dosage level required to cause chemosensitization.

16. The use of a compound according to any one of claims 1 to 3 for the preparation of a pharmaceutical composition for treating or preventing multi-drug resistance in a patient.

17. A process for the synthesis of a compound of formula (I'): 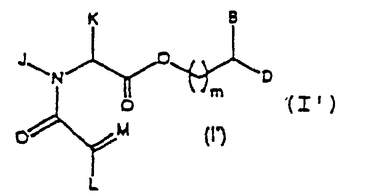 comprising the steps of:
(a) esterifying a protected amino acid of formula (X) with an alcohol of formula (XI): 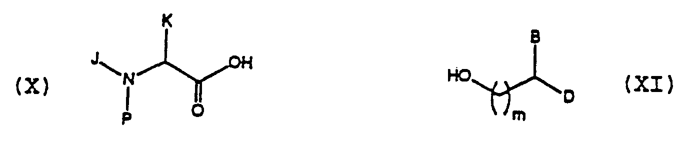 to give an intermediate of formula (XII) : 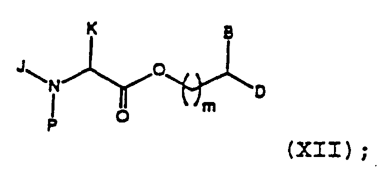
(b) deprotecting the amino protecting group in the intermediate of formula (XII) to give an amino ester of formula (XIII): 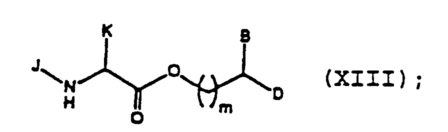 and
(c) acylating the free amino group in the compound of formula (XIII) with a compound of formula (XIV) : 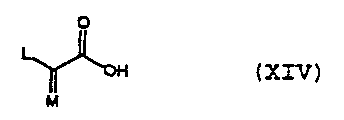 or an activated derivative thereof;
wherein P is a protecting group and A, B, D, J, R, L, and M are defined as in claim 1.

18. A process for the synthesis of a compound of formula (I"): 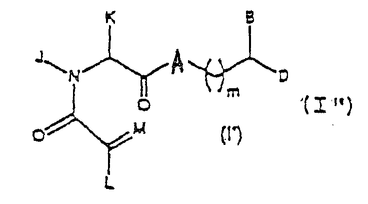 wherein A is NH or N-(C1-C4 alkyl) comprising the steps of:
(a) a peptide coupling reaction between a carboxylic acid of formula (X) and an amine of formula (XI''') 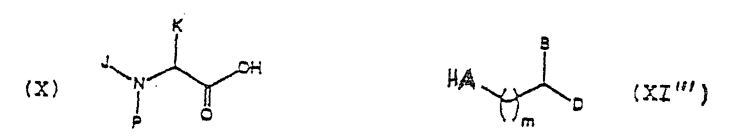 to give an intermediate of formula (XIII'): 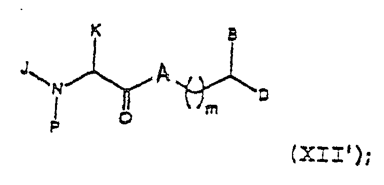
(b) deprotecting the amino protecting group in the intermediate of formula (XII') to give the compound of formula (XIII'): 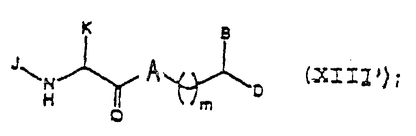 and
(c) acylating the free amino group in the compound of formula (XIII') with a compound of formula (XIV): 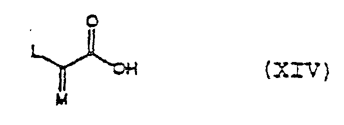 or an activated derivative thereof;
wherein P is a protecting group and A, B, D, J, K, L, and M are defined as in claim 1.

19. The process according to claim 17 or 18, wherein said protecting group is an alkoxycarbonyl group.

20. A process for the preparation of a pharmaceutical composition according to any one of claims 4, 5, 6, 14 or 15 which comprises combining the compound according to any one of claims 1 to 3 with a pharmaceutically acceptable carrier, adjuvant or vehicle and, optionally, a chemotherapeutic agent and/or a chemoserisitizer.

## Patentansprüche

1. Verbindung der Formel (I), 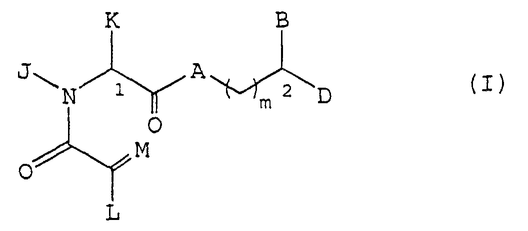 worin A CH₂, ein Sauerstoffatom, eine NH-Gruppe oder ein N-(C1―C4)-Alkylrest ist; worin B und D unabhängig voneinander
(i) Ar, ein gerader oder verzweigter (C1―C10)-Alkyl-, ein gerader oder verzweigter (C2―C10)-Alkenyl- oder -Alkinylrest, ein mit einem (C5―C7)-Cycloalkylrest substituierter gerader oder verzweigter (C1―C6)-Alkylrest, ein gerader oder verzweigter (C2―C6)- Alkenyl- oder -Alkinylrest, ein mit einem (C5―C7)-Cycloalkenylrest substituierter gerader oder verzweigter (C1―C6)-Alkylrest, gerader oder verzweigter (C2―C6)-Alkenyl- oder -Alkinylrest oder ein mit Ar substituierter gerader oder verzweigter (C1― C6)-Alkylrest, ein gerader oder verzweigter (C2―C6)-Alkenyl- oder -Alkinylrest sind, wobei in jedem Fall jeder der CH₂-Reste der Alkyl-, Alkenyl- oder Alkinylketten gegebenenfalls durch ein Heteroatom ersetzt sein kann, ausgewählt aus der Gruppe bestehend aus O, S, SO, SO₂, N und NR, wobei R ausgewählt ist aus der Gruppe bestehend aus einem Wasserstoffatom, einem geraden oder verzweigten (C1―C4)-Alkyl-, einem geraden oder verzweigten (C2―C4)-Alkenyl- oder -Alkinylrest oder einem überbrückenden (C1―C4)- Alkylrest, wobei eine Brücke gebildet wird zwischen dem Stickstoffatom und einem Kohlenstoffatom der Heteroatom-enthaltenen Kette, um einen Ring zu bilden, und wobei der Ring gegebenenfalls an einen Ar-Rest anelliert ist; oder 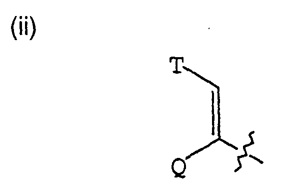
worin Q ein Wasserstoffatom, ein gerader oder verzweigter (C1―C6)-Alkyl- oder ein gerader oder verzweigter (C2―C6)-Alkenyl- oder -Alkinylrest ist; wobei T Ar ist oder ein substituierter 5-7-gliedriger Cycloalkylrest mit Substituenten an den Positionen 3 und 4, welche unabhängig von einander ausgewählt sind aus der Gruppe bestehend aus Oxo, einem Wasserstoffatom, einer Hydroxylgruppe, einem O-(C1―C4)-Alkyl- oder O-(C2―C4)-Alkenylrest; mit der Maßgabe, daß mindestens einer der Substituenten B oder D unabhängig voneinander ausgewählt ist aus der Gruppe bestehend aus einem geraden oder verzweigten (C2-C10)-Alkinylrest, einem mit einem (C5―C7)-Cycloalkylrest substituierten geraden oder verzweigten (C2―C6)-Alkinylrest, einem mit einem (C5―C7)-Cycloalkenylrest substituierten geraden oder verzweigten (C2―C6)-Alkinylrest, und einem mit Ar substituierten geraden oder verzweigten (C2―C6)-Alkinylrest, wobei Ar ein carbocyclischer aromatischer Rest ist, ausgewählt aus der Gruppe bestehend aus einer Phenyl-, 1-Naphthyl-, 2-Naphthyl-, Indenyl-, Azulenyl-, Fluorenyl- und Anthracenylgruppe; oder ein heterocyclischer aromatischer Rest ist, ausgewählt aus der Gruppe bestehend aus einer 2-Furyl-, 3-Furyl, 2-Thienyl-, 3-Thienyl-, 2-Pyridyl-, 3-Pyridyl-, 4-Pyridyl-, Pyrrolyl-, Oxazolyl-, Thiazolyl-, Imidazolyl-, Pyrazolyl-, 2-Pyrazolinyl-, Pyrazolidinyl-, Isoxazolyl-, Isothiazolyl-, 1,2,3-Oxadiazolyl-, 1,2,3-Triazolyl-, 1,3,4-Thiadiazolyl-, Pyridazinyl-, Pyrimidinyl-, Pyrazinyl-, 1,3,5-Triazinyl-, 1,3,5-Trithianyl-, Indolizinyl-, Indolyl-, Isoindolyl-, 3H-Indolyl-, Indolinyl-, Benzo[b]furanyl-, Benzo[b]thiophenyl-, 1H-Indazolyl-, Benzimidazolyl-, Benzthiazolyl-, Purinyl-, 4H-Chinolizinyl-, Chinolinyl-, Isochinolinyl-, Cinnolinyl-, Phthalazinyl-, Chinazolinyl-, Chinoxalinyl-, 1,8-Naphthyridinyl-, Pteridinyl-, Carbazolyl-, Acridinyl-, Phenazinyl-, Phenothiazinyl- und Phenoxazinylgruppe,
wobei Ar ein bis drei Substituenten enthalten kann, welche unabhängig ausgewählt sind aus der Gruppe bestehend aus einem Wasserstoff-, Halogenatom, einer Hydroxyl-, Nitro-, Trifluormethyl-, Trifluormethoxygruppe, einem geraden oder verzweigten (C1―C6)-Alkyl-, einem geraden oder verzweigten (C2―C6)- Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino- und einer Carboxylgruppe, geraden oder verzweigten N-(C1―C5)-Alkyl- oder -Alkenyl-Carboxamidresten, geraden oder verzweigten N,N-di-[(C1―C5)-Alkyloder (C2-C5)-Alkenyl]-Carboxamidresten, einer N-Morpholinocarboxamid-, N-Benzylcarboxamid-, N-Thiomorpholinocarboxamid-, N-Picolinoylcarboxamidgruppe, einem Rest der Formel O-X, CH₂-(CH₂)_{q}-X, O-(CH₂)_{q}-X, (CH₂)_{q}-O-X, und CH=CH-X;
worin X eine 4-Methoxyphenyl-, 2-Pyridyl-, 3-Pyridyl-, 4-Pyridyl-, Pyrazyl-, Chinolyl-, 3,5-Dimethylisoxazoyl-, Isoxazoyl-, 2-Methylthiazoyl-, Thiazoyl-, 2-Thienyl-, 3-Thienyl-, und Pyrimidylgruppe ist, und q 0 ― 2 ist;
worin L entweder ein Wasserstoffatom oder U ist; M entweder ein Sauerstoffatom oder ein Rest der Formel CH-U ist, mit der Maßgabe, daß, wenn L ein Wasserstoffatom ist, M ein Rest der Formel CH-U ist, oder wenn M ein Sauerstoffatom ist, L gleich U ist;
worin U ein Wasserstoffatom, ein gerader oder verzweigter O-(C1―C4)-Alkyl- oder gerader oder verzweigter O-(C2―C4)-Alkenylrest, ein gerader oder verzweigter (C1―C6)-Alkyl- oder gerader oder verzweigter (C2―C6)-Alkenylrest, ein (C5―C7)-Cycloalkyl- oder (C5―C7)-Cycloalkenylrest substituiert mit einem geraden oder verzweigten (C1―C4)- Alkyl- oder einem geradem oder verzweigten (C2―C4)-Alkenylrest oder einem Rest der Formel [(C1―C4)-Alkyl oder (C2―C4)-Alkenyl]- Y oder Y;
worin Y ein carbocyclischer aromatischer Rest ist, ausgewählt aus der Gruppe bestehend aus einer Phenyl-, 1-Naphthyl, 2-Naphthyl-, Indenyl-, Azulenyl-, Fluorenyl- und einer Anthracenylgruppe oder ein heterocyclischer aromatischer Rest wie oben definiert ist; worin Y ein bis drei Substituenten enthalten kann, welche unabhängig voneinander ausgewählt sind aus der Gruppe bestehend aus einem Wasserstoff-, Halogenatom, einer Hydroxyl-, Nitro-, Trifluormethyl-, Trifluormethoxygruppe, einem geraden oder verzweigten (C1―C6)-Alkyl-, einem geraden oder verzweigten (C1―C6)-Alkenylrest, einem geraden oder verzweigten O-(C1―C4)-Alkylrest, geraden oder verzweigten O-(C2―C4)-Alkenylrest, einer O-Benzyl-, O-Phenyl-, 1,2-Methylendioxy-, Amino- und Carboxylgruppe;
worin J ein Wasserstoffatom, ein (C1―C2)-Alkylrest oder eine Benzylgruppe ist; K ein gerader oder verzweigter (C1―C4)-Alkylrest, eine Benzyloder Cyclohexylmethylgruppe ist oder worin J und K zusammen einen 5-7-gliedrigen heterocyclischen Ring bilden können, welcher ein Heteroatom ausgewählt aus der Gruppe bestehend aus O, S, SO und SO₂ enthalten kann; und
worin m 0 - 3 ist.

2. Verbindung der Formel (I): 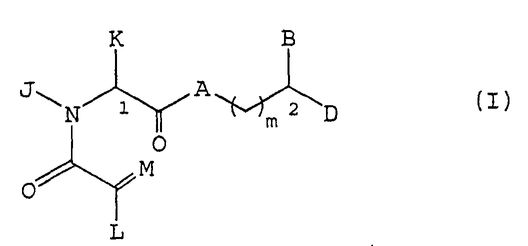 worin A CH₂, ein Sauerstoffatom, eine NH-Gruppe oder ein N-(C1―C4)-Alkylrest ist, worin B und D unabhängig voneinander:
(i) Ar, ein gerader oder verzweigter (C1―C10)- Alkyl-, -Alkenyl- oder -Alkinylrest, ein mit einem (C5―C7)-Cycloalkylrest substituierter gerader oder verzweigter (C1―C6)-Alkyl-, -Alkenyl- oder -Alkinylrest, ein mit einem (C5―C7)-Cycloalkenylrest substituierter gerader oder verzweigter (C1― C6)- Alkyl-, -Alkenyl- oder -Alkinylrest, oder ein mit Ar substituierter gerader oder verzweigter (C1―C6)- Alkyl-, -Alkenyl- oder -Alkinylrest sind,
wobei in jedem Fall jeder der CH₂-Reste der Alkyl-, Alkenyl- oder Alkinylketten gegebenfalls ersetzt sein kann durch ein Heteroatom ausgewählt aus der Gruppe bestehend aus O, S, SO und SO₂; oder ein Rest der Formel 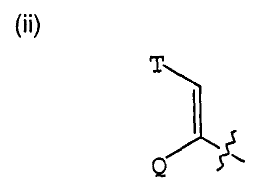 worin Q ein Wasserstoffatom, ein gerader oder verzweigter (C1―C6)-Alkylrest oder ein gerader oder verzweigter (C2―C6)-Alkenylrest ist; worin T Ar ist oder ein substituierter 5 - 7-gliedriger Cycloalkylrest mit Substituenten an den Positionen 3 und 4 ist, welche unabhängig voneinander ausgewählt sind aus der Gruppe bestehend aus Oxo, einem Wasserstoffatom, einer Hydroxylgruppe, einem O-(C1―C4)-Alkyl- und O-(C2―C4)-Alkenylrest; mit der Maßgabe, daß zumindest einer der Substiuenten B oder D unabhängig ausgewählt ist aus der Gruppe bestehend aus einem geraden oder verzweigten (C2―C10)-Alkinylrest, einem mit einem (C5― C7)-Cycloalkylrest substituierten geraden oder verzweigten (C2―C6)-Alkinylrest, einem mit einem (C5―C7)-Cycloalkenylrest substituierten geraden oder verzweigten (C2―C6)-Alkinylrest und einem mit Ar substituierten geraden oder verzweigten (C2―C6)-Alkinylrest;
worin Ar ausgewählt ist aus der Gruppe bestehend aus einer Phenyl-, 1-Naphthyl-, 2-Naphthyl-, 2-Furyl-, 3-Furyl-, 2-Thienyl-, 3-Thienyl-, 2-Pyridyl-, 3-Pyridyl-, 4-Pyridylgruppe, und mono- und bicyclischen heterocyclischen Ringsystemen, wobei die individuelle Ringgröße 5 oder 6 ist, welche entweder in einem oder beiden Ringen insgesamt 1 ― 4 Heteroatome enthalten können, unabhängig ausgewählt aus einem Sauerstoff-, Stickstoff- und einem Schwefelatom;
worin Ar ein bis drei Substituenten enthalten kann, welche unabhängig voneinander ausgewählt sind aus der Gruppe bestehend aus einem Wasserstoff-, Halogenatom, eine Hydroxyl-, Nitro-, Trifluormethyl-, Trifluormethoxygruppe, einem geraden oder verzweigten (C1―C6)-Alkyl-, einem geraden oder verzweigten (C2―C6)-Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino-, Carboxylgruppe, geraden oder verzweigten N-(C1―C5)-Alkyl- oder -Alkenyl-Carboxamidresten, geraden oder verzweigten N,N-di-[(C1―C5)-Alkyloder -Alkenyl]-Carboxamidresten, einer N-Morpholinocarboxamid-, N-Benzylcarboxamid-, N-Thiomorpholinocarboxamid-, N-Picolinoylcarboxamidgruppe, einem Rest der Formel O-X, CH₂-(CH₂)q-X, O-(CH₂)_{q}-X, (CH₂)q-O-X und CH=CH-X; worin X eine 4-Methoxyphenyl-, 2-Pyridyl-, 3-Pyridyl-, 4-Pyridyl-, Pyrazyl-, Chinolyl-, 3,5-Dimethylisoxazoyl-, Isoxazoyl-, 2-Methylthiazoyl-, Thiazoyl-, 2-Thienyl-, 3-Thienyl- und Pyrimidylgruppe ist und q 0 ― 2 ist;
worin L entweder ein Wasserstoffatom oder U ist und M entweder ein Sauerstoffatom oder ein Rest der Formel CH-U ist, mit der Maßgabe, daß wenn L ein Wasserstoffatom ist, M ein Rest der Formel CH-U ist oder wenn M ein Sauerstoffatom ist, L gleich U ist;
worin U ein Wasserstoffatom, ein gerader oder verzweigter O-(C1―C4)-Alkyl- oder ein gerader oder verzweigter O-(C1―C4)-Alkenyl-, ein gerader oder verzweigter (C1―C6)-Alkyl- oder ein gerader oder verzweigter (C1― C6)-Alkenylrest ist, ein mit einem geraden oder verzweigten (C1―C4)-Alkylrest oder einem geraden oder verzweigten (C2-C4)-Alkenylrest substituierter (C5―C7)-Cycloalkyl- oder (C5―C7)-Cycloalkenylrest ist oder ein Rest der Formel [(C1―C4)-Alkyl- oder (C2―C4)-Alkenyl]-Y oder Y ist;
worin Y ausgewählt ist aus der Gruppe bestehend aus einer Phenyl-, 1-Naphthyl-, 2-Naphthyl-, 2-Furyl-, 3-Furyl-, 2-Thienyl-, 3-Thienyl-, 2-Pyridyl-, 3-Pyridyl-, 4-Pyridylgruppe, und mono- und bicyclischen heterocyclischen Ringsystemen, wobei die Größe des einzelnen Ringes zwischen 5 und 6 sein kann, welche entweder in einem oder beiden Ringen insgesamt 1 - 4 Heteroatome enthalten können, unabhängig ausgewählt aus einem Sauerstoffatom, Stickstoffatom und einem Schwefelatom;
worin Y ein bis drei Substituenten enthalten kann, welche unabhängig ausgewählt sind aus der Gruppe bestehend aus einem Wasserstoff-, Halogenatom, einer Hydroxyl-, Nitro-, Trifluormethyl-, Trifluoromethoxygruppe, einem geraden oder verzweigten (C1―C6)- Alkyl-, einem geraden oder verzweigten (C1―C6)-Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino- und Carboxylgruppe;
worin J ein Wasserstoffatom, ein (C1―C2)-Alkylrest oder eine Benzylgruppe ist, K ein gerader oder verzweigter (C1―C4)-Alkylrest, eine Benzyloder Cyclohexylmethylgruppe ist oder worin J und K zusammen einen 5-7-gliedrigen heterocyclischen Ring bilden können, welcher einen O-, S-, SO- oder SO₂-Substituenten enthalten kann; und
worin m 0 ― 3 ist.

3. Verbindung der Formel (I): 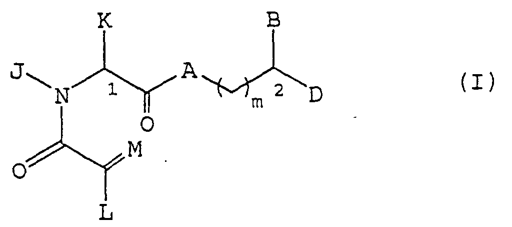 worin A CH₂, ein Sauerstoffatom, eine NH-Gruppe oder ein N-(C1-C4)-Alkylrest ist;
worin B und D unabhängig voneinander
worin B und D unabhängig voneinander
(i) Ar, ein gerader oder verzweigter (C1―C10)-Alkyl-, ein gerader oder verzweigter (C2―C10)-Alkenyl- oder -Alkinylrest, ein mit einem (C5― C7)-Cycloalkylrest substituierter gerader oder verzweigter (C1―C6)-Alkylrest, gerader oder verzweigter (C2―C6)-Alkenyl- oder -Alkinylrest, ein mit einem (C5―C7)-Cycloalkenylrest substituierter gerader oder verzweigter (C1―C6)-Alkylrest, ein gerader oder verzweigter (C2―C6)-Alkenyl- oder -Alkinylrest, oder ein mit Ar substituierter gerader oder verzweigter (C1―C6)-Alkyl-, gerader oder verzweigter (C2―C6)-Alkenyloder -Alkinylrest sind, worin in jedem Fall jeder der CH₂-Reste der Alkyl-, Alkenyl- oder Alkinylketten gegebenenfalls durch ein Heteroatom ersetzt sein kann, ausgewählt aus der Gruppe bestehend aus O, S, SO, SO₂, N und NR, worin R ausgewählt ist aus der Gruppe bestehend aus einem Wasserstoffatom, einem geraden oder verzweigten (C1― C4)-Alkyl-, geraden oder verzweigten (C2―C4)-Alkenyl- oder -Alkinylrest, und einem verbrückenden (C1―C4)-Alkylrest, wobei eine Brücke gebildet wird zwischen dem Stickstoffatom und einem Kohlenstoffatom der Heteroatom-enthaltenden Kette, um einen Ring zu bilden, und worin der Ring gegebenenfalls an einen Ar-Rest anelliert ist; oder ein Rest der Formel 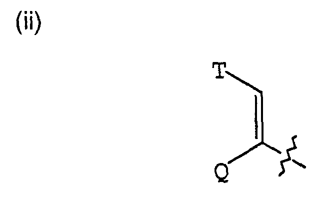
sind,
worin Q ein Wasserstoffatom, ein gerader oder verzweigter (C1―C6)-Alkyl-, oder gerader oder verzweigter (C2―C6)-Alkenyl- oder -Alkinylrest ist;
worin T Ar ist oder ein substituierter 5 ― 7-gliedriger Cycloalkyfrest ist, mit Substituenten an den Positionen 3 und 4, welche unabhängig voneinander ausgewählt sind aus der Gruppe bestehend aus Oxo, einem Wasserstoffatom, einer Hydroxylgruppe, einem O-(C1―C4)-Alkyl- und O-(C2―C4)-Alkenylrest; mit der Maßgabe, daß mindestens einer der Substituenten B oder D unabhängig voneinander ausgewählt ist aus der Gruppe bestehend aus Ar', einem mit Ar' substituierten geraden oder verzweigten (C1― C6)-Alkylrest und einem mit Ar' substituierten geraden oder verzweigten (C2 ― C6)-Alkenyl- oder -Alkinylrest;
wobei Ar' ein Ar-Rest ist, substituiert mit ein bis drei Substituenten, welche unabhängig voneinander ausgewählt sind, aus der Gruppe aus geraden oder verzweigten N-(C1―C5)-Alkyl- oder (C2―C5)-Alkenyl-Carboxamidresten, geraden oder verzweigten N,N-di-[(C1―C5)-Alkyloder (C2―C5)-Alkenyl)]-Carboxamidresten, einer N-Morpholinocarboxamid-, N-Benzylcarboxamid-, N-Thiomorpholinocarboxamid-, N-Picolinoylcarboxamidgruppe, O-X, CH₂-(CH₂)_{q}-X, O-(CH₂)_{q}-X, (CH₂)_{q}-O-X und CH=CH-X; worin X eine 4-Methoxyphenyl-, 2-Pyridyl-, 3-Pyridyl-, 4-Pyridyl-, Pyrazyl-, Chinolyl-, 3,5-Dimethylisoxazoyl-, Isoxazoyl-, 2-Methylthiazoyl-, Thiazoyl-, 2-Thienyl-, 3-Thienyl- und Pyrimidylgruppe ist;
worin q 0 ― 2 ist;
worin Ar ein carbocyclischer aromatischer Rest ist ausgewählt aus der Gruppe bestehend aus einer Phenyl-, 1-Naphthyl, 2-Naphthyl-, Indenyl-, Azulenyl-, Fluorenyl- und einer Anthracenylgruppe, oder ein heterocyclischer aromatischer Rest ist, ausgewählt aus der Gruppe bestehend aus 2-Furyl-, 3-Furyl-, 2-Thienyl-, 3-Thienyl-, 2-Pyridyl-, 3-Pyridyl-, 4-Pyridyl-, Pyrrolyl-, Oxazolyl-, Thiazolyl-, Imidazolyl-, Pyrazolyl-, 2-Pyrazolinyl-, Pyrazolidinyl-, Isoxazolyl-, Isothiazolyl-, 1,2,3-Oxadiazolyl-, 1,2,3-Triazolyl-, 1,3,4-Thiadiazolyl-, Pyridazinyl-, Pyrimidinyl-, Pyrazinyl-, 1,3,5-Triazinyl-, 1,3,5-Trithianyl-, Indolizinyl-, Indolyl-, Isoindolyl-, 3H-Indolyl-, Indolinyl-, Benzo[b]furanyl-, Benzo[b]thiophenyl-, 1H-Indazolyl-, Benzimidazolyl-, Benzthiazolyl-, Purinyl-, 4H-Chinolizinyl-, Chinolinyl-, Isochinolinyl-, Cinnolinyl-, Phthalazinyl-, Chinazolinyl-, Chinoxalinyl-, 1,8-Naphthyridinyl-, Pteridinyl-, Carbazolyl-, Acridinyl-, Phenazinyl-, Phenothiazinyl- und Phenoxazinylgruppe ist;
worin Ar ein bis drei Substituenten enthalten kann, welche unabhängig voneinander ausgewählt sind aus der Gruppe bestehend aus einem Wasserstoff-, Halogenatom, einer Hydroxyl-, Nitro-, Trifluormethyl-, Trifluormethoxygruppe, einem geraden oder verzweigten (C1―C6)-Alkyl-, einem geraden oder verzweigten (C2―C6)-Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino-, Carboxylgruppe, geraden oder verzweigten N-(C1―C5)-Alkyl- oder -Alkenyl-Carboxamidresten, geraden oder verzweigten N,N-di-[(C1―C5)-Alkyloder (C2―C5)-Alkenyl]-Carboxamidresten, einer N-Morpholinocarboxamid-, N-Benzylcarboxamid-, N-Thiomorpholinocarboxamid-, N-Picolinoylcarboxamidgruppe, einem Rest der Formel O-X, CH₂-(CH₂)_{q}-X, O-(CH₂)q-X, (CH₂)_{q}-O-X und CH=CH-X;
worin X eine 4-Methoxyphenyl-, 2-Pyridyl-, 3-Pyridyl-, 4-Pyridyl-, Pyrazyl-, Chinolyl-, 3,5-Dimethylisoxazoyl-, Isoxazoyl-, 2-Methylthiazoyl-, Thiazoyl-, 2-Thienyl-, 3-Thienyl- und Pyrimidylgruppe und q 0―2 ist; worin L entweder ein Wasserstoffatom oder U ist und M entweder ein Sauerstoffatom oder ein Rest der Formel CH-U ist, mit der Maßgabe, daß wenn L ein Wasserstoffatom ist, M ein Rest der Formel CH-U ist oder wenn M ein Sauerstoffatom ist, L gleich U ist;
worin U ein Wasserstoffatom, ein gerader oder verzweigter O-(C1―C4)-Alkyl- oder ein gerader oder verzweigter O-(C2―C4)-Alkenyl-, ein gerader oder verzweigter (C1―C6)-Alkyl- oder ein gerader oder verzweigter (C2― C6)-Alkenylrest ist, ein mit einem geraden oder verzweigten (C1―C4)-Alkylrest oder geraden oder verzweigten (C2―C4)-Alkenylrest substituierter (C5―C7)-Cycloalkyl- oder (C5―C7)-Cycloalkenylrest ist oder ein Rest der Formel [(C1―C4)-Alkyl- oder (C2―C4)-Alkenyl]-Y oder Y ist;
worin Y ausgewählt ist aus der Gruppe bestehend aus einer einer Phenyl-, 1-Naphthyl-, 2-Naphthyl-, Indenyl-, Azulenyl-, Fluorenyl- und Anthracenylgruppe und heterocyclischen aromatischen Resten, wie oben definiert;
worin Y ein bis drei Substituenten enthalten kann, welche unabhängig ausgewählt sind aus der Gruppe bestehend aus einem Wasserstoff-, Halogenatom, einer Hydroxyl-, Nitro-, Trifluormethyl-, Trifluormethoxygruppe, einem geraden oder verzweigtem (C1―C6)-Alkyl-, einem geraden oder verzweigten (C1―C6)-Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino- und Carboxylgruppe;
worin J ein Wasserstoffatom, ein (C1―C2)-Alkylrest oder eine Benzylgruppe ist; K ein gerader oder verzweigter (C1-C4)-Alkylrest, eine Benzyloder Cyclohexylmethylgruppe ist oder worin J und K zusammen einen 5-7-gliedrigen heterocyclischen Ring bilden können, welcher ein Heteroatom ausgewählt aus der Gruppe bestehend aus O, S, SO und SO₂ enthalten kann; und
worin m 0 - 3 ist.

4. Arzneimittel, umfassend eine Verbindung nach einem der Ansprüche 1 - 3 und einem pharmazeutisch verträglichen Träger, Hilfsstoff oder Vehikel.

5. Arzneimittel nach Anspruch 4, zusätzlich umfassend ein Chemotherapeutikum.

6. Arzneimittel nach Anspruch 4 oder 5, zusätzlich umfassend einen Chemosensitizer, welcher verschieden ist von der Verbindung nach einem der Ansprüche 1 bis 3.

7. Verwendung einer Verbindung der Formel (I) zur Herstellung eines Medikaments zur Behandlung oder Vorbeugung von Multi-Drug-Resistenz in einem Patienten, dadurch charakterisiert, daß die Verbindung die folgende Formel (I) hat: 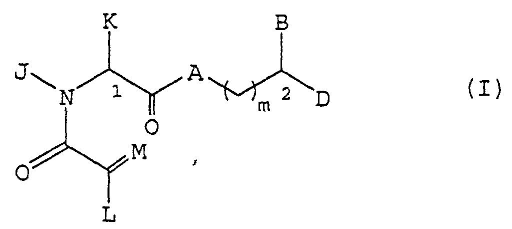 worin A CH₂, ein Sauerstoffatom, eine NH-Gruppe oder ein N-(C1―C4)-Alkylrest ist; worin B und D unabhängig voneinander
(i) ein Wasserstoffatom, Ar, ein gerader oder verzweigter (C1―C10)-Alkyl-, ein gerader oder verzweigter (C2―C10)-Alkenyl- oder -Alkinylrest, ein mit einem (C5―C7)-Cycloalkylrest substituierter gerader oder verzweigter (C1―C6)-Alkylrest, ein gerader oder verzweigter (C2―C6)-Alkenyl- oder -Alkinylrest, ein mit einem (C5―C7)- Cycloalkenylrest substituierter gerader oder verzweigter (C1―C6)-Alkylrest, ein gerader oder verzweigter (C2-C6)-Alkenyl- oder -Alkinylrest oder ein mit Ar substituierter gerader oder verzweigter (C1―C6)-Alkylrest, ein gerader oder verzweigter (C2― C6)- Alkenyl- oder -Alkinylrest sind, wobei in jedem Fall jeder der CH₂-Reste der Alkyl-, Alkenyl- oder Alkinylketten gegebenenfalls durch ein Heteroatom ersetzt sein kann, ausgewählt aus der Gruppe bestehend aus O, S, SO, SO₂, N und NR, wobei R ausgewählt ist aus der Gruppe bestehend aus einem Wasserstoffatom, einem geraden oder verzweigten (C1― C4)-Alkyl-, einem geraden oder verzweigten (C2―C4)-Alkenyl- oder -Alkinylrest oder einem überbrückenden (C1―C4)-Alkylrest, wobei eine Brücke gebildet wird zwischen dem Stickstoffatom und einem Kohlenstoffatom der Heteroatom-enthaltenen Kette, um einen Ring zu bilden, und wobei der Ring gegebenenfalls an einen Ar-Rest anelliert ist; oder einen Rest der Formel 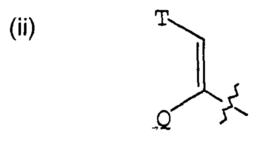
sind,
worin Q ein Wasserstoffatom, ein gerader oder verzweigter (C1―C6)-Alkyl- oder gerader oder verzweigter (C2―C6)-Alkenyl- oder -Alkinylrest;
worin T Ar ist oder ein substituierter 5 ― 7-gliedriger Cycloalkylrest mit Substituenten an den Positionen 3 und 4 ist, welche unabhängig von einander ausgewählt sind aus der Gruppe bestehend aus Oxo, einem Wasserstoffatom, einer Hydroxylgruppe, einem O-(C1―C4)-Alkyl- oder O-(C2― C4)-Alkenylrest ist;
worin Ar ein carbocyclischer aromatischer Rest ist ausgewählt aus der Gruppe bestehend aus einer Phenyl-, 1-Naphthyl-, 2-Naphthyl-, Indenyl-, Azulenyl-, Fluorenyl- und Anthracenylgruppe; oder ein heterocyclischer aromatischer Rest ist, ausgewählt aus der Gruppe bestehend aus einer 2-Furyl-, 3-Furyl, 2-Thienyl-, 3-Thienyl-, 2-Pyridyl-, 3-Pyridyl-, 4-Pyridyl-, Pyrrolyl-, Oxazolyl-, Thiazolyl-, Imidazolyl-, Pyrazolyl-, 2-Pyrazolinyl-, Pyrazolidinyl-, Isoxazolyl-, Isothiazolyl-, 1,2,3-Oxadiazolyl-, 1,2,3-Triazolyl-, 1,3,4-Thiadiazolyl-, Pyridazinyl-, Pyrimidinyl-, Pyrazinyl-, 1,3,5-Triazinyl-, 1,3,5-Trithianyl-, Indolizinyl-, Indolyl-, Isoindolyl-, 3H-Indolyl-, Indolinyl-, Benzo[b]furanyl-, Benzo[b]thiophenyl-, 1H-Indazolyl-, Benzimidazolyl-, Benzthiazolyl-, Purinyl-, 4H-Chinolizinyl-, Chinolinyl-, Isochinolinyl-, Cinnolinyl-, Phthalazinyl-, Chinazolinyl-, Chinoxalinyl-, 1,8-Naphthyridinyl-, Pteridinyl-, Carbazolyl-, Acridinyl-, Phenazinyl-, Phenothiazinyl- und Phenoxazinylgruppe ist;
wobei Ar ein bis drei Substituenten enthalten kann, welche ausgewählt sind aus der Gruppe bestehend aus einem Wasserstoff-, Halogenatom, einer Hydroxyl-, Nitro-, Trifluormethyl-, Trifluormethoxygruppe, einem geraden oder verzweigtem (C1―C6)-Alkyl-, einem geraden oder verzweigten (C2―C6)-Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino- und Carboxylgruppe, geraden oder verzweigten N-(C1―C5)-Alkyl- oder -Alkenyl-Carboxamidresten, geraden oder verzweigten N,N-di-[(C1―C5)-Alkyl oder (C2―C5)-Alkenyl)]-Carboxamidresten, einer N-Morpholinocarboxamid-, N-Benzylcarboxamid-N-Thiomorpholinocarboxamid-, N-Picolinoylcaboxamidgruppe, ein Rest der Formel O-X, CH₂-(CH₂)_{q}-X, O-(CH₂)_{q}-X, (CH₂)_{q}-O-X, und CH=CH-X;
worin X eine 4-Methoxyphenyl-, 2-Pyridyl-, 3-Pyridyl-, 4-Pyridyl-, Pyrazyl-, Chinolyl-, 3,5-Dimethylisoxazoyl-, lsoxazoyl-, 2-Methylthiazoyl-, Thiazoyl-, 2-Thienyl-, 3-Thienyl- und Pyrimidylgruppe ist, und q 0-2 ist;
worin L entweder ein Wasserstoffatom oder U ist; M entweder ein Sauerstoffatom oder ein Rest der Formel CH-U ist, mit der Maßgabe, daß wenn L ein Wasserstoffatom ist, M ein Rest der Formel CH-U ist oder wenn M ein Sauerstoffatom ist, L gleich U ist;
worin U ein Wasserstoffatom, ein gerader oder verzweigter O-(C1―C4)-Alkyl- oder gerader oder verzweigter O-(C2―C4)-Alkenylrest, ein gerader oder verzweigter (C1―C6)-Alkyl- oder gerader oder verzweigter (C2―C6)-Alkenylrest, ein (C5―C7)-Cycloalkyl- oder (C5―C7)-Cycloalkenylrest substituiert mit einem geraden oder verzweigten (C1―C4)-Alkyl- oder einem geraden oder verzweigten (C2―C4)-Alkenylrest ist oder einem Rest der Formel [(C1―C4)-Alkyl oder (C2―C4)-Alkenyl]-Y oder Y ist;
worin Y ausgewählt ist aus der Gruppe bestehend aus einer Phenyl-, 1-Naphthyl, 2-Naphthyl-, Indenyl-, Azulenyl-, Fluorenyl-, Anthracenyl-, 2-Pyrrolinyl-, 3-Pyrrolinyl-, Pyrolidinyl-, 1,3-Dioxolyl-, 2-Imidazolinyl-, Imidazolidinyl-, 2H-Pyranyl-, 4H-Pyranyl-, Piperidyl-, 1,4-Dioxanyl-, Morpholinyl-, 1,4-Dithianyl-, Thiomorpholinyl-, Piperazinyl-, Chinuclidinylgruppe oder einem heterocyclischen aromatischen Rest, der wie oben definiert ist;
worin Y ein bis drei Substituenten enthalten kann, welche unabhängig voneinander ausgewählt sind aus der Gruppe bestehend aus einem Wasserstoff-, Halogenatom, einer Hydroxyl-, Nitro-, Trifluormethyl-, Trifluormethoxygruppe, einem geraden oder verzweigten (C1―C6)-Alkyl-, einem geraden oder verzweigten (C1―C6)-Alkenylrest, einem geraden oder verzweigten O-(C1―C4)-Alkylrest, einem geraden oder verzweigten O-(C2― C4)-Alkenylrest, einer O-Benzyl-, O-Phenyl-, 1,2-Methylendioxy-, Aminound Carboxylgruppe;
worin J ein Wasserstoffatom, ein (C1∗C2)-Alkylrest oder eine Benzylgruppe ist; K ein gerader oder verzweigter (C1―C4)-Alkylrest, eine Benzyloder Cyclohexylmethylgruppe ist oder worin J und K zusammen einen 5― 7-gliedrigen heterocyclischen Ring bilden können, welcher ein Heteroatom ausgewählt aus der Gruppe bestehend aus O, S, SO und SO₂ enthalten kann; und
worin m 0 ― 3 ist.

8. Verwendung einer Verbindung der Formel (I) zur Herstellung eines Medikaments zur Behandlung oder Vorbeugung von Multi-Drug-Resistenz in einem Patienten, **dadurch gekennzeichnet, daß** die Verbindung die folgende Formel hat: 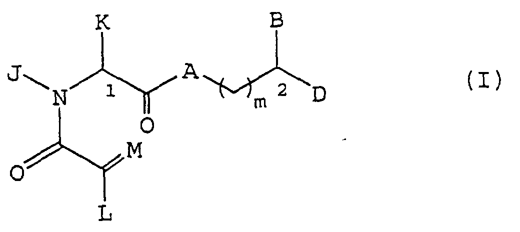 worin A CH₂, ein Sauerstoffatom, eine NH-Gruppe oder ein N-(C1―C4)-Alkylrest ist, worin B und D unabhängig voneinander:
(i) Ar, ein gerader oder verzweigter (C1―C10)-Alkyl-, ein gerader oder verzweigter (C2―C10)-Alkenyl- oder -Alkinylrest, ein mit einem (C5―C7)-Cycloalkylrest substituierter gerader oder verzweigter (C1-C6)- Alkyl-, -Alkenyl- oder -Alkinylrest, ein mit einem (C5―C7)-Cycloalkenylrest substituierter gerader oder verzweigter (C1―C6)-Alkyl-, -Alkenyl- oder -Alkinylrest, oder ein mit Ar substituierter gerader oder verzweigter (C1― C6)-Alkyl-, -Alkenyl- oder -Alkinylrest sind, wobei in jedem Fall jeder der CH₂-Reste der Alkyl-, Alkenyl- oder Alkinylketten gegebenfalls ersetzt sein kann durch ein Heteroatom ausgewählt aus der Gruppe bestehend aus O, S, SO und SO₂; oder ein Rest der Formel 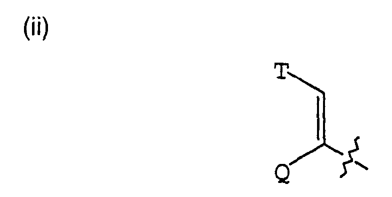 sind,
worin Q ein Wasserstoffatom, ein gerader oder verzweigter (C1―C6)-Alkylrest oder ein gerader oder verzweigter (C1―C6)-Alkenylrest ist;
worin T Ar oder ein substituierter 5 - 7-gliedriger Cycloalkylrest mit Substituenten an den Positionen 3 und 4 ist, welche unabhängig voneinander ausgewählt sind aus der Gruppe bestehend aus Oxo, einem Wasserstoffatom, einer Hydroxylgruppe, einem O-(C1―C4)-Alkyl-, und O-(C1―C4)-Alkenylrest;
worin Ar ausgewählt ist aus der Gruppe bestehend aus einer Phenyl-, 1-Naphthyl-, 2-Naphthyl-, 2-Furyl-, 3-Furyl-, 2-Thienyl-, 3-Thienyl-, 2-Pyridyl-, 3-Pyridyl-, 4-Pyridylgruppe und mono- und bicyclischen heterocyclischen Ringsystemen, wobei die individuelle Ringgröße 5 oder 6 ist, welche entweder in einem oder beiden Ringen insgesamt 1 ― 4 Heteroatome enthalten können, unabhängig ausgewählt von einem Sauerstoff-, Stickstoffund einem Schwefelatom;
worin Ar ein bis drei Substituenten enthalten kann, welche unabhängig voneinander ausgewählt sind aus der Gruppe bestehend aus einem Wasserstoff-, Halogenatom, eine Hydroxyl-, Nitro-, Trifluormethyl-, Trifluormethoxygruppe, einem geraden oder verzweigten (C1―C6)-Alkyl-, einem geraden oder verzweigten (C2―C6)-Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino-, Carboxylgruppe, geraden oder verzweigten N-(C1―C5)-Alkyl- oder -Alkenyl-Carboxamidresten, geraden oder verzweigten N,N-di-[(C1―C5)-Alkyloder (C2―C5)-Alkenyl]-Carboxamidresten, einer N-Morpholinocarboxamid-, N-Benzylcarboxamid-, N-Thiomorpholinocarboxamid-, N-Picolinoylcarboxamidgruppe, einem Rest der Formel O-X, CH₂-(CH₂)_{q}-X, O-(CH₂)_{q}-X, (CH₂)_{q}-O-X und CH=CH-X; worin X eine 4-Methoxyphenyl-, 2-Pyridyl-, 3-Pyridyl-, 4-Pyridyl-, Pyrazyl-, Chinolyl-, 3,5-Dimethylisoxazoyl-, Isoxazoyl-, 2-Methylthiazoyl-, Thiazoyl-, 2-Thienyl-, 3-Thienyl- und Pyrimidylgruppe und q 0 ― 2 ist;
worin L entweder ein Wasserstoffatom oder U ist und M entweder ein Sauerstoffatom oder ein Rest der Formel CH-U, mit der Maßgabe, daß wenn L ein Wasserstoffatom ist, M ein Rest der Formel CH-U ist oder wenn M ein Sauerstoffatom ist, L gleich U ist;
worin U ein Wasserstoffatom, ein gerader oder verzweigter O-(C1―C4)-Alkyl- oder ein gerader oder verzweigter O-(C1―C4)-Alkenyl-, ein gerader oder verzweigter (C1―C6)-Alkyl- oder ein gerader oder verzweigter (C1― C6)-Alkenylrest ist, ein mit einem geraden oder verzweigten (C1―C4)-Alkylrest oder geradem oder verzweigten (C2―C4)-Alkenylrest substituierter (C5―C7)-Cycloalkyl- oder (C5―C7)-Cycloalkenylrest oder ein Rest der Formel [(C1―C4)-Alkyl- oder (C2―C4)-Alkenyl]-Y oder Y ist;
worin Y ausgewählt ist aus der Gruppe bestehend aus einer Phenyl-, 1-Naphthyl-, 2-Naphthyl-, 2-Furyl-, 3-Furyl-, 2-Thienyl-, 3-Thienyl-, 2-Pyridyl-, 3-Pyridyl-, 4-Pyridylgruppe und mono- und bicyclischen heterocyclischen Ringsystemen, wobei die Größe des einzelnen Ringes zwischen 5 und 6 sein kann, welche entweder in einem oder beiden Ringen insgesamt 1 ― 4 Heteroatome enthalten können, unabhängig ausgewählt aus einem Sauerstoffatom, Stickstoffatom und einem Schwefelatom;
worin Y ein bis drei Substituenten enthalten kann, welche unabhängig ausgewählt sind aus der Gruppe bestehend aus einem Wasserstoff-, Halogenatom, einer Hydroxyl-, Nitro-, Trifluormethyl-, Trifluormethoxygruppe, einem geraden oder verzweigten (C1―C6)-Alkyl-, einem geraden oder verzweigten (C1―C6)-Alkenylrest, einem geraden oder verzweigten O-(C1―C4)-Alkyl-, einem geraden oder verzweigten O-(C2―C4)-Alkenylrest, einer O-Benzyl-, O-Phenyl-, 1,2-Methylendioxy-, Amino- und Carboxylgruppe;
worin J ein Wasserstoffatom, ein (C1―C2)-Alkylrest oder eine Benzylgruppe ist, K ein gerader oder verzweigter (C1―C4)-Alkylrest, eine Benzyloder Cyclohexylmethylgruppe ist oder worin J und K zusammen einen 5― 7-gliedrigen heterocyclischen Ring bilden können, welcher ein Heteroatom ausgewählt aus der Gruppe bestehend aus O, S, SO oder SO₂ enthalten kann; und
worin m 0―3 ist.

9. Verwendung nach Anspruch 7 oder 8, worin in Formel (I) zumindest einer der Substituenten B oder D unabhängig voneinander ausgewählt ist aus der Gruppe bestehend aus einem geraden oder verzweigten (C2―C10)-Alkinylrest, einem mit einem (C5―C7)-Cycloalkylrest substituierten geraden oder verzweigten (C2―C6)-Alkinylrest; einem mit einem (C5―C7)-Cycloalkenylrest substituierten geraden oder verzweigten (C2―C6)-Alkinylrest und einem mit Ar substituierten geraden oder verzweigten (C2―C6)-Alkinylrest.

10. Verwendung nach Anspruch 7 oder 8, worin in Formel (I) zumindest einer der Substituenten B oder D unabhängig voneinander ausgewählt ist aus der Gruppe bestehend aus Ar', einem mit Ar' substituierten geraden oder verzweigten (C1―C6)-Alkylrest und einem mit Ar' substituierten geraden oder verzweigten (C2―C6)-Alkenyl- oder -Alkinylrest;
worin Ar' ein Ar-Rest ist, welcher mit ein bis drei Substituenten substituiert ist, welche unabhängig voneinander ausgewählt sind aus der Gruppe bestehend aus geraden oder verzweigten N-(C1―C5)-Alkyl- oder (C2―C5)-Alkenyl-Carboxamidresten, geraden oder verzweigten N,N-di-[(C1―C5)-Alkyl- oder (C2―C5)-Alkenyl]-Carboxamidresten, einer N-Morpholinocarboxamid-, N-Benzylcarboxamid-, N-Thiomorpholinocarboxamid-, N-Picolinoylcarboxamidgruppe, einem Rest der Formel O-X, CH₂-(CH₂)_{q}-X, O-(CH₂)_{q}-X, (CH₂)_{q}-O-X und CH=CH-X;
worin X eine 4-Methoxyphenyl-, 2-Pyridyl-, 3-Pyridyl-, 4-Pyridyl-, Pyrazyl-, Chinolyl-, 3,5-Dimethylisoxazoyl-, Isoxazoyl-, 2-Methylthiazoyl-, Thiazoyl-, 2-Thienyl-, 3-Thienyl- und Pyrimidylgruppe ist,
wobei q 0―2 ist.

11. Verwendung nach einem der Ansprüche 7 bis 10, wobei in Formel (I) J und K zusammen einen 5 ― 7-gliedrigen Ring bilden.

12. Verwendung nach einem der Ansprüche 7 bis 11, wobei in Formel (I) zumindest einer der Substituenten B oder D unabhängig voneinander dargestellt wird durch die Formel (CH₂)ᵣ-(X)-(CH₂)ₛ-Ar,
worin:
r 0 - 4 ist;
s 0 -1 ist;
Ar wie in Anspruch 1 definiert ist; und
jedes X unabhängig voneinander ausgewählt ist
aus der Gruppe bestehend aus CH₂, O, S, SO, SO₂, N und NR, worin R ausgewählt ist aus der Gruppe bestehend aus einem Wasserstoffatom, einem geraden oder verzweigten (C1―C4)-Alkyl-, einem geraden oder verzweigten (C2―C4)-Alkenyl- oder -Alkinylrest und einem verbrückenden (C1―C4)-Alkylrest, wobei eine Brücke gebildet wird zwischen dem Stickstoffatom und dem Ar-Rest.

13. Verwendung nach Anspruch 7, wobei die Verbindung der Formel (I) ausgewählt ist aus der Gruppe bestehend aus:
(S)-1-(2-Oxo-2-(3,4,5-trimethoxyphenyl)acetyl)piperidin-2-carbonsäure-4-pyridin-3-yl-1-(3-pyridin-3-yl)propyl)butylester;
(R)-1-(2-Oxo-2-(3,4,5-trimethoxyphenyl)acetyl)piperidin-2-carbonsäure-4-pyridin-3-yl-1-(3-pyridin-3-yl)propyl)butylester;
pharmazeutisch verträglichen Derivaten davon und Mischungen davon.

14. Arzneimittel nach einem der Ansprüche 4 bis 6 zur Behandlung oder Vorbeugung von Multi-Drug-Resistenz.

15. Arzneimittel nach einem der Ansprüche 4 bis 6, wobei die Verbindung bei der Dosierung, welche für die Auslösung der Chemosensitivierung benötigt wird, im wesentlichen nicht immunsuppressiv wirkt.

16. Verwendung einer Verbindung nach einem der Ansprüche 1 bis 3 zur Herstellung eines Arzneimittels zur Behandlung oder Vorbeugung von Multi-Drug-Resistenz in einem Patienten.

17. Verfahren zur Synthese einer Verbindung der Formel (I') 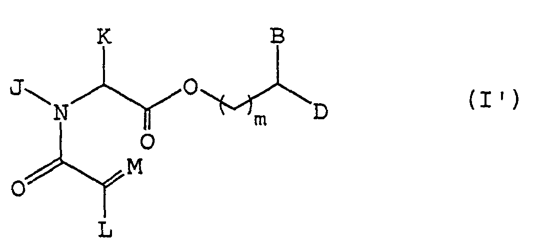 umfassend die Schritte von:
(a) Veresterung einer geschützten Aminosäure der Formel (X) mit einem Alkohol der Formel (XI): 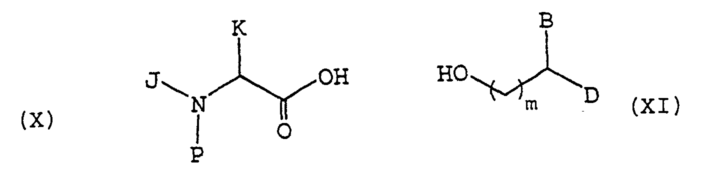 zur Herstellung eines Zwischenprodukts der Formel (XII): 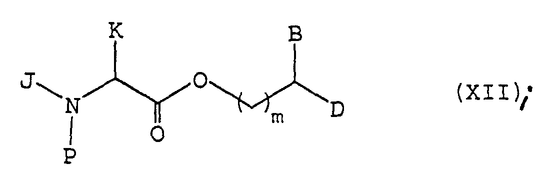
(b) Entfernen der Aminoschutzgruppe in dem Zwischenprodukt der Formel (XII) zur Herstellung eines Aminoesters der Formel (XIII): 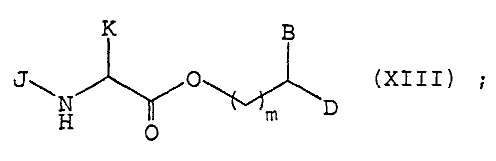 und
(c) Acylierung der freien Aminogruppe in der Verbindung der Formel (XIII) mit einer Verbindung der Formel (XIV): 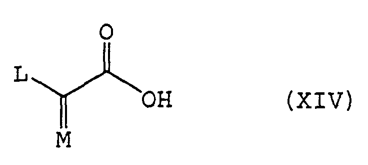 oder einem aktivierten Derivat davon; wobei P eine Schutzgruppe ist und A, B, D, J, K, L und M wie in Anspruch 1 definiert sind.

18. Verfahren zur Synthese einer Verbindung der Formel (I"): 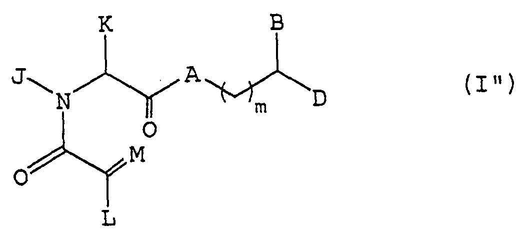 wobei A eine NH-Gruppe oder ein N-(C1-C4)-Alkylrest ist, umfassend die Schritte:
(a) Peptid-Kupplungsreaktion zwischen einer Carbonsäure der Formel (X) und einem Amin der Formel (XI''') 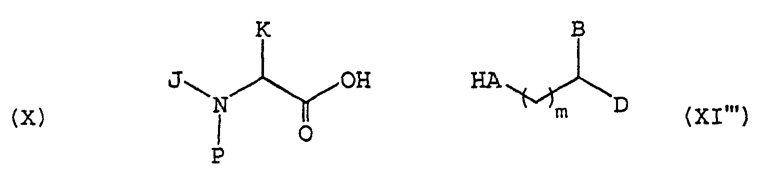 zur Synthese eines Zwischenprodukts der Formel (XII'): 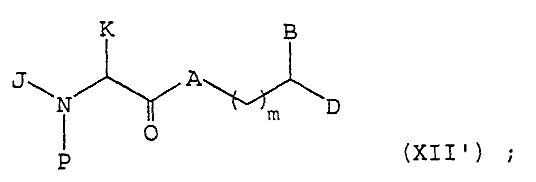
(b) Entfernen der Aminoschutzgruppe in dem Zwischenprodukt der Formel (XII') zur Herstellung einer Verbindung der Formel (XIII'): 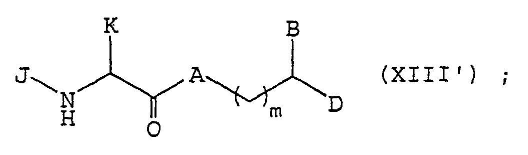 und
(c) Acylierung der freien Aminogruppe in der Verbindung der Formel (XIII') mit einer Verbindung der Formel (XIV): 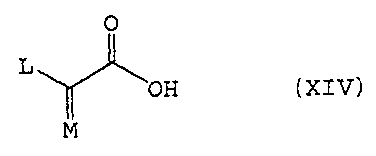 oder einem aktivierten Derivat dieser Verbindung;
wobei P eine Schutzgruppe ist und A, B, D, J, K, L und M wie in Anspruch 1 definiert sind.

19. Verfahren nach Anspruch 17 oder 18, wobei die Schutzgruppe ein Alkoxycarbonylrest ist.

20. Verfahren zur Herstellung eines Arzneimittels nach einem der Ansprüche 4, 5, 6, 14 oder 15, umfassend das Kombinieren der Verbindung nach einem der Ansprüche 1 bis 3 mit einem pharmazeutisch verträglichen Träger, Adjuvans oder Vehikel und, gegebenenfalls, einem chemotherapeutischen Mittel und/oder einem Chemosensitizer.

## Revendications

1. Composé de formule (I) : 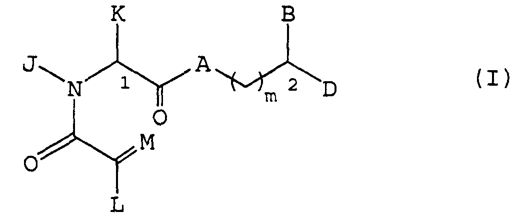 dans lequel A est CH₂, l'oxygène, NH ou N-(alkyle en C1-C4) ;
dans lequel B et D sont indépendamment :
(i) Ar, un radical alkyle (en C1-C10) linéaire ou ramifié, alcényle ou alcynyle (en C2-C10) linéaire ou ramifié, un radical alkyle en (C1-C6) linéaire ou ramifié, alcényle ou alcynyle en (C2-C6) linéaire ou ramifié, substitués par un radical cycloalkyle (en C5-C7), alkyle (en C1-C6) linéaire ou ramifié, alcényle ou alcynyle (en C2-C6) linéaire ou ramifié, substitués par un radical cycloalcényle (en C5-C7), ou alkyle (en C1-C6) linéaire ou ramifié, alcényle ou alcynyle (en C2-C6) linéaire ou ramifié substitué par un radical Ar, dans lequel, dans chaque cas, l'un quelconque des groupes CH₂ desdites chaînes alkyle, alcényle ou alcynyle peut être en option remplacé par un hétéro-atome choisi dans le groupe constitué par O, S, SO, SO₂, N, et NR, dans lequel R est choisi dans le groupe constitué par l'hydrogène, un radical alkyle (en C1-C4) linéaire ou ramifié, alcényle ou alcynyle (en C2-C4) linéaire ou ramifié, et alkyle (en C1-C4) pontant dans lequel le pont est formé entre l'azote et un atome de carbone de ladite chaîne contenant un hétéro-atome pour former un cycle, et dans lequel ledit cycle est en option fusionné à un groupe Ar ; ou 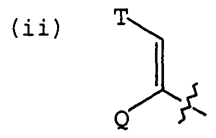
dans lequel Q est l'hydrogène, un radical alkyle (en C1-C6) linéaire ou ramifié ou alcényle ou alcynyle (en C2-C6) linéaire ou ramifié ;
dans lequel T est Ar ou un radical cycloalkyle de 5-7 membres avec des substituants en position 3 et 4 qui sont choisis indépendamment dans le groupe constitué par l'hydrogène, les radicaux oxo, hydroxyle, O-alkyle (en C1-C4) ou O-alcényle (en C2-C4) ;
sous réserve qu'au moins un de B ou D soit choisi indépendamment dans le groupe constitué par un radical alcynyle linéaire ou ramifié (en C2-C10), alcynyle linéaire ou ramifié (en C2-C6) substitué par un radical cycloalkyle (en C5-C7), alcynyle linéaire ou ramifié (en C2-C6) substitué par un radical cycloalcényle (en C5-C7), et alcynyle linéaire ou ramifié (en C2-C6) substitué par un radical Ar ;
dans lequel Ar est un groupe aromatique carbocyclique choisi dans le groupe constitué par les groupes phényle, 1-naphtyle, 2-naphtyle, indényle, azulényle, fluorényle, et anthracényle ; ou un
groupe aromatique hétérocyclique choisi dans le groupe constitué par les groupes 2-furyle, 3-furyle, 2-thiényle, 3-thïényle, 2-pyridyle, 3-pyridyle, 4-pyridyle, pyrrolyle, oxazolyle, thiazolyle, imidazolyle, pyrazolyle, 2-pyrazolinyle, pyrazolidinyle, isoxazolyle, isothiazolyle, 1,2,3-oxadiazolyle, 1,2,3-triazolyle, 1,3,4-thiadiazolyle, pyridazinyle, pyrimidinyle, pyrazinyle, 1,3,5-triazinyle, 1,3,5-trithianyle, indolizinyle, indolyle, isoindolyle, 3H-indolyle, indolinyle, benzo[b]furanyle, benzo[b]thiophényle, 1H-indazolyle, benzimidazolyle, benzthiazolyle, purinyle, 4H-quinolizinyle, quinolinyle, isoquinolinyle, cinnolinyle, phtalazinyle, quinazolinyle, quinoxalinyle, 1,8-naphtyridinyle, ptéridinyle, carbazolyle, acridinyle, phénazinyle, phénothiazinyle, et phénoxazinyle ;
dans lequel Ar peut contenir un à trois substituants qui sont choisis indépendamment dans le groupe constitué par l'hydrogène, un halogène, un groupe hydroxyle, nitro, trifluorométhyle, trifluorométhoxy, alkyle (en C1-C6) linéaire ou ramifié, alcényle (en C2-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, carboxyle, N-(alkyl ou alcényl en C1-C5 linéaire ou ramifié)carboxamide, N,N-di-(alkyl en C1-C5 linéaire ou ramifié ou alcényl en C2-C5 linéaire ou ramifié)carboxamide, N-morpholino-carboxamide, N-benzylcarboxamide, N-thiomorpholino-carboxamide, N-picolinoylcarboxamide, O-X, CH₂-(CH₂)_{q}-X, O-(CH₂)_{q}-X, (CH2)_{q}-O- et CH=CH-X; dans lequel X est un radial 4-méthoxyphényle, 2-pyridyle, 3-pyridyle, 4-pyridyle, pyrazyle, quinolyle, 3,5-diméthylisoxazoyle, isoxazoyle, 2-méthylthiazoyle, thiazoyle, 2-thiényle, 3-thiényle, et pyrimidyle, et q vaut 0-2 ;
dans lequel L est l'hydrogène ou U ; M est l'oxygène ou CH-U, sous réserve que si L est l'hydrogène, alors M est CH-U ou si M est l'oxygène, alors L est U ;
dans lequel U est l'hydrogène, un radical O-alkyle (en C1-C4) linéaire ou ramifié ou O-alcényle (en C2-C4) linéaire ou ramifié, alkyle (en C1-C6) linéaire ou ramifié ou àlcényle (en C2-C6) linéaire ou ramifié, cycloalkyle (en C5-C7) ou cycloalcényle (en C5-C7) substitué par un radical alkyle (en C1-C4) linéaire ou ramifié ou alcényle (en C2-C4) linéaire ou ramifié, [alkyl (en C1-C4) ou alcényl (en C2-C4)]-Y ou Y ;
dans lequel Y est un groupe aromatique carbocyclique choisi dans le groupe constitué par les groupes phényle, 1-naphtyle, 2-naphtyle, indényle, azulényle, fluorényle, et anthracényle ; ou un
groupe aromatique hétérocyclique tel que défini ci-dessus ;
dans lequel Y peut contenir un à trois substituants qui sont choisis indépendamment dans le groupe constitué par l'hydrogène, un halogène, un radical hydroxyle, nitro, trifluorométhyle, trifluorométhoxy, alkyle (en C1-C6) linéaire ou ramifié, alcényle (en C1-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, et carboxyle ;
dans lequel J est l'hydrogène, un radical alkyle en (C1-C2) ou benzyle ; K est un radical alkyle (en C1-C4) linéaire ou ramifié, benzyle ou cyclohexylméthyle, ou dans lequel J et K peuvent être pris ensemble pour former un cycle hétérocyclique de 5-7 membres qui peut contenir un hétéro-atome choisi dans le groupe constitué par O, S, SO et SO₂ ; et
dans lequel m vaut 0-3.

2. Composé de formule (I) : 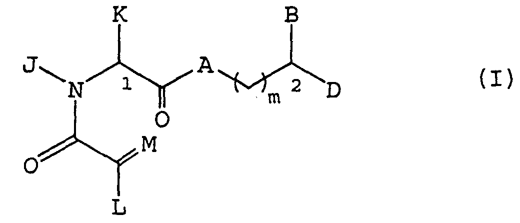 dans lequel A est CH₂, l'oxygène, NH ou N-(alkyle en C1-C4) ;
dans lequel B et D sont indépendamment :
(i) Ar, un radical alkyle, alcényle ou alcynyle (en C1-C10) linéaire ou ramifié, un radical alkyle, alcényle ou alcynyle en (C1-C6) linéaire ou ramifié, substitués par un radical cycloalkyle (en C5-C7), alkyle, alcényle ou alcynyle (en C1-C6) linéaire ou ramifié, substitués par un radical cycloalcényle (en C5-C7), ou alkyle, alcényle ou alcynyle (en C1-C6) linéaire ou ramifié, substitués par un radical Ar ; dans lequel dans chaque cas, l'un quelconque des groupes CH₂ desdites chaînes alkyle, alcényle ou alcynyle peut être en option remplacé par un hétéro-atome choisi dans le groupe constitué par O, S, SO, et SO₂ ; ou 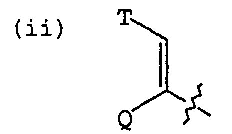
dans lequel Q est l'hydrogène, un radical alkyle (en C1-C6) linéaire ou ramifié ou alcényle(en C2-C6) linéaire ou ramifié ;
dans lequel T est Ar, ou un radical cycloalkyle de 5-7 membres substitué avec des substituants en positions 3 et 4 qui sont indépendamment choisis dans le groupe constitué par l'hydrogène, les radicaux oxo, hydroxyle, O-alkyle (en C1-C4) et O-alcényle (en C2-C4);
sous réserve qu'au moins un de B ou D soit choisi indépendamment dans le groupe constitué par un radical alcynyle linéaire ou ramifié (en C2-C10), alcynyle linéaire ou ramifié (en C2-C6) substitué par un radical cycloalkyle (en C5-C7), alcynyle linéaire ou ramifié (en C2-C6) substitué par un radical cycloalcényle (en C5-C7), et alcynyle linéaire ou ramifié (en C2-C6) substitué par un radical Ar ;
dans lequel Ar est choisi dans le groupe constitué par les groupes phényle, 1-naphtyle, 2-naphtyle, 2-furyle, 3-furyle, 2-thiényle, 3-thiényle, 2-pyridyle, 3-pyridyle, 4-pyridyle, et des systèmes hétérocycliques mono et bicycliques avec des tailles de cycles individuels étant 5 ou 6 qui peuvent contenir dans l'un ou les deux cycles un total de 1-4 hétéroatomes choisis indépendamment parmi l'oxygène, l'azote et le soufre ;
dans lequel Ar peut contenir un à trois substituants qui sont choisis indépendamment dans le groupe constitué par l'hydrogène, un halogène, un groupe hydroxyle, nitro, trifluorométhyle, trifluorométhoxy, alkyle (en C1-C6) linéaire ou ramifié, alcényle (en C2-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, carboxyle, N-(alkyl ou alcényl en C1-C5 linéaire ou ramifié)carboxamide, N,N-di-(alkyl ou alcényl en C1-C5 linéaire ou ramifié)carboxamide, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH2)_{q}-O-X et CH=CH-X ; dans lequel X est un radial 4-méthoxyphényle, 2-pyridyle, 3-pyridyle, 4-pyridyle, pyrazyle, quinolyle, 3,5-diméthylisoxazoyle, isoxazoyle, 2-méthylthiazoyle, thiazoyle, 2-thiényle, 3-thiényle, et pyrimidyle, et q vaut 0-2 ;
dans lequel L est l'hydrogène ou U et M est l'oxygène ou CH-U, sous réserve que si L est l'hydrogène, alors M est CH-U ou si M est l'oxygène, alors L est U ;
dans lequel U est l'hydrogène, un radical O-alkyle (en C1-C4) linéaire ou ramifié ou O-alcényle (en C1-C4) linéaire ou ramifié, alkyle (en C1-C6) linéaire ou ramifié ou alcényle (en C1-C6) linéaire ou ramifié, cycloalkyle (en C5-C7) ou cycloalcényle (en C5-C7) substitué par un radical alkyle (en C1-C4) linéaire ou ramifié ou alcényle (en C2-C4) linéaire ou ramifié, [alkyl (en C1-C4) ou alcényl (en C2-C4)]-Y ou Y ;
dans lequel Y est choisi dans le groupe constitué par les groupes phényle, 1-naphtyle, 2-naphtyle, 2-furyle, 3-furyle, 2-thiényle, 3-thiényle, 2-pyridyle, 3-pyridyle, 4-pyridyle, et des systèmes de cycles hétérocycliques mono et bicycliques avec des tailles de cycles individuels étant 5 ou 6 qui peuvent contenir dans l'un ou les deux cycles un totàl de 1-4 hétéro-atomes choisis indépendamment parmi l'oxygène, l'azote et le soufre ;
dans lequel Y peut contenir un à trois substituants qui sont choisis indépendamment dans le groupe constitué par l'hydrogène, un halogène, un radical hydroxyle, nitro, trifluorométhyle, trifluorométhoxy, alkyle (en C1-C6) linéaire ou ramifié, alcényle (en C1-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, et carboxyle ;
dans lequel J est l'hydrogène, un radical alkyle en (C1-C2) ou benzyle ; K est un radical alkyle (en C1-C4) linéaire ou ramifié, benzyle ou cyclohexylméthyle, ou dans lequel J et K peuvent être pris ensemble pour former un cycle hétérocyclique de 5-7 membres qui peut contenir un substituant O, S, SO et SO₂ dans celui-ci ; et
dans lequel m vaut 0-3.

3. Composé de formule (I) : 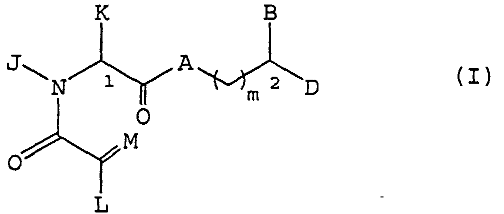 dans lequel A est CH₂, l'oxygéne, NH ou N-(alkyle en C1-C4 ) ;
dans lequel B et D sont indépendamment :
(i) Ar, un radical alkyle (en C1-C10) linéaire ou ramifié, alcényle ou alcynyle (en C2-C10) linéaire ou ramifié, un radical alkyle en (C1-C6) linéaire ou ramifié, alcényle ou alcynyle en (C2-C6) linéaire ou ramifié substitués par un radical cycloalkyle (en C5-C7), alkyle (en C1-C6) linéaire ou ramifié, alcényle ou alcynyle (en C2-C6) linéaire ou ramifié substitués par un radical cycloalcényle (en C5-C7), ou alkyle (en C1-C6) linéaire ou ramifié, alcényle ou alcynyle (en C2-C6) linéaire ou ramifié substitués par un radical Ar, dans lequel dans chaque cas, l'un quelconque des groupes CH₂, desdites chaînes alkyle, alcényle ou alcynyle peut être en option remplacé par un hétéro-atome choisi dans le groupe constitué par O, S, SO, SO₂, N, et NR, dans lequel R est choisi dans le groupe constitué par l'hydrogène, un radical alkyle (en C1-C4) linéaire ou ramifié, un radical alcényle ou alcynyle (en C2-C4) linéaire ou ramifié, et un radical alkyle (en C1-C4) pontant dans lequel le pont est formé entre l'azote et un atome de carbone de ladite chaîne contenant un hétéro-atome pour former un cycle, et dans lequel ledit cycle est en option fusionné à un groupe Ar ; ou 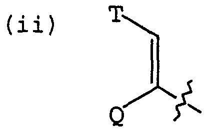
dans lequel Q est l'hydrogène, un radical alkyle (en C1-C6) linéaire ou ramifié, alcényle ou alcynyle (en C2-C6) linéaire ou ramifié ;
dans lequel T est Ar ou un radical cycloalkyle de 5-7 membres avec des substituants en position 3 et 4 qui sont choisis indépendamment dans le groupe constitué par l'hydrogène, les radicaux oxo, hydroxyle, O-alkyle (en C1-C4) ou O-alcényle (en C2-C4) ;
sous réserve qu'au moins un de B ou D soit choisi indépendamment dans le groupe constitué par Ar', un radical alkyle (en C1-C6) linéaire ou ramifié substitué par Ar', et un radical alcényle ou alcynyle linéaire ou ramifié (en C2-C6) substitué par Ar' ;
dans lequel Ar' est un groupe Ar substitué par un à trois substituants qui sont indépendamment choisis dans le groupe constitué par les radicaux N-(alkyl en C1-C5 ou alcényl en C2-C5 linéaire ou ramifié)carboxamide, N,N-di-(alkyl en C1-C5 ou alcényl en C2-C5 linéaire ou ramifié)carboxamide, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, et CH=CH-X ; dans lequel X est le radical 4-méthoxyphényle, 2-pyridyle, 3-pyridyle, 4-pyridyle, pyrazyle, quinolyle, 3,5-diméthylisoxazoyle, isoxazoyle, 2-méthylthiazolyle, thiazolyle, 2-thiényle, 3-thiényle, et pyrimidyle, dans lequel q vaut 0-2 ;
dans lequel Ar est un groupe aromatique carbocyclique choisi dans le groupe constitué par les groupes phényle, 1-naphtyle, 2-naphtyle, indényle, azulényle, fluorényle, et anthracényle ; ou un
groupe aromatique hétérocyclique choisi dans le groupe constitué par les groupes 2-furyle, 3-furyle, 2-thiényle, 3-thiényle, 2-pyridyle, 3-pyridyle, 4-pyridyle, pyrrolyle, oxazolyle, thiazolyle, imidazolyle, pyrazolyle, 2-pyrazolinyle, pyrazolidinyle, isoxazolyle, isothiazolyle, 1,2,3-oxadiazolyle, 1,2,3-triazolyle, 1,3,4-thiadiazolyle, pyridazinyle, pyrimidinyle, pyrazinyle, 1,3,5-triazinyle, 1,3,5-trithianyle, indolizinyle, indolyle, isoindolyle, 3H-indolyle, indolinyle, benzo[b]furanyle, benzo[b]thiophényle, 1H-indazolyle, benzimidazolyle, benzthiazolyle, purinyle, 4H-quinolizinyle, quinolinyle, isoquinolinyle, cinnolinyle, phtalazinyle, quinazolinyle, quinoxalinyle, 1,8-naphtyridinyle, ptéridinyle, carbazolyle, acridinyle, phénazinyle, phénothiazinyle, et phénoxazinyle ;
dans lequel Ar peut contenir un à trois substituants qui sont choisis indépendamment dans le groupe constitué par l'hydrogène, un halogène, un groupe hydroxyle, nitro, trifluorométhyle, trifluorométhoxy, alkyle (en C1-C6) linéaire ou ramifié, alcényle (en C2-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, carboxyle, N-(alkyl ou alcényl en C1-C5 linéaire ou ramifié)carboxamide, N,N-di-(alkyl en C1-C5 linéaire ou ramifié ou alcényl en C2-C5 linéaire ou ramifié)carboxamide, N-morpholino-carboxamide, N-benzylcarboxamide, N-thiomorpholino-carboxamide, N-picolinoylcarboxamide, O-X, CH₂-(CH₂)_{q}-X, O-(CH₂)_{q}-X, (CH₂)_{q}-O-X et CH=CH-X ; dans lequel X est un radial 4-méthoxyphényle, 2-pyridyle, 3-pyridyle, 4-pyridyle, pyrazyle, quinolyle, 3,5-diméthylisoxazoyle, isoxazoyle, 2-méthylthiazoyle, thiazoyle, 2-thiényle, 3-thiényle, et pyrimidyle, et q vaut 0-2 ;
dans lequel L est l'hydrogène ou U et M est l'oxygène ou CH-U, sous réserve que si L est l'hydrogène, alors M est CH-U ou si M est l'oxygène, alors L est U ;
dans lequel U est l'hydrogène, un radical O-alkyle (en C1-C4) linéaire ou ramifié ou O-alcényle (en C2-C4) linéaire ou ramifié, alkyle (en C1-C6) linéaire ou ramifié ou alcényle (en C2-C6) linéaire ou ramifié, cycloalkyle (en C5-C7) ou cycloalcényle (en C5-C7) substitué par un radical alkyle (en C1-C4) linéaire ou ramifié ou alcényle (en C2-C4) linéaire ou ramifié, [alkyl (en C1-C4) ou alcényl (en C2-C4)]-Y ou Y ;
dans lequel Y est choisi dans le groupe constitué par les groupes phényle, 1-naphtyle, 2-naphtyle, indényle, azulényle, fluorényle, anthracényle, et les groupes aromatiques hétérocycliques tels que définis ci-dessus ;
dans lequel Y peut contenir un à trois substituants qui sont choisis indépendamment dans le groupe constitué par l'hydrogène, un halogène, un radical hydroxyle, nitro, trifluorométhyle, trifluorométhoxy, alkyle (en C1-C6) linéaire ou ramifié, alcényle (en C1-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, et carboxyle ;
dans lequel J est l'hydrogène, un radical alkyle en (C1-C2) ou benzyle ; K est un radical alkyle (en C1-C4) linéaire ou ramifié, benzyle ou cyclohexylméthyle, ou dans lequel J et K peuvent être pris ensemble pour former un cycle hétérocyclique de 5-7 membres qui peut contenir un hétéro-atome choisi dans le groupe constitué par O, S, SO et SO₂ ; et
dans lequel m vaut 0-3.

4. Composition pharmaceutique comprenant un composé selon l'une quelconque des revendications 1 à 3 et un support, un adjuvant ou un véhicule acceptable du point de vue pharmaceutique.

5. Composition pharmaceutique selon la revendication 4, comprenant en outre un argent chimiothérapeutique.

6. Composition pharmaceutique selon la revendication 4 ou 5, comprenant en outre un chimio- sensibilisateur, autre que le composé selon l'une quelconque des revendications 1 à 3.

7. Utilisation d'un composé de formule (I) pour la fabrication d'un médicament pour le traitement ou la prévention de la résistance à des médicaments multiples d'un patient, **caractérisée en ce que** ledit composé a la formule : 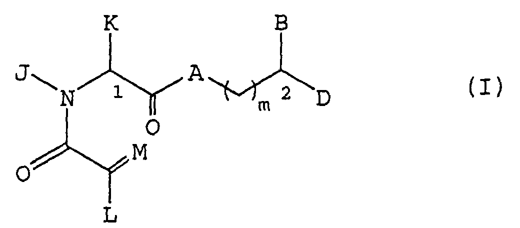 dans lequel A est CH₂, l'oxygène, NH ou N-(alkyle en C1-C4) ;
dans lequel B et D sont indépendamment :
(i) Ar, un radical alkyle (en C1-C10) linéaire ou ramifié, alcényle ou alcynyle (en C2-C10) linéaire ou ramifié, un radical alkyle en (C1-C6) linéaire ou ramifié, alcényle ou alcynyle en (C2-C6) linéaire ou ramifié, substitués par un radical cycloalkyle (en C5-C7), alkyle (en C1-C6) linéaire ou ramifié, alcényle ou alcynyle (en C2-C6) linéaire ou ramifié, substitués par un radical cycloalcényle (en C5-C7), ou alkyle (en C1-C6) linéaire ou ramifié, alcényle ou alcynyle (en C2-C6) linéaire ou ramifié substitué par un radical Ar, dans lequel, dans chaque cas, l'un quelconque des groupes CH₂ desdites chaînes alkyle, alcényle ou alcynyle peut être en option remplacé par un hétéro-atome choisi dans le groupe constitué par O, S, SO, SO₂, N; et NR, dans lequel R est choisi dans le groupe constitué par l'hydrogène, un radical alkyle (en C1-C4) linéaire ou ramifié, un radical alcényle ou alcynyle (en C2-C4) linéaire ou ramifié, et un radical alkyle (en C1-C4) pontant dans lequel le pont est formé entre l'azote et un atome de carbone de ladite chaîne contenant un hétéro-atome pour former un cycle, et dans lequel ledit cycle est en option fusionné à un groupe Ar ; ou 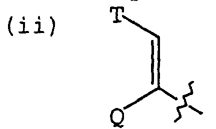
dans lequel Q est l'hydrogène, un radical alkyle (en C1-C6) linéaire ou ramifié, alcényle ou alcynyle (en C2-C6) linéaire ou ramifié ;
dans lequel T est Ar ou un radical cycloalkyle de 5-7 membres avec des substituants en positions 3 et 4 qui sont indépendamment choisis dans le groupe constitué par l'hydrogène, les radicaux oxo, hydroxyle, O-alkyle (en C1-C4) ou O-alcényle (en C2-C4) ;
dans lequel Ar est un groupe aromatique carbocyclique choisi dans le groupe constitué par les groupes phényle, 1-naphtyle, 2-naphtyle, indényle, azulényle, fluorényle, et anthracényle ; ou un
groupe aromatique hétérocyclique choisi dans le groupe constitué par les groupes 2-furyle, 3-furyle, 2-thiényle, 3-thiényle, 2-pyridyle, 3-pyridyle, 4-pyridyle, pyrrolyle, oxazolyle, thiazolyle, imidazolyle, pyrazolyle, 2-pyrazolinyle, pyrazolidinyle, isoxazolyle, isothiazolyle, 1,2,3-oxadiazolyle, 1,2,3-triazolyle, 1,3,4-thiadiazolyle, pyridazinyle, pyrimidinyle, pyrazinyle, 1,3,5-triazinyle, 1,3,5-trithianyle, indolizinyle, indolyle, isoindolyle, 3H-indolyle, indolinyle, benzo[b]furanyle, benzo[b]thiophényle, 1H-indazolyle, benzimidazolyle, benzthiazolyle, purinyle, 4H-quinolizinyle, quinolinyle, isoquinolinyle, cinnolinyle, phtalazinyle, quinazolinyle, quinoxalinyle, 1,8-naphtyridinyle, ptéridinyle, carbazolyle, acridinyle, phénazinyle, phénothiazinyle, et phénoxazinyle ;
dans lequel Ar peut contenir un à trois substituants qui sont choisis indépendamment dans le groupe constitué par l'hydrogène, un halogène, un groupe hydroxyle, nitro, trifluorométhyle, trifluorométhoxy, alkyle (en C1-C6) linéaire ou ramifié, alcényle (en C2-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, carboxyle, N-(alkyl ou alcényl en C1-C5 linéaire ou ramifié)carboxamide, N,N-di-(alkyl en C1-C5 linéaire ou ramifié ou alcényl en C2-C5 linéaire ou ramifié)carboxamide, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X et CH=CH-X ; dans lequel X est un radial 4-méthoxyphényle, 2-pyridyle, 3-pyridyle, 4-pyridyle, pyrazyle, quinolyle, 3,5-diméthylisoxazoyle, isoxazoyle, 2-méthylthiazoyle, thiazoyle, 2-thiényle, 3-thiényle, et pyrimidyle, et q vaut 0-2 ;
dans lequel L est l'hydrogène ou U ; M est l'oxygène ou CH-U, sous réserve que si L est l'hydrogène, alors M est CH-U ou si M est l'oxygène, alors L est U ;
dans lequel U est l'hydrogène, un radical O-alkyle (en C1-C4) linéaire ou ramifié ou O-alcényle (en C2-C4) linéaire ou ramifié, alkyle (en C1-C6) linéaire ou ramifié ou alcényle (en C2-C6) linéaire ou ramifié, cycloalkyle (en C5-C7) ou cycloalcényle (en C5-C7) substitué par un radical alkyle (en C1-C4) linéaire ou ramifié ou alcényle (en C2-C4) linéaire ou ramifié, [alkyl (en C1-C4) ou alcényl (en C2-C4)]-Y ou Y ;
dans lequel Y est un groupe choisi dans le groupe constitué par les groupes phényle, 1-naphtyle, 2-naphtyle, indényle, azulényle, fluorényle, et anthracényle , 2-pyrrolinyle, 3-pyrrolinyle, pyrolidinyle, 1,3-dioxolyle, 2-imidazolyinyle, imidazolidinyle, 2H-pyranyle, 4H-pyranyle, pipéridyle, 1,4-dioxanyle, morpholinyle, 1,4-dithianyle, thiomorpholinyle, pipérazinylke, quinuclidinyle, et des groupes aromatiques hétérocycliques tels que définis ci-dessus ;
dans lequel Y peut contenir un à trois substituants qui sont choisis indépendamment dans le groupe constitué par l'hydrogène, un halogène, un radical hydroxyle, nitro, trifluorométhyle, trifluorométhoxy, alkyle (en C1-C6) linéaire ou ramifié, alcényle (en C1-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, et carboxyle ;
dans lequel J est l'hydrogène, un radical alkyle en (C1-C2) ou benzyle ; K est un radical alkyle (en C1-C4) linéaire ou ramifié, benzyle ou cyclohexylméthyle, ou dans lequel J et K peuvent être pris ensemble pour former un cycle hétérocyclique de 5-7 membres qui peut contenir un hétéro-atome choisi dans le groupe constitué par O, S, SO et SO₂ ; et
dans lequel m vaut 0-3.

8. Utilisation d'un composé de formule (I) pour la fabrication d'un médicament pour le traitement ou la prévention de la résistance à des médicaments multiples d'un patient, **caractérisée en ce que** ledit composé a la formule : 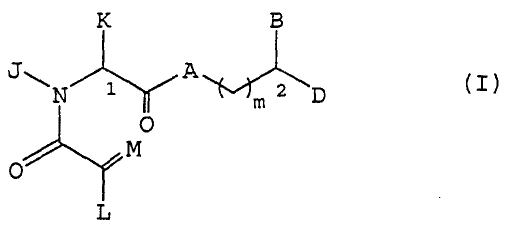 dans lequel A est CH₂, l'oxygène, NH ou N-(alkyle en C1-C4) ;
dans lequel B et D sont indépendamment :
(i) Ar, un radical alkyle (en C1-C10) linéaire ou ramifié, alcényle ou alcynyle (en C2-C10) linéaire ou ramifié, un radical alkyle, alcényle ou alcynyle en (C1-C6) linéaire ou ramifié substitués par un radical cycloalkyle (en, C5-C7), alkyle, alcényle ou alcynyle (en C1-C6) linéaire ou ramifié substitués par un radical cycloalcényle (en C5-C7), ou alkyle, alcényle ou alcynyle (en C1-C6) linéaire ou ramifié substitués par un radical Ar ; dans lequel, dans chaque cas, l'un quelconque des groupes CH₂ desdites chaînes alkyle, alcényle ou alcynyle peut être en option remplacé par un hétéro-atome choisi dans le groupe constitué par O, S, SO, SO₂ ; ou 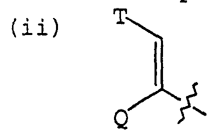
dans lequel Q est l'hydrogène, un radical alkyle (en C1-C6) linéaire ou ramifié ou alcényle (en C1-C6) linéaire ou ramifié ;
dans lequel T est Ar ou un radical cycloalkyle de 5-7 membres avec des substituants en positions 3 et 4 qui sont indépendamment choisis dans le groupe constitué par l'hydrogène, les radicaux oxo, hydroxyle, O-alkyle (en C1-C4) ou O-alcényle (en C1-C4) ;
dans lequel Ar est choisi dans le groupe constitué par les groupes phényle, 1-naphtyle, 2-naphtyle, 2-furyle, 3-furyle, 2-thiényle, 3-thiényle, 2-pyridyle, 3-pyridyle, 4-pyridyle, et des systèmes hétérocycliques mono et bicycliques avec des tailles de cycles individuels étant 5 ou 6 qui peuvent contenir dans l'un ou les deux cycles un total de 1-4 hétéroatomes choisis indépendamment parmi l'oxygène, l'azote et le soufre ;
dans lequel Ar peut contenir un à trois substituants qui sont choisis indépendamment dans le groupe constitué par l'hydrogène, un halogène, un groupe hydroxyle, nitro, trifluorométhyle, trifluorométhoxy, alkyle (en C1-C6) linéaire ou ramifié, alcényle (en C2-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, carboxyle, N-(alkyl ou alcényl en C1-C5 linéaire ou ramifié)carboxamide, N,N-di-(alkyl ou alcényl en C1-C5 linéaire ou ramifié)carboxamide, N-morpholinocarboxamide, N-benzylcarboxamide, N-thiomorpholinocarboxamide, N-picolinoylcarboxamide, O-X, CH₂- (CH₂)_{q}-X, O-(CH₂)_{q}-X, (CH₂)_{q}-O-X et CH=CH-X ; dans lequel X est un radial 4-méthoxyphényle, 2-pyridyle, 3-pyridyle, 4-pyridyle, pyrazyle, quinolyle, 3,5-diméthylisoxazoyle, isoxazoyle, 2-méthylthiazoyle, thiazoyle, 2-thiényle, 3-thiényle, et pyrimidyle, et q vaut 0-2 ;
dans lequel L est l'hydrogène ou U et M est l'oxygène ou CH-U, sous réserve que si L est l'hydrogène, alors M est CH-U ou si M est l'oxygène, alors L est U ;
dans lequel U est l'hydrogène, un radical O-alkyle (en C1-C4) linéaire ou ramifié ou O-alcényle (en C1-C4) linéaire ou ramifié, alkyle (en C1-C6) linéaire ou ramifié ou alcényle (en C1-C6) linéaire ou ramifié, cycloalkyle (en C5-C7) ou cycloalcényle (en C5-C7) substitué par un radical alkyle (en C1-C4) linéaire ou ramifié ou alcényle (en C2-C4) linéaire ou ramifié, [alkyl (en C1-C4) ou alcényl (en C2-C4)]-Y ou Y ;
dans lequel Y est choisi dans le groupe constitué par les groupes phényle, 1-naphtyle, 2-naphtyle, 2-furyle, 3-furyle, 2-thiényle, 3-thiényle, 2-pyridyle, 3-pyridyle, 4-pyridyle, et des systèmes de cycles hétérocycliques mono et bicycliques avec des tailles de cycles individuels étant 5 ou 6 qui peuvent contenir dans l'un ou les deux cycles un total de 1-4 hétéro-atomes choisi indépendamment parmi l'oxygène, l'azote et le soufre ;
dans lequel Y peut contenir un à trois substituants qui sont choisis indépendamment dans le groupe constitué par l'hydrogène, un halogène, un radical hydroxyle, nitro, trifluorométhyle, trifluorométhoxy, alkyle (en C1-C6) linéaire ou ramifié, alcényle (en C1-C6) linéaire ou ramifié, O-alkyle (en C1-C4) linéaire ou ramifié, O-alcényle (en C2-C4) linéaire ou ramifié, O-benzyle, O-phényle, 1,2-méthylènedioxy, amino, et carboxyle ;
dans lequel J est l'hydrogène, un radical alkyle en (C1-C2) ou benzyle ; K est un radical alkyle (en C1-C4) linéaire ou ramifié, benzyle ou cyclohexylméthyle, ou dans lequel J et K peuvent être pris ensemble pour former un cycle hétérocyclique de 5-7 membres qui peut contenir un hétéro-atome choisi dans le groupe constitué par O, S, SO et SO₂ dans celui-ci ; et
dans lequel m vaut 0-3.

9. Utilisation selon la revendication 7 ou 8, **caractérisée en ce que** dans la formule (I), au moins un de B ou D est choisi indépendamment dans le groupe constitué par les radicaux alcynyle (en C2-C10) linéaire ou ramifié, alcynyle (en C2-C10) linéaire ou ramifié substitués par un radical cycloalkyle (en C5-C7), alcynyle (en C2-C6) linéaire ou ramifié substitué par un radical cycloalcényle (en C5-C7) ; et alcynyle (en C2-C6) linéaire ou ramifié substitué par Ar.

10. Utilisation selon la revendication 7 ou 8, **caractérisée en ce que** dans la formule (I), au moins un de B ou D est choisi indépendamment dans le groupe constitué par Ar', un radical alkyle linéaire ou ramifié (en C1-C6) substitué par Ar' et alcényle ou alcynyle linéaire ou ramifié (en C2-C6) substitué par Ar' ;
dans lequel Ar' est un groupe Ar substitué par un à trois substituants qui sont indépendamment choisis dans le groupe constitué par les radicaux N-(alkyl en C1-C5 ou alcényl en C2-C5 linéaire ou ramifié)carboxamide, N,N-di-(alkyl en C1-C5 ou alcényl en C2-C5 linéaire ou ramifié)carboxamide, N-morpholinocarboxamide, N-benzylcarboxamide, N-thiomorpholinocarboxamide, N-picolinoylcarboxamide, O-X, CH₂-(CH₂)_{q}-X, O-(CH₂)_{q}-X, (CH₂)_{q}-O-X, et CH=CH-X ; dans lequel X est le radical 4-méthoxyphényle, 2-pyridyle, 3-pyridyle, 4-pyridyle, pyrazyle, quinolyle, 3,5-diméthylisoxazoyle, isoxazoyle, 2-méthylthiazolyle, thiazolyle, 2-thiényle, 3-thiényle, et pyrimidyle, dans lequel q vaut 0-2

11. Utilisation selon l'une quelconque des revendications 7 à 10, **caractérisée en ce que**, dans la formule (I), J et K sont pris ensemble pour former un cycle de 5-7 membres.

12. Utilisation selon l'une quelconque des revendications 7 à 10, **caractérisée en ce que**, dans la formule (I), au moins un de B ou D est représenté indépendamment par la formule -(CH₂)ᵣ-(X)-(CH₂)ₛ-Ar, dans laquelle :
r vaut 0-4 ;
s vaut 0-1 ;
Ar est tel que défini dans la revendication 1 ; et
chaque X est choisi indépendamment dans le groupe constitué par CH₂, O, S, SO, SO₂, N, et NR, dans lequel R est choisi dans le groupe constitué par l'hydrogène, un radical alkyle (en C1-C4) linéaire ou ramifié, un radical alcényle ou alcynyle (en C2-C4) linéaire ou ramifié, et un radical alkyle (en C1-C4) pontant dans lequel le pont est formé entre l'azote et le groupe Ar.

13. Utilisation selon la revendication 7, **caractérisé en ce que** ledit composé de formule (I) est choisi dans le groupe constitué par :
l'ester 4-pyridin-3-yl-1-(3-pyridin-3-yl)propyl)-butyle de l'acide (S)-1-(2-oxo-2-(3,4,5-triméthoxy-ghényl)-acétyl)pipéridine-2-carboxylique ;
l'ester 4-pyridin-3-yl-1-(3-pyridin-3-yl)propyl)-butyle de l'acide (R)-1-(2-oxo-2-(3,4,5-triméthoxy-phényl)-acétyl)pipéridine-2-carboxylique ; et leurs dérivés acceptables du point de vue pharmaceutique, et les mélanges de ceux-ci.

14. Composition pharmaceutique selon l'une quelconque des revendications 4 à 6 pour le traitement ou la prévention de la résistance à des médicaments multiples.

15. Composition pharmaceutique selon l'une quelconque des revendications 4 à 6, **caractérisée en ce que** le composé n'est pas sensiblement immunosuppresseur à la quantité de dosage requise pour provoquer la chimio-sensibilisation.

16. Utilisation d'un composé selon l'une quelconque des revendications 1 à 3 pour la préparation d'une composition pour le traitement ou la prévention de la résistance à des médicaments multiples chez un patient.

17. Procédé de synthèse d'un composé de formule (I') 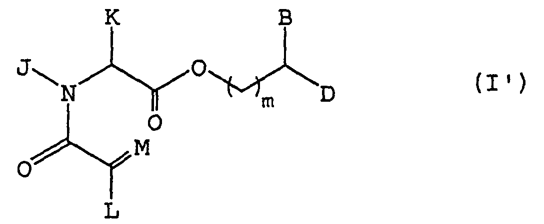 comprenant les étapes consistant à :
(a) estérifier un acide aminé protégé de formule (X) avec un alcool de formule (XI) : 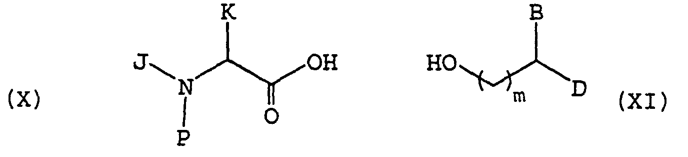 pour obtenir un intermédiaire de formule (XII) : 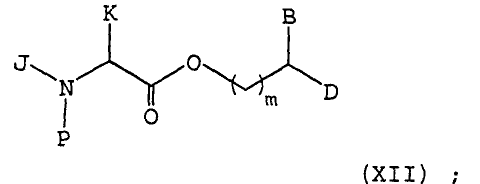
(b) déprotéger le groupe protecteur de groupe amino dans l'intermédiaire de formule (XII) pour obtenir un amino-ester de formule (XIII) : 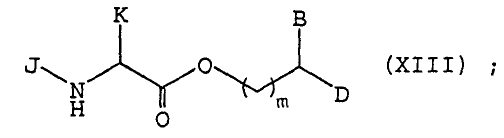 et
(c) acyler le groupe amino libre dans le composé de formule (XIII) par un composé de formule (XIV) : 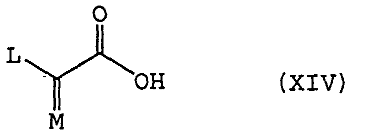
ou un dérivé activé de celui-ci ;
où P est un groupe protecteur et A, B, D, J, K, L, et M sont tels que définis dans la revendication 1.

18. Procédé de synthèse d'un composé de formule (I") 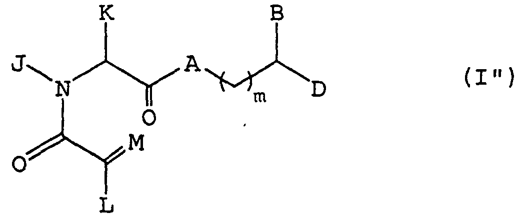 dans laquelle A est NH ou N-(alkyle en C1-C4) comprenant les étapes consistant à :
(a) effectuer une réaction de couplage peptidique entre un acide carboxylique de formule (X) et une amine de formule (XI''') 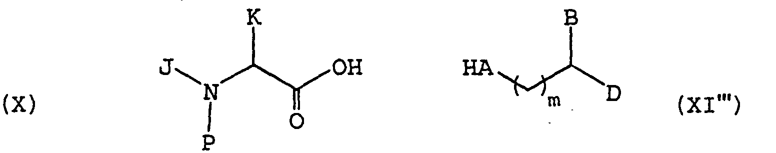 pour obtenir un intermédiaire de formule (XII') : 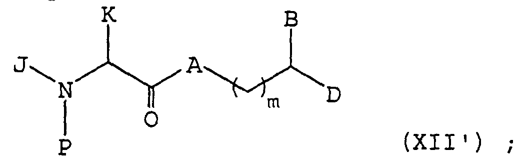
(b)déprotéger le groupe protecteur du groupe amino dans l'intermédiaire de formule (XII') pour obtenir le composé de formule (XIII') : 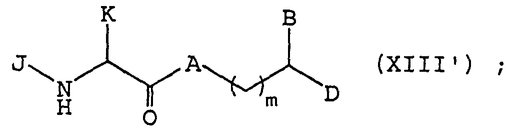 et
(c) acyler le groupe amino libre dans le composé de formule (XIII') par un composé de formule (XIV) : 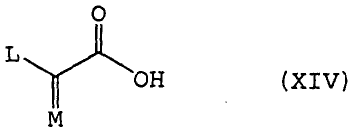
ou un dérivé activé de celui-ci ;
où P est un groupe protecteur et A, B, D, J, K, L, et M sont tels que définis dans la revendication 1.

19. Procédé selon la revendication 17 ou 18, **caractérisé en ce que** ledit groupe protecteur est le groupe alcoxycarbonyle.

20. Procédé de préparation d'une composition pharmaceutique selon l'une quelconque des revendications 4, 5, 6, 14 ou 15 qui comprend la combinaison d'un composé selon l'une quelconque des revendications 1 à 3 avec un support, un adjuvant ou un véhicule acceptable du point de vue pharmaceutique et, en option, un agent chimiothérapeutique et/ou un chimiosensibilisateur.
